(19) Europäisches Patentamt
European Patent Office
Office européen des brevets

(11) Veröffentlichungsnummer: **0 239 815 B1**

# (12) EUROPÄISCHE PATENTSCHRIFT

(45) Veröffentlichungstag der Patentschrift: **24.07.91**

(21) Anmeldenummer: **87102995.5**

(22) Anmeldetag: **03.03.87**

(51) Int. Cl.5: **C07D 277/24**, C07D 263/32, C07D 417/04, C07D 277/42, C07D 277/40, C07D 413/04, C07D 277/32, C07D 277/46, C07D 277/22, A61K 31/425, A61K 31/42

## (54) Substituierte Thiazole und Oxazole, diese Verbindungen enthaltende Arzneimittel und Verfahren zu ihrer Herstellung.

(30) Priorität: **13.03.86 DE 3608290**
**28.06.86 DE 3621775**

(43) Veröffentlichungstag der Anmeldung:
**07.10.87 Patentblatt 87/41**

(45) Bekanntmachung des Hinweises auf die Patenterteilung:
**24.07.91 Patentblatt 91/30**

(84) Benannte Vertragsstaaten:
**AT BE CH DE ES FR GB GR IT LI LU NL SE**

(56) Entgegenhaltungen:
**EP-A- 0 005 848**
**DE-A- 2 458 744**
**FR-M- 8 021**

(73) Patentinhaber: **Dr. Karl Thomae GmbH**
**Postfach 1755**
**W-7950 Biberach (Riss)(DE)**

(72) Erfinder: **Reiffen, Manfred, Dr. Dipl.-Chem.**
**Matthias-Erzberger-Strasse 40**
**W-7950 Biberach 1(DE)**
Erfinder: **Hurnaus, Rudolf, Dr. Dipl.-Chem.**
**Silcherstrasse 19**
**W-7950 Biberach 1(DE)**
Erfinder: **Sauter, Robert, Dr. Dipl.-Chem.**
**Albert-Schweizer-Weg 9**
**W-7958 Laupheim(DE)**
Erfinder: **Grell, Wolfgang, Dr. Dipl.-Chem.**
**Amriswilstrasse 7**
**W-7950 Biberach 1(DE)**
Erfinder: **Rupprecht, Eckhard, Dr. Dipl.-Biol.**
**Riedbachstrasse 15**
**W-7960 Aulendorf-Tannhausen(DE)**

**EP 0 239 815 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

In der BSM 8.021 M werden 2-Hydroxy-2-phenylethylamine beschrieben, in welchen der Aminstickstoff durch eine in 3-Stellung durch einen Heteroarylrest substituierte -3-Hydroxy-propyl- oder Propan-3-on-gruppe substituiert ist, welche eine Herz-/Kreislaufwirkung aufweisen.

Außerdem fallen unter die allgemeine Formel der EP-A-0.005.848 u. a. Verbindungen der allgemeinen Formel

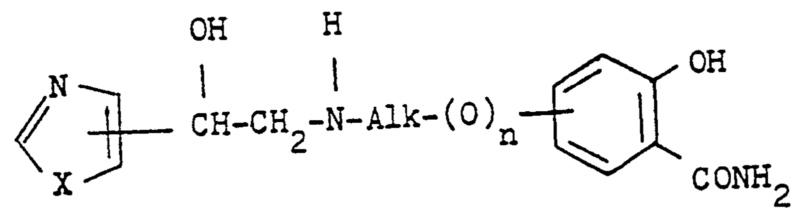

in der
X ein Sauerstoff- oder Schwefelatom,
Alk eine geradkettige oder verzweigte Alkylengruppe mit 2 bis 5 Kohlenstoffatomen und
n die Zahl 0 oder 1 darstellen. Keine dieser Verbindungen wird jedoch in der obenerwähnten EP-A-0.005.848 explizit beschrieben. Diese Verbindungen sollen wertvolle pharmakologische Eigenschaften aufweisen, insbesondere eine stimulierende Wirkung auf die cardialen ß-Rezeptoren, z.B. eine positiv inotrope oder positiv chronotrope Wirkung.

Überraschenderweise wurde nun gefunden, daß die neuen substituierten Thiazole und Oxazole der allgemeinen Formel

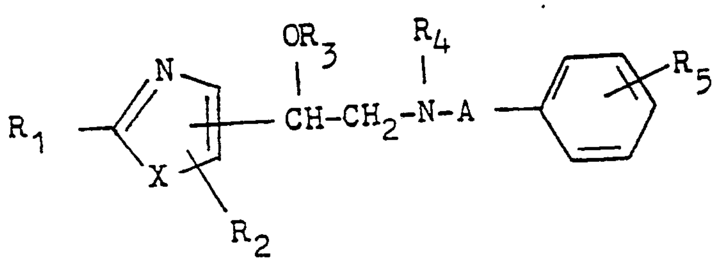

,(I)

deren optische Isomere und Diastereomere, da die neuen Verbindungen ein oder mehrere optisch aktive Kohlenstoffatome enthalten, sowie deren Säureadditionssalze wertvolle Eigenschaften aufweisen.

So stellen die Lactame der allgemeinen Formel I, deren optische Isomere und Diastereomere wertvolle Zwischenprodukte zur Herstellung der Morpholine der allgemeinen Formel I dar, in denen $R_3$ und $R_4$ zusammen eine Ethylengruppe darstellen. Diese Morpholine und die übrigen Verbindungen der obigen allgemeinen Formel I (ausgenommen die Lactame), deren optische Isomere und deren Diastereomere sowie deren Säureadditionssalze, insbesondere deren physiologisch verträgliche Säureadditionssalze mit anorganischen oder organischen Säuren, weisen ganz andere pharmakologische Eigenschaften auf, nämlich eine Wirkung auf den Stoffwechsel, vorzugsweise eine blutzuckersenkende und körperfettreduzierende Wirkung, sowie eine senkende Wirkung auf die atherogenen ß-Lipoproteine VLDL und LDL. Außerdem weisen einige der vorstehend erwähnten Verbindungen auch eine anabole Wirkung auf.

In der obigen allgemeinen Formel I bedeutet
A eine gegebenenfalls durch Methyl- oder Ethylgruppen mono-oder disubstituierte n-Alkylengruppe mit 2 oder 3 Kohlenstoffatomen,
X ein Sauerstoff- oder Schwefelatom,
$R_1$ ein Wasserstoff- oder Halogenatom, eine Trifluormethyl-, Alkyl-, Phenyl- oder Piperidinogruppe oder eine gegebenenfalls durch ein oder zwei Alkylgruppen oder eine Alkanoyl- oder Benzoylgruppe substituierte Aminogruppe,
$R_2$ ein Wasserstoffatom oder eine Alkylgruppe,
$R_3$ ein Wasserstoffatom oder eine Alkylgruppe, die in 2-oder 3-Stellung durch eine Hydroxygruppe

substituiert sein kann, oder $R_3$ zusammen mit $R_4$ eine Alkoxycarbonylmethylengruppe oder eine Ethylengruppe, wobei die zum N- oder O-Atom benachbarte Methylengruppe durch eine Carbonylgruppe ersetzt sein kann,

$R_4$ ein Wasserstoffatom, eine gegebenenfalls durch eine Phenyl-, Carboxy-, Alkoxycarbonyl- oder Cyanogruppe oder in 2- oder 3-Stellung durch eine Hydroxygruppe substituierte Alkylgruppe oder eine Alkenylgruppe und

$R_5$ eine Hydroxy-, Alkoxy-, Carboxy-, Alkoxycarbonyl-, Aminocarbonyl-, Alkylaminocarbonyl- oder Dialkylaminocarbonylgruppe, eine Alkoxygruppe mit l bis 6 Kohlenstoffatomen, die endständig durch eine Carboxy-, Alkoxycarbonyl-, Aminocarbonyl-, Alkylaminocarbonyl- oder Dialkylaminocarbonylgruppe substituiert ist, eine Alkoxygruppe mit 2 bis 7 Kohlenstoffatomen, die endständig durch eine Hydroxy-, Alkoxy-, Phenylalkoxy-, Amino-, Alkylamino-, Dialkylamino-, Pyrrolidino-, Piperidino- oder Hexamethyleniminogruppe substituiert ist, oder eine gegebenenfalls durch eine Alkylgruppe substituierte Ethenylengruppe, die endständig durch eine Carboxy-, Alkoxycarbonyl-, Aminocarbonyl-, Alkylaminocarbonyl- oder Dialkylaminocarbonylgruppe substituiert ist,

wobei alle vorstehend erwähnten Alkyl-, Alkoxy- oder Alkanoylgruppen, sofern nichts anderes erwähnt wurde, jeweils l bis 3 Kohlenstoffatome und die vorstehend erwähnten Alkenylgruppen 3 bis 5 Kohlenstoffatomen enthalten können.

Für die bei der Definition der Reste eingangs erwähnten Bedeutungen kommt beispielsweise

für A die der Ethylen-, l-Methyl-ethylen-, 2-Methyl-ethylen-, l-Ethyl-ethylen-, 2-Ethyl-ethylen-, l,2-Dimethyl-ethylen-, l,l-Dimethyl-ethylen-, l,l-Diethyl-ethylen-, l-Ethyl-l-methyl-ethylen-, 2,2-Dimethyl-ethylen-, 2,2-Diethyl-ethylen-, 2-Ethyl-2-methyl-ethylen-, n-Propylen-, l-Methyl-n-propylen-, 2-Methyl-n-propylen-, 3-Methyl-n-propylen-, l-Ethyl-n-propylen-,2-Ethyl-n-propylen-, 3-Ethyl-n-propylen, l,l-Dimethyl-n-propylen-,l,l-Diethyl-n-propylen-, 2,3-Dimethyl-n-propylen-, 3,3-Dimethyl-n-propylen- oder 3-Ethyl-3-methyl-n-propylengruppe,

für $R_1$ die des Wasserstoff-, Fluor-, Chlor- oder Bromatoms, der Trifluormethyl-, Methyl-, Ethyl-, n-Propyl-, Isopropyl-, Phenyl-, Amino-, Methylamino-, Ethylamino-, Isopropylamino-, Dimethylamino-, Diethylamino-, Di-n-propylamino-, N-Ethyl-methylamino-, N-Ethyl-n-propylamino-, Piperidino-, Formylamino-, Acetamino-, Propionylamino- oder Benzoylaminogruppe,

für $R_2$ die des Wasserstoffatoms, der Methyl-, Ethyl-, n-Propyl- oder Isopropylgruppe,

für $R_3$ die des Wasserstoffatoms, der Methyl-, Ethyl-, n-Propyl-, Isopropyl-, 2-Hydroxy-ethyl-, 2-Hydroxy-propyl-oder 3-Hydroxy-propylgruppe oder

für $R_3$ zusammen mit $R_4$ die der Ethylengruppe, in welcher eine Methylengruppe durch eine Carbonylgruppe ersetzt sein kann, die der Methoxycarbonylmethylen-, Ethoxycarbonylmethylen-, n-Propoxycarbonylmethylen- oder Isopropoxycarbonylmethylengruppe,

für $R_4$ die des Wasserstaffatoms, der Methyl-, Ethyl-, n-Propyl-, Isopropyl-, Benzyl-, l-Phenylethyl-, 2-Phenylethyl-, 3-Phenyl-n-propyl-, Carboxymethyl-, l-Carboxyethyl-, 2-Carboxyethyl-, 3-Carboxy-n-propyl-, Methoxycarbonylmethyl-, Ethoxycarbonylmethyl-, n-Propoxycarbonylmethyl-, l-Ethoxycarbonylethyl-, 2-Methoxycarbonylethyl-, 2-Ethoxycarbonylethyl-, 3-Isopropoxycarbonyl-n-propyl-, Cyanomethyl-, l-Cyanoethyl-, 2-Cyanoethyl-, 3-Cyano-n-propyl-, 2-Hydroxy-ethyl-, 3-Hydroxy-n-propyl-, Allyl-, Buten-2-yl-, Buten-3-yl- oder Penten-2-yl-gruppe und

für $R_5$ die der Hydroxy-, Methoxy-, Ethoxy-, n-Propoxy-, Isopropoxy-, Carboxy-, Methoxycarbonyl-, Ethoxycarbonyl-, n-Propoxycarbonyl-, Aminocarbonyl-, Methylaminocarbonyl-, Ethylaminocarbonyl-, Isopropylaminocarbonyl-, Dimethylaminocarbonyl-, Diethylaminocarbonyl-, Di-n-propylaminocarbonyl-, N-Ethyl-methylaminocarbonyl-, N-Ethyl-isopropylaminocarbonyl-, 2-Hydroxy-ethoxy-, 3-Hydroxy-n-propoxy-, 4-Hydroxy-n-butoxy-, 5-Hydroxy-n-pentoxy-, 6-Hydroxy-n-hexoxy-, 7-Hydroxy-n-heptoxy-,2-Methoxy-ethoxy-, 2-Ethoxy-ethoxy-, 2-n-Propoxy-ethoxy-, 3-Ethoxy-n-propoxy-, 4-Methoxy-n-butoxy-,6-Ethoxy-n-hexoxy-, 2-Phenethoxy-ethoxy-, 2-Amino-ethoxy-, 2-Methylamino-ethoxy-, 2-Dimethylamino-ethoxy-, 2-Isopropylamino-ethoxy-, 2-Di-n-propylamino-ethoxy-, 2-(l-Pyrrolidino)ethoxy-, 2-(l-Piperidino)ethoxy-, 2-(l-Hexamethylenimino)ethoxy-, 3-Amino-n-propoxy-, 6-Amino-n-hexoxy-, 7-Methylamino-n-heptoxy-,3-Diethylamino-n-propoxy-, 3-(l-Piperidino)-n-propoxy-, 4-Dimethylamino-n-butoxy-, Carboxymethoxy-, 2-Carboxy-ethoxy-, 3-Carboxy-n-propoxy-, 4-Carboxy-n-butoxy-, Methoxycarbonylmethoxy-, 2-Methoxycarbonyl-ethoxy-, 6-Methoxycarbonylhexoxy-, Ethoxycarbonylmethoxy-, 2-Ethoxycarbonyl-ethoxy-, 3-Ethoxycarbonyl-n-propoxy-, n-Propoxycarbonylmethoxy-, 2-Isopropoxycarbonyl-ethoxy-, 4-n-Propoxycarbonyl-n-butoxy-, Aminocarbonylmethoxy-, 2-Aminocarbonyl-ethoxy-, 4-Aminocarbonyl-n-butoxy-, Methylaminocarbonylmethoxy-, 2-Methylaminocarbonyl-ethoxy-, Dimethylaminocarbonylmethoxy-, 2-Dimethylaminocarbonyl-ethoxy-, 4-Dimethylaminocarbonyl-n-butoxy-, Diethylaminocarbonylmethoxy-, 2-Diethylaminocarbonyl-ethoxy-, 2-Di-n-propylaminocarbonyl-ethoxy-, 2-Carboxy-ethenyl-, 2-Carboxy-l-methyl-ethenyl-, 2-Carboxy-2-methyl-ethenyl-, 2-Carboxy-l-ethyl-ethenyl-, 2-Carboxy-l-n-propyl-ethenyl-, 2-Methoxycarbonyl-ethenyl-, 2-Methoxycarbonyl-l-methyl-ethenyl-, 2-Ethoxycarbonyl-ethenyl-, 2-Ethoxycarbonyl-l-methylethenyl- oder 2-Isopropoxycarbonyl-ethenylgruppe in Betracht.

Beispielsweise seien zusätzlich zu den in den Beispielen genannten Verbindungen noch folgende Verbindungen genannt, die unter die vorstehend erwähnte allgemeine Formel I fallen:

N-[2-(4-Carbomethoxymethoxyphenyl)-l-methylethyl]-2-hydroxy-2-(2-dimethylamino-thiazol-4-yl)ethanamin,
N-[2-(4-Carbomethoxymethoxyphenyl)-l-methylethyl]-2-hydroxy-2-(2-methylamino-thiazol-4-yl)ethanamin,
N-[2-(4-Carbomethoxymethoxyphenyl)-l-methylethyl]-2-hydroxy-2-(2-amino-thiazol-4-yl)ethanamin,
N-[2-(4-Carbomethoxymethoxyphenyl)-l-methylethyl]-2-hydroxy-2-(2-trifluormethyl-thiazol-5-yl)ethanamin,
N-[2-(4-Carbomethoxymethoxyphenyl)-l-methylethyl]-2-hydroxy-2-(2-trifluormethyl-4-methyl-thiazol-5-yl)-ethanamin,
N-[2-(4-Carbomethoxymethoxyphenyl)-l-methylethyl]-2-methoxy-2-(2-trifluormethyl-thiazol-4-yl)ethanamin,
N-[2-(4-Carbomethoxymethoxyphenyl)-l-methylethyl]-N-methyl-2-methoxy-2-(2-trifluormethyl-thiazol-4-yl)-ethanamin,
N-[2-(4-Carbomethoxymethoxyphenyl)-l-methylethyl]-N-methyl-2-hydroxy-2-(2-methyl-thiazol-4-yl)-ethanamin,
N-[2-(4-Carbomethoxymethoxyphenyl)-l-methylethyl]-N-allyl-2-hydroxy-2-(2-trifluormethyl-thiazol-4-yl)-ethanamin,
N-[2-(4-Carbomethoxymethoxyphenyl)-l-methylethyl]-N-(2-phenylethyl)-2-hydroxy-2-(2-trifluormethyl-thiazol-4-yl)ethanamin,
N-[2-(4-Carbomethoxymethoxyphenyl)-l-methylethyl]-N-cyanomethyl-2-hydroxy-2-(2-trifluormethyl-thiazol-4-yl)ethanamin,
N-[2-(4-Methylaminocarbonylmethoxyphenyl)-l-methylethyl]-2-hydroxy-2-(2-dimethylamino-thiazol-4-yl)-ethanamin,
N-[2-(4-Methylaminocarbonylmethoxyphenyl)-l-methylethyl]-2-hydroxy-2-(2-methylamino-thiazol-4-yl)-ethanamin,
N-[2-(4-Methylaminocarbonylmethoxyphenyl)-l-methylethyl]-2-hydroxy-2-(2-trifluormethyl-thiazol-5-yl)-ethanamin,
N-[2-(4-Methylaminocarbonylmethoxyphenyl)-l-methylethyl]-2-hydroxy-2-(2-methyl-thiazol-5-yl)ethanamin,
N-[2-(4-Methylaminocarbonylmethoxyphenyl)-l-methylethyl]-2-hydroxy-2-(2-trifluormethyl-4-methyl-thiazol-5-yl)ethanamin,
N-[2-(4-Methylaminocarbonylmethoxyphenyl)-l-methylethyl]-2-hydroxy-2-(2-methyl-oxazol-4-yl)ethanamin,
N-[2-(4-Methylaminocarbonylmethoxyphenyl)-l-methylethyl]-2-methoxy-2-(2-trifluormethyl-thiazol-4-yl)-ethanamin,
N-[2-(4-Methylaminocarbonylmethoxyphenyl)-l-methylethyl]-N-methyl-2-methoxy-2-(2-trifluormethyl-thiazol-4-yl)ethanamin,
N-[2-(4-Methylaminocarbonylmethoxyphenyl)-l-methylethyl]-N-methyl-2-hydroxy-2-(2-methyl-thiazol-4-yl)-ethanamin,
N-[2-(4-Methylaminocarbonylmethoxyphenyl)-l-methylethyl]-2-methoxy-2-(2-methyl-thiazol-4-yl)ethanamin,
N-[2-(4-Methylaminocarbonylmethoxyphenyl)-l-methylethyl]-N-allyl-2-hydroxy-2-(2-trifluormethyl-thiazol-4-yl)ethanamin,
N-[2-(4-Methylaminocarbonylmethoxyphenyl)-l-methylethyl]-N-(2-phenylethyl)-2-hydroxy-2-(2-trifluormethyl-thiazol-4-yl)ethanamin,
N-[2-(4-Methylaminocarbonylmethoxyphenyl)-l-methylethyl]-N-cyanomethyl-2-hydroxy-2-(2-trifluormethyl-thiazol-4-yl)-ethanamin,
N-[2-(4-Methylaminocarbonylmethoxyphenyl)ethyl]-2-hydroxy-2-(2-trifluormethyl-thiazol-4-yl)ethanamin,
N-[2-(4-Methylaminocarbonylmethoxyphenyl)ethyl]-2-hydroxy-2-(2-methyl-thiazol-4-yl)ethanamin,
N-[2-(4-Aminocarbonylmethoxyphenyl)-l-methylethyl]-N-(2-hydroxyethyl)-2-hydroxy-2-(2-trifluormethyl-thiazol-4-yl)ethanamin,
N-[2-(4-(6-Hydroxyhexoxy)phenyl)-l-methylethyl]-2-hydroxy-2-(2-methyl-thiazol-4-yl)ethanamin,
N-[2-(4-(2-(l-Piperidino)ethoxy)phenyl)-l-methylethyl]-2-hydroxy-2-(2-trifluormethyl-thiazol-4-yl)ethanamin,
N-[2-(4-(2-Methylaminoethoxy)phenyl)-l-methylethyl]-2-hydroxy-2-(2-methyl-thiazol-4-yl)ethanamin,
N-[2-(4-Methylaminocarbonylmethoxyphenyl)-l-methylethyl]-2-hydroxy-2-(2-methyl-thiazol-4-yl)ethanamin,
N-[2-(4-Aminocarbonylmethoxyphenyl)-l-methylethyl]-2-(2-methyl-thiazol-4-yl)morpholin,
N-[2-(4-Carbomethoxyphenyl)-l-methylethyl]-2-(2-trifluormethyl-thiazol-4-yl)morpholin,
N-[2-(4-(2-(l-Piperidino)ethoxy)phenyl)-l-methylethyl]-2-(2-trifluormethyl-thiazol-4-yl)morpholin,
3-[2-(4-Carbomethoxymethoxyphenyl)-l-methylethyl]-5-(2-methyl-thiazol-4-yl)-2-oxazolidincarbonsäure-methylester,
3-[2-(4-Aminocarbonylmethoxyphenyl)-l-methylethyl]-5-(2-methyl-thiazol-4-yl)-2-oxazolidincarbonsäure-methylester,
3-[2-(4-Methylaminocarbonylmethoxyphenyl)-l-methylethyl]-5-(2-methyl-thiazol-4-yl)-2-oxazolidincarbonsäure-methylester,
3-[2-(4-Carbomethoxymethoxyphenyl)ethyl]-5-(2-trifluormethylthiazol-4-yl)-2-oxazolidincarbonsäure-methylester,
3-[2-(4-Aminocarbonylmethoxyphenyl)-l-methylethyl]-5-(2-trifluormethyl-thiazol-4-yl)-2-oxazolidincarbonsäuremethylester,
3-[2-(4-Methylaminocarbonylmethoxyphenyl)-l-methylethyl]-5-(2-trifluormethyl-thiazol-4-yl)-2-oxazolidincarbonsäure-methylester,
3-[2-(4-Carboxymethoxyphenyl)-l-methylethyl]-5-(2-trifluormethyl-thiazol-4-yl)-2-oxazolidincarbonsäure-methylester,
3-[2-(4-(6-Hydroxyhexoxy)phenyl)-l-methylethyl]-5-(2-trifluormethyl-thiazol-4-yl)-2-oxazolidincarbonsäure-methylester,
3-[2-(4-(2-Hydroxyethoxy)phenyl)-l-methylethyl]-5-(2-trifluormethyl-thiazol-4-yl)-2-oxazolidincarbonsäure-methylester,
3-[2-(4-(2-Methylaminoethoxy)phenyl)-l-methylethyl]-5-(2-trifluormethyl-thiazol-4-yl)-2-oxazolidincarbonsäure-methylester,
3-[2-(4-(2-(l-Piperidino)ethoxy)phenyl)-l-methylethyl]-5-(2-trifluormethyl-thiazol-4-yl)-2-oxazolidincarbonsäuremethylester,
3-[3-(4-Carboxamidophenyl)-l-methylpropyl)-5-(2-trifluormethyl-thiazol-4-yl)-2-oxazolidincarbonsäure-methylester,
N-[2-(4-Methylaminocarbonylmethoxyphenyl)-l-methylethyl]-2-hydroxy-2-(2-chlor-thiazol-4-yl)ethanamin,
N-[2-(4-Carbomethoxymethoxyphenyl)-l-methylethyl]-2-methoxy-2-(2-chlor-thiazol-4-yl)ethanamin,
N-[2-(4-Carbomethoxymethoxyphenyl)-l-methylethyl]-N-methyl-2-hydroxy-2-(2-chlor-thiazol-4-yl)ethanamin,
N-[2-(4-Carbomethoxymethoxyphenyl)-l-methylethyl]-N-methyl-2-methoxy-2-(2-chlor-thiazol-4-yl)ethanamin,
N-[2-(4-Carbomethoxymethoxyphenyl)-l-methylethyl]-5-(2-chlor-thiazol-4-yl)-2-oxazolidincarbonsäuremethylester,
N-[2-(4-(2-Ethoxyethoxy)phenyl)-l-methylethyl]-N-(2-hydroxy-ethyl)-2-hydroxy-2-(2-chlor-thiazol-4-yl)-ethanamin,
3-[2-(4-(2-Carbomethoxy-l-methylethenyl)phenyl)-l-methyl-ethyl]-5-(2-chlor-thiazol-4-yl)-2-oxazolidincarbonsäuremethyl-ester,
N-[2-(4-(2-Hydroxyethoxy)phenyl)-l-methylethyl]-2-hydroxy-2-(2-methyl-oxazol-4-yl)ethanamin,
N-[2-(4-Carbomethoxymethoxyphenyl)-l-methylethyl]-2-(2-methyl-oxazol-4-yl)morpholin,
N-[2-(4-Carbomethoxymethoxyphenyl)-l-methylethyl]-N-(2-hydroxyethyl)-2-hydroxy-2-(2-methyl-oxazol-4-yl)-ethanamin,
3-[2-(4-(2-Carbomethoxy-l-methylethenyl)phenyl)-l-methylethyl]-5-(2-methyl-oxazol-4-yl)-2-oxazolidincarbonsäure-methylester,
N-[2-(4-(2-Hydroxyethoxy)phenyl)-l-methylethyl]-N-methyl-2-hydroxy-2-(2-trifluormethyl-thiazol-4-yl)-ethanamin,
N-[2-(4-Carbomethoxymethoxyphenyl)-l-methylethyl]-N-(2-hydroxyethyl)-2-methoxy-2-(2-trifluormethyl-thiazol-4-yl)ethanamin,
N-[2-(4-(2-Hydroxyethoxy)phenyl)-l-methylethyl]-2-methoxy-2-(2-trifluormethyl-thiazol-4-yl)ethanamin,
N-[2-(4-(2-Hydroxyethoxy)phenyl)-l-methylethyl]-2-(2-trifluormethyl-thiazol-4-yl)morpholin-6-on,
N-[2-(4-Carboxymethoxyphenyl)-l-methylethyl]-2-(2-trifluormethyl-thiazol-4-yl)morpholin-6-on,
N-[2-(4-Carbomethoxymethoxyphenyl)-l-methylethyl]-2-(2-chlor-thiazol-4-yl)morpholin-6-on,
N-[2-(4-Methylaminocarbonylmethoxyphenyl)-l-methylethyl]-2-(2-trifluormethyl-thiazol-4-yl)morpholin-6-on,
N-[2-(4-Aminocarbonylmethoxyphenyl)-l-methylethyl]-N-(2-hydroxyethyl)-2-hydroxy-2-(2-trifluormethyl-thiazol-4-yl)ethanamin,
N-[2-(4-Methylaminocarbonylmethoxyphenyl)-l-methylethyl]-N-(2-hydroxyethyl)-2-hydroxy-2-(2-trifluormethyl-thiazol-4-yl)-ethanamin,
N-[2-(4-Hydroxyethoxy)phenyl)-l-methylethyl]-N-carbethoxymethyl-2-hydroxy-2-(2-trifluormethyl-thiazol-4-yl)-ethanamin,
N-[2-(4-(2-Methoxyethoxy)phenyl)-l-methylethyl]-2-hydroxy-2-(trifluormethyl-thiazol-4-yl)ethanamin,
N-[2-(4-(2-Methoxyethoxy)phenyl)-l-methylethyl]-2-(2-trifluormethyl-thiazol-4-yl)morpholin,
N-[2-(4-Carboxymethoxyphenyl)-l-methylethyl]-N-methyl-2-hydroxy-2-(2-trifluormethyl-thiazol-4-yl)ethanamin,
N-[2-(4-(2-Ethoxyethoxy)phenyl)-l-methylethyl]-2-hydroxy-2-(2-chlor-thiazol-4-yl)ethanamin,
N-[2-(4-(2-Ethoxyethoxy)phenyl)-l-methylethyl]-2-(2-chlor-thiazol-4-yl)morpholin,
N-[2-(4-(2-Phenethoxyethoxy)phenyl)-l-methylethyl]-2-hydroxy-2-(2-chlor-thiazol-4-yl)ethanamin,

N-[2-(4-(2-Phenethoxyethoxy)phenyl)-l-methylethyl]-2-(2-chlor-thiazol-4-yl)morpholin,
N-[2-(4-(2-Phenethoxyethoxy)phenyl)-l-methylethyl]-N-(2-hydroxyethyl)-2-hydroxy-2-(2-chlor-thiazol-4-yl)-ethanamin und
3-[2-(4-(2-Ethoxyethoxy)phenyl)-l-methylethyl]-5-(2-trifluormethyl-thiazol-4-yl)-2-oxazolidincarbonsäure-methylester,
deren optisch Isomere, deren Diastereomere und deren Säureadditionssalze.

Bevorzugt sind jedoch die Verbindungen der allgemeinen Formel I, in der
A eine gegebenenfalls durch eine Methylgruppe substituierte Ethylen- oder n-Propylengruppe,
X ein Sauerstoff- oder Schwefelatom,
$R_1$ ein Wasserstoff- oder Chloratom, eine Alkylgruppe mit l bis 3 Kohlenstoffatomen, eine Trifluormethyl-, Phenyl-, Amino-, Methylamino-, Dimethylamino-, Piperidino-, Acetylamino- oder Benzoylaminogruppe,
$R_2$ ein Wasserstoffatom oder eine Methylgrupppe,
$R_3$ ein Wasserstoffatom, eine Methylgruppe oder $R_3$ und $R_4$ zusammen eine Methoxycarbonylmethylengruppe oder eine Ethylengruppe, wobei die zum O-Atom benachbarte Methylengruppe durch eine Carbonylgruppe ersetzt sein kann,
$R_4$ ein Wasserstoffatom, eine Methyl-, 2-Hydroxy-ethyl-, Carboxymethyl-, Carbethoxymethyl- oder Benzylgruppe und
$R_5$ eine Hydroxy-, Methoxy-, Carboxy-, Methoxycarbonyl-, Ethoxycarbonyl-, Aminocarbonyl-, Carboxymethoxy-, Methoxycarbonylmethoxy-, Ethoxycarbonylmethoxy-, Aminocarbonylmethoxy-, Methylaminocarbonylmethoxy-, 2-Hydroxy-ethoxy-, 2-Ethoxy-ethoxy-, 2-Phenethoxy-ethoxy-, 2-Amino-ethoxy-, 2-Methylamino-ethoxy-, 2-(l-Piperidino)-ethoxy-, 6-Hydroxy-n-hexoxy- oder 2-Carbomethoxy-l-methyl-ethenylgruppe darstellen, bedeuten.

Besonders bevorzugt sind jedoch die Verbindungen der allgemeinen Formel

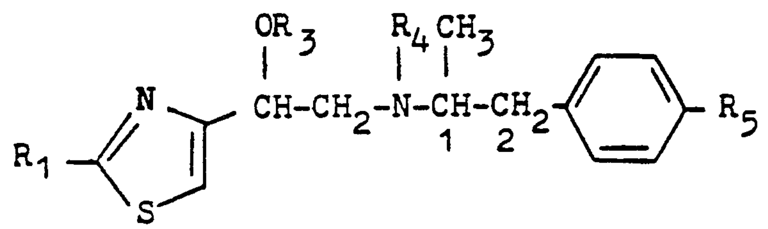

,(Ia)

in der
$R_1$ ein Chloratom, eine Methyl- oder Trifluormethylgruppe,
$R_3$ ein Wasserstoffatom oder $R_3$ und $R_4$ zusammen die Ethylen- oder Methoxycarbonylmethylengruppe,
$R_4$ ein Wasserstoffatom, eine Methyl-, 2-Hydroxy-ethyl-oder Carbethoxymethylgruppe und
$R_5$ eine Carboxymethoxy-, Carbomethoxymethoxy-, Ethoxycarbonylmethoxy-, Aminocarbonylmethoxy-, Methylaminocarbonylmethoxy-, 2-Methylamino-ethoxy-, 2-Hydroxy-ethoxy- oder 2-Carbomethoxy-l-methyl-ethenylgruppe bedeuten.

Erfindungsgemäß erhält man die neuen Verbindungen nach folgenden Verfahren:

a) Umsetzung einer Verbindung der allgemeinen Formel

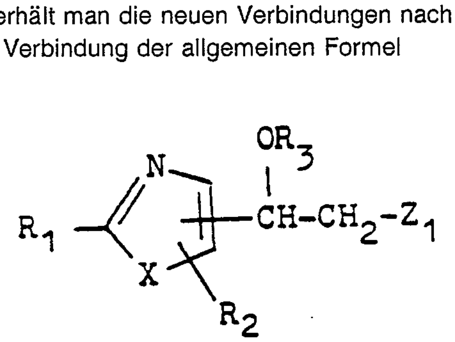

,(II)

mit einer Verbindung der allgemeinen Formel

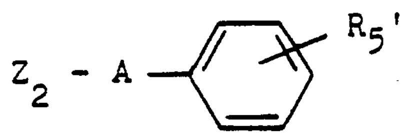

,(III)

in denen

$R_1$ bis $R_3$, A und X wie eingangs definiert sind,

$Z_1$ eine nukleophile Austrittsgruppe und

$Z_2$ eine $R_4$-NH-Gruppe oder

$Z_2$ eine nukleophile Austrittsgruppe und

$Z_1$ eine $R_4$-NH-Gruppe darstellen, wobei $R_4$ wie eingangs definiert ist, und

$R_5'$ die für $R_5$ eingangs erwähnten Bedeutungen besitzt, wobei jedoch $R_5'$ keine der eingangs erwähnten Alkoxycarbonylmethoxygruppen darstellen kann und eine im Rest $R_5$ enthaltene Carboxy-, Amino- oder Alkylaminogruppe durch einen Schutzrest geschützt sein kann, und gegebenenfalls anschließende Abspaltung eines verwendeten Schutzrestes.

Als nukleophile Austrittsgruppen kommen beispielsweise Halogenatome oder Sulfonyloxygruppen, z.B. ein Chlor-, Brom-oder Jodatom, die Methansulfonyloxy-, p-Toluolsulfonyloxy- oder Ethoxysulfonyloxygruppe,

als Schutzreste für eine Carboxygruppe kommen beispielsweise die Benzyl-, tert.Butyl-, Tetrahydropyranyl-, Trimethylsilyl-, Benzyloxymethyl-, 2-Chlorethyl- oder Methoxymethylgruppe oder eine Phenacylgruppe wie die Benzoylmethylgruppe und

als Schutzreste für eine Amino- oder Alkylaminogruppe die Acetyl-, Benzoyl-, tert.Butoxycarbonyl-, Benzyloxycarbonyl-, Ethoxycarbonyl- oder Benzylgruppe in Betracht.

Die Umsetzung wird zweckmäßigerweise in einem Lösungsmittel oder Lösungsmittelgemisch wie Aceton, Diethylether, Methylformamid, Dimethylformamid, Dimethylsulfoxid, Benzol, Chlorbenzol, Tetrahydrofuran, Benzol/Tetrahydrofuran, Dioxan oder in einem Überschuß der eingesetzten Verbindungen der allgemeinen Formeln II und/oder III und gegebenenfalls in Gegenwart eines säurebindenden Mittels, z.B. eines Alkoholats wie Kalium-tert.butylat, eines Alkalihydroxids wie Natrium- oder Kaliumhydroxid, eines Alkalicarbonats wie Kaliumcarbonat, eines Alkaliamids wie Natriumamid, eines Alkalihydrids wie Natriumhydrid, einer tertiären organischen Base wie Triethylamin, N,N-Diisopropylethylamin oder Pyridin, wobei die letzteren gleichzeitig auch als Lösungsmittel dienen können, oder eines Reaktionsbeschleunigers wie Kaliumjodid je nach der Reaktionsfähigkeit des nukleophil austauschbaren Restes zweckmäßigerweise bei Temperaturen zwischen 0 und 150°C, vorzugsweise bei Temperaturen zwischen 50 und 120°C, z.B. bei der Siedetemperatur des verwendeten Lösungsmittels, durchgeführt. Die Umsetzung kann jedoch auch ohne Lösungsmittel durchgeführt werden. Besonders vorteilhaft wird die Umsetzung jedoch in Gegenwart einer tertiären organischen Base oder eines Überschusses des eingesetzten Amins der allgemeinen Formel II oder III durchgeführt.

Die gegebenenfalls anschließende Abspaltung eines verwendeten Schutzrestes erfolgt vorzugsweise hydrolytisch in einem wässrigen Lösungsmittel, z.B. in Wasser, Isopropanol/Wasser, Tetrahydrofuran/Wasser oder Dioxan/Wasser, in Gegenwart einer Säure wie Salzsäure oder Schwefelsäure oder in Gegenwart einer Alkalibase wie Natriumhydroxid oder Kaliumhydroxid bei Temperaturen zwischen 0 und 100°C, vorzugsweise bei der Siedetemperatur des Reaktionsgemisches. Die Abspaltung eines Benzyl- oder Benzyloxycarbonylrestes erfolgt jedoch vorzugsweise hydrogenolytisch, z.B. mit Wasserstoff in Gegenwart eines Katalysators wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 50°C, vorzugsweise jedoch bei Raumtemperatur, und einem Wasserstoffdruck von 1 bis 7 bar, vorzugsweise jedoch von 3 bis 5 bar.

b) Reduktion einer gegebenenfalls im Reaktionsgemisch gebildeten Schiff'schen Base der allgemeinen Formel

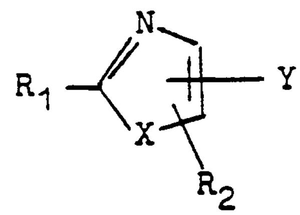

, (IV)

in der

$R_1$, $R_2$ und X wie eingangs definiert sind und

Y eine Gruppe der Formel

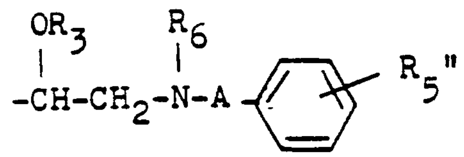

oder

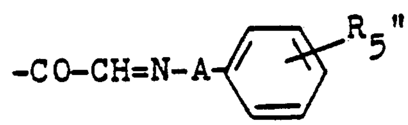

darstellen, wobei

$R_3$ und A wie eingangs definiert sind,

$R_5''$ die für $R_5$ eingangs erwähnten Bedeutungen besitzt, wobei jedoch eine im Rest $R_5$ enthaltene Amino- oder Alkylaminogruppe durch einen Schutzrest geschützt sein kann, und

$R_6$ zusammen mit einem Wasserstoffatom des benachbarten Kohlenstoffatomes des Restes A eine weitere Bindung darstellt, und gegebenenfalls anschließende Abspaltung eines verwendeten Schutzrestes.

Als Schutzreste für eine Amino- oder Alkylaminogruppe kommen beispielsweise die Acetyl-, Benzoyl-, tert.Butoxycarbonyl-, Benzyloxycarbonyl-, Ethoxycarbonyl- oder Benzylgruppe in Betracht.

Die Reduktion wird in einem geeigneten Lösungsmittel wie Methanol, Ethanol, Diethylether, Tetrahydrofuran, Dioxan, Essigsäureethylester oder Ethanol/Essigsäureethylester mit einem Metallhydrid wie Lithiumaluminiumhydrid, Diboran, Natriumcyanborhydrid oder Boran/Dimethylsulfid, vorzugsweise jedoch mit Natriumborhydrid, oder mit Wasserstoff in Gegenwart eines Hydrierungskatalysators wie Platin, Palladium/Kohle oder Raney-Nickel bei einem Wasserstoffdruck von l bis 5 bar oder mit Hydrazin in Gegenwart eines Hydrierungskatalysators wie Platin, Palladium/Kohle oder Raney-Nickel, bei Temperaturen zwischen 0 und 50 °C, vorzugsweise bei Raumtemperatur, durchgeführt. - Bei der Reduktion mit einem komplexen Metallhydrid wie Lithiumaluminiumhydrid, Diboran oder Boran/Dimethylsulfid, kann eine im Rest $R_5''$ vorhandene Carbonylfunktion zu einer Methylengruppe mitreduziert werden.

Die gegebenenfalls anschließende Abspaltung eines verwendeten Schutzrestes erfolgt vorzugsweise hydrolytisch in einem wässrigen Lösungsmittel, z.B. in Wasser, Isopropanol/Wasser, Tetrahydrofuran/Wasser oder Dioxan/Wasser, in Gegenwart einer Säure wie Salzsäure oder Schwefelsäure oder in Gegenwart einer Alkalibase wie Natriumhydroxid oder Kaliumhydroxid bei Temperaturen zwischen 0 und l00 °C, vorzugsweise bei der Siedetemperatur des Reaktionsgemisches. Die Abspaltung eines Benzyl- oder Benzyloxycarbonylrestes erfolgt jedoch vorzugsweise hydrogenolytisch, z.B. mit Wasserstoff in Gegenwart eines Katalysators wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 50 °C, vorzugsweise jedoch bei Raumtemperatur, und einem Wasserstoffdruck von l bis 7 bar, vorzugsweise jedoch von 3 bis 5 bar.

c) Zur Herstellung von Verbindungen der allgemeinen Formel I, in der $R_3$ und $R_4$ wie eingangs definiert sind, wobei jedoch $R_3$ und $R_4$ keine Ethylengruppe, in der die zum N-Atom benachbarte Methylengruppe durch eine Carbonylgruppe ersetzt ist, darstellen:

Reduktive Aminierung einer Carbonylverbindung der allgemeinen Formel

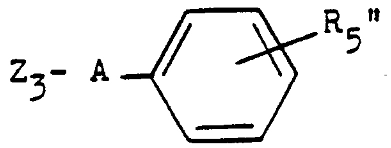

, (V)

in der
A wie eingangs definiert ist,
$R_5''$ die für $R_5$ eingangs erwähnten Bedeutungen besitzt, wobei jedoch eine im Rest $R_5$ enthaltene Amino- oder Alkylaminogruppe durch einen Schutzrest geschützt sein kann, und
$Z_3$ zusammen mit einem Wasserstoffatom des benachbarten Kohlenstoffatomes des Restes A ein Sauerstoffatom darstellt, mit einem Amin der allgemeinen Formel

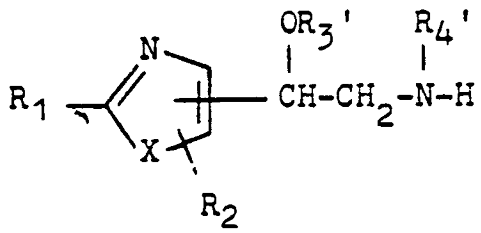

, (VI)

in der
$R_1$, $R_2$ und X wie eingangs definiert sind,
$R_3'$ und $R_4'$ die für $R_3$ und $R_4$ eingangs erwähnten Bedeutungen besitzen, wobei jedoch $R_3$ und $R_4$ zusammen keine Ethylengruppe, in der die zum N-Atom benachbarte Methylengruppe durch eine Carbonylgruppe ersetzt ist, bedeuten, in Gegenwart eines geeigneten Reduktionsmittels und gegebenenfalls anschließende Abspaltung eines verwendeten Schutzrestes.

Als Schutzreste für eine Amino- oder Alkylaminogruppe kommen beispielsweise die Acetyl-, Benzoyl-, tert.Butoxycarbonyl-, Benzyloxycarbonyl-, Ethoxycarbonyl- oder Benzylgruppe in Betracht.

Die reduktive Aminierung wird in einem geeigneten Lösungsmittel wie Methanol, Ethanol, Tetrahydrofuran, Dioxan oder Acetonitril in Gegenwart eines geeigneten Reduktionsmittels wie einem geeigneten komplexen Metallhydrid, vorzugsweise jedoch in Gegenwart von Natriumcyanborhydrid bei einem pH-Wert von 5 bis 7, bei Temperaturen zwischen 0 und 50 °C, vorzugsweise jedoch bei Raumtemperatur, durchgeführt.

Die gegebenenfalls anschließende Abspaltung eines verwendeten Schutzrestes erfolgt vorzugsweise hydrolytisch in einem wässrigen Lösungsmittel, z.B. in Wasser, Isopropanol/Wasser, Tetrahydrofuran/Wasser oder Dioxan/Wasser, in Gegenwart einer Säure wie Salzsäure oder Schwefelsäure oder in Gegenwart einer Alkalibase wie Natriumhydroxid oder Kaliumhydroxid bei Temperaturen zwischen 0 und l00 °C, vorzugsweise bei der Siedetemperatur des Reaktionsgemisches. Die Abspaltung eines Benzyl- oder Benzyloxycarbonylrestes erfolgt jedoch vorzugsweise hydrogenolytisch, z.B. mit Wasserstoff in Gegenwart eines Katalysators wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 50 °C, vorzugsweise jedoch bei Raumtemperatur, und einem Wasserstoffdruck von l bis 7 bar, vorzugsweise jedoch von 3 bis 5 bar.

d) Zur Herstellung von Verbindungen der allgemeinen Formel I, in der $R_3$ ein Wasserstoffatom darstellt: Reduktion einer gegebenenfalls im Reaktionsgemisch gebildeten Verbindung der allgemeinen Formel

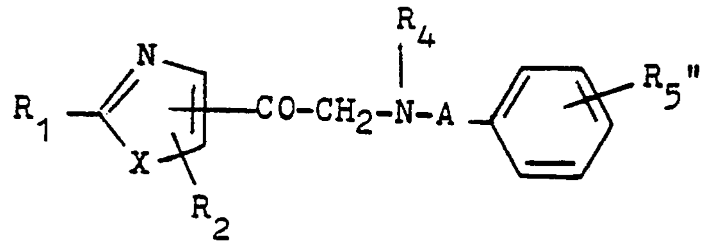

,(VII)

in der
A, X, $R_1$, $R_2$ und $R_4$ wie eingangs definiert sind und
$R_5''$ die für $R_5$ eingangs erwähnten Bedeutungen besitzt, wobei jedoch eine im Rest $R_5$ enthaltene Amino- oder Alkylaminogruppe durch einen Schutzrest geschützt sein kann, und gegebenenfalls anschließende Abspaltung eines verwendeten Schutzrestes.

Als Schutzreste für eine Amino- oder Alkylaminogruppe kommen beispielsweise die Acetyl-, Benzoyl-, tert.Butoxycarbonyl-, Benzyloxycarbonyl-, Ethoxycarbonyl- oder Benzylgruppe in Betracht.

Die Reduktion wird vorzugsweise in einem geeigneten Lösungsmittel wie Methanol, Ethanol, Diethylether oder Tetrahydrofuran in Gegenwart eines Metallhydrids wie Natriumborhydrid, Lithiumaluminiumhydrid, Diboran, Boran/Dimethylsulfid oder Natriumcyanborhydrid, vorzugsweise jedoch mit Natriumborhydrid in Methanol oder Ethanol, zwischen 0 und 40 °C, vorzugsweise jedoch bei Raumtemperatur, durchgeführt.

Bei der Reduktion mit einem komplexen Metallhydrid wie Lithiumaluminiumhydrid, Diboran oder Boran/Dimethylsulfid, kann eine im Rest $R_5''$ vorhandene Carbonylfunktion zu einer Methylengruppe mitreduziert werden.

Die gegebenenfalls anschließende Abspaltung eines verwendeten Schutzrestes erfolgt vorzugsweise hydrolytisch in einem wässrigen Lösungsmittel, z.B. in Wasser, Isopropanol/Wasser, Tetrahydrofuran/Wasser oder Dioxan/Wasser, in Gegenwart einer Säure wie Salzsäure oder Schwefelsäure oder in Gegenwart einer Alkalibase wie Natriumhydroxid oder Kaliumhydroxid bei Temperaturen zwischen 0 und 100 °C, vorzugsweise bei der Siedetemperatur des Reaktionsgemisches. Die Abspaltung eines Benzyl- oder Benzyloxycarbonylrestes erfolgt jedoch vorzugsweise hydrogenolytisch, z.B. mit Wasserstoff in Gegenwart eines Katalysators wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 50 °C, vorzugsweise jedoch bei Raumtemperatur, und einem Wasserstoffdruck von 1 bis 7 bar, vorzugsweise jedoch von 3 bis 5 bar.

e) Zur Herstellung von Verbindungen der allgemeinen Formel I, in der $R_3$ und $R_4$ zusammen eine Alkoxycarbonylmethylengruppe darstellen:
Umsetzung einer Verbindung der allgemeinen Formel

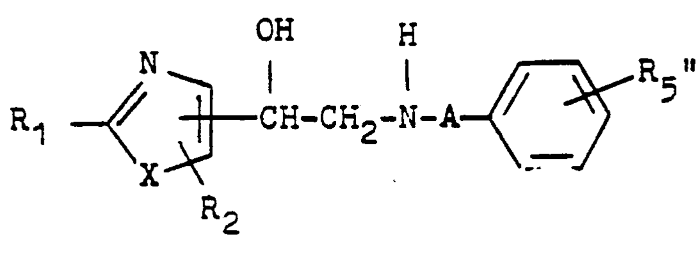

,(VIII)

in der
A, X, $R_1$ und $R_2$ wie eingangs definiert sind und
$R_5''$ die für $R_5$ eingangs erwähnten Bedeutungen besitzt, wobei jedoch eine im Rest $R_5$ enthaltene Amino- oder Alkylaminogruppe durch einen Schutzrest geschützt sein kann, mit einer Verbindung der allgemeinen Formel

$O = CH - COOR_7$ ,(IX)

in der

$R_7$ eine Alkylgruppe mit I bis 3 Kohlenstoffatomen darstellt, und gegebenenfalls anschließende Abspaltung eines verwendeten Schutzrestes.

Als Schutzreste für eine Amino- oder Alkylaminogruppe kommen beispielsweise die Trityl-, Fluorenylmethyloxycarbonyl-, Benzyloxycarbonyl- oder Benzylgruppe in Betracht.

Die Umsetzung wird zweckmäßigerweise in einem Lösungsmittel wie Methylenchlorid, Chloroform, Dioxan, Benzol oder Toluol und gegebenenfalls in Gegenwart eines wasserentziehenden Mittels wie p-Toluolsulfonsäure oder einem Molekularsieb bei Temperaturen zwischen 0 °C und der Siedetemperatur des verwendeten Lösungsmittels, vorzugsweise jedoch in Benzol oder Toluol durch azeotrope Destillation des Reaktionsgemisches, durchgeführt.

Die gegebenenfalls anschließende Abspaltung eines verwendeten Schutzrestes erfolgt vorzugsweise hydrolytisch unter milden sauren oder basischen Bedingungen in einem wässrigen Lösungsmittel, z.B. in Wasser, Isopropanol/Wasser, Tetrahydrofuran/Wasser oder Dioxan/Wasser, in Gegenwart einer Säure wie Essigsäure oder Trifluoressigsäure, oder unter nichtwäßrigen Bedingungen mit tert. organischen Basen wie Triethylamin oder Diazabicycloundecen (DBU), vorzugsweise jedoch hydrogenolytisch, z.B. mit Wasserstoff in Gegenwart eines Katalysators wie Palladium/Kohle in einem Lösungsmittel wie Methanol, Ethanol, Essigsäureethylester oder Eisessig und einem Wasserstoffdruck von I bis 7 bar, vorzugsweise jedoch von 3 bis 5 bar, bei Temperaturen zwischen 0 und 50 °C, vorzugsweise jedoch bei Raumtemperatur.

f) Zur Herstellung von Verbindungen der allgemeinen Formel I, in der $R_5$ eine Alkoxygruppe mit I bis 3 Kohlenstoffatomen, eine Alkoxygruppe mit I bis 6 Kohlenstoffatomen, die endständig durch eine Carboxy-, Alkoxycarbonyl-, Aminocarbonyl-, Alkylaminocarbonyl- oder Dialkylaminocarbonylgruppe substituiert ist, oder eine Alkoxygruppe mit 2 bis 7 Kohlenstoffatomen, die endständig durch eine Hydroxy-, Alkoxy-, Amino-, Alkylamino-, Dialkylamino-, Pyrrolidino-, Piperidino-oder Hexamethyleniminogruppe substituiert ist, darstellt:

Umsetzung einer Verbindung der allgemeinen Formel

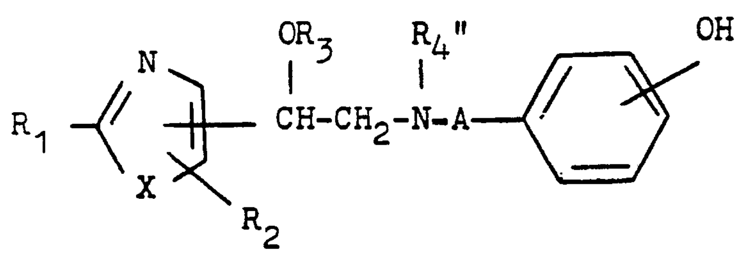

,(X)

in der

A, X und $R_1$ bis $R_3$ wie eingangs definiert sind und

$R_4''$ die für $R_4$ eingangs erwähnten Bedeutungen besitzt oder eine leicht abspaltbare Schutzgruppe für eine Aminogruppe darstellt, mit einer Verbindung der allgemeinen Formel

$Z_4 - R_8$ ,(XI)

in der

$R_8$ eine Alkylgruppe mit I bis 3 Kohlenstoffatomen, eine Alkylgruppe mit I bis 6 Kohlenstoffatomen, die endständig durch eine Carboxy-, Alkoxycarbonyl-, Aminocarbonyl-, Alkylaminocarbonyl- oder Dialkylaminocarbonylgruppe substituiert ist, oder eine Alkylgruppe mit 2 bis 7 Kohlenstoffatomen, die endständig durch eine Hydroxy-, Alkoxy-, Amino-, Alkylamino-, Dialkylamino-, Pyrrolidino-, Piperidino- oder Hexamethyleniminogruppe substituiert ist, und

$Z_4$ eine nukleophile Austrittsgruppe wie ein Halogenatom oder eine Sulfonyloxygruppe, z.B. ein Chlor-, Brom- oder Jodatom, die Methansulfonyloxy-, p-Toluolsulfonyloxy- oder Ethoxysulfonyloxygruppe, oder $Z_4$ zusammen mit einem β-Wasserstoffatom des Restes $R_8$ ein Sauerstoffatom darstellen, und gegebenenfalls anschließende Abspaltung eines verwendeten Schutzrestes.

Als leicht abspaltbare Schutzreste für das zentrale Stickstoffatom ($R_4''$) kommen beispielsweise die Acetyl-, Benzoyl-, tert.Butoxycarbonyl-, Benzyloxycarbonyl-, Ethoxycarbonyl- oder Benzylgruppe in Betracht.

Die Umsetzung wird zweckmäßigerweise in einem Lösungsmittel wie Diethylether, Tetrahydrofuran,

Dioxan, Methanol, Ethanol oder Dimethylformamid und vorzugsweise in Gegenwart eines säurebindenden Mittels wie Natriumhydroxid oder Kaliumtert.butylat, vorzugsweise jedoch in Gegenwart von Kaliumcarbonat oder Natriumhydrid, oder Pyridin, wobei eine organische Base wie Pyridin auch als Lösungsmittel dienen kann, oder zur Herstellung von 2-Hydroxy-ethoxy-Verbindungen der allgemeinen Formel I mit Ethylenoxid bei Temperaturen zwischen 0 und 100° C, vorzugsweise jedoch bei Temperaturen zwischen 20 und 80° C, durchgeführt.

Die gegebenenfalls anschließende Abspaltung eines verwendeten Schutzrestes erfolgt vorzugsweise hydrolytisch in einem wässrigen Lösungsmittel, z.B. in Wasser, Isopropanol/Wasser, Tetrahydrofuran/Wasser oder Dioxan/Wasser, in Gegenwart einer Säure wie Salzsäure oder Schwefelsäure oder in Gegenwart einer Alkalibase wie Natriumhydroxid oder Kaliumhydroxid bei Temperaturen zwischen 0 und 100° C, vorzugsweise bei der Siedetemperatur des Reaktionsgemisches. Die Abspaltung eines Benzyl- oder Benzyloxycarbonylrestes erfolgt jedoch vorzugsweise hydrogenolytisch, z.B. mit Wasserstoff in Gegenwart eines Katalysators wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 50° C, vorzugsweise jedoch bei Raumtemperatur, und einem Wasserstoffdruck von 1 bis 7 bar, vorzugsweise jedoch von 3 bis 5 bar.

g) Zur Herstellung von Verbindungen der allgemeinen Formel I, in der $R_3$ ein Wasserstoffatom darstellt:
Reduktion einer Verbindung der allgemeinen Formel

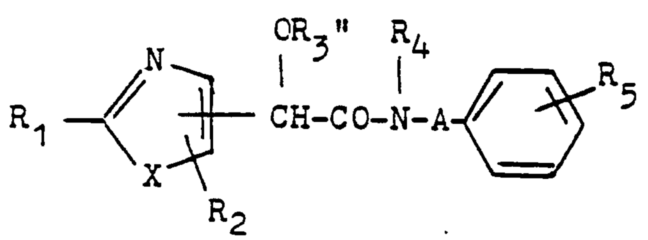
,(XII)

in der
A, X, $R_1$, $R_2$ und $R_4$ wie eingangs definiert sind und $R_3''$ ein Wasserstoffatom oder eine Alkylgruppe bedeutet.

Die Reduktion wird in einem geeigneten Lösungsmittel wie Diethylether oder Tetrahydrofuran mit einem Reduktionsmittel wie einem Metallhydrid, z.B. mit Lithiumaluminiumhydrid, Diboran oder Diboran/Dimethylsulfid, vorzugsweise jedoch mit Natriumborhydrid in Gegenwart von Eisessig oder Trifluoressigsäure, bei Temperaturen zwischen 0 und 50° C, vorzugsweise jedoch bei Temperaturen zwischen 10 und 25° C, durchgeführt. Bei der Reduktion kann eine gegebenenfalls in den Resten $R_4$ und $R_5$ vorhandene Carbonylfunktion gleichzeitig zu einer Methylengruppe mitreduziert werden. Desweiteren kann eine im Rest $R_4$ vorhandene Cyanogruppe zu einer Aminomethylengruppe mitreduziert werden.

h) Zur Herstellung von Verbindungen der allgemeinen Formel I, in der $R_5$ eine Alkoxycarbonyl-, Aminocarbonyl-, Alkylaminocarbonyl- oder Dialkylaminocarbonylgruppe darstellt oder enthält:
Umsetzung einer Verbindung der allgemeinen Formel

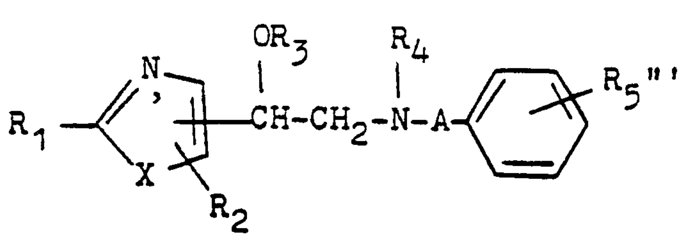
,(XIII)

in der
$R_1$ bis $R_4$, A und X wie eingangs definiert sind und
$R_5'''$ eine Carboxygruppe oder eine Alkoxygruppe mit 1 bis 6 Kohlenstoffatomen, welche endständig durch eine Carboxygruppe substituiert ist, darstellt, oder deren reaktionsfähige Derivate wie beispielsweise deren Ester, mit einer Verbindung der allgemeinen Formel

H - $R_9$ ,(XIV)

in der
$R_9$ eine Alkoxy-, Amino-, Alkylamino- oder Dialkylaminogruppe darstellt, wobei jeweils der Alkyl- oder Alkoxyteil I bis 3 Kohlenstoffatome enthalten kann.

Die Veresterung oder Amidierung wird zweckmäßigerweise in einem Lösungsmittel wie Methylenchlorid, Chloroform, Tetrachlorkohlenstoff, Ether, Tetrahydrofuran, Dioxan, Benzol, Toluol, Acetonitril oder Dimethylformamid, besonders vorteilhaft jedoch in einem Überschuß einer eingesetzten Verbindung der allgemeinen Formel XIV, z.B. in Methanol, Ethanol, n-Propanol, Isopropanol, Ammoniak, Methylamin, Ethylamin, Dimethylamin oder Diethylamin, gegebenenfalls in Gegenwart eines die Säure aktivierenden Mittels oder eines wasserentziehenden Mittels, z.B. in Gegenwart von Chlorameisensäureäthylester, Thionylchlorid, Phosphortrichlorid, Phosphorpentoxid, N,N'-Dicyclohexylcarbodiimid, N,N'-Dicyclohexylcarbodiimid/- N-Hydroxy-succinimid, N,N'-Carbonyldiimidazol oder N,N'-Thionyldiimidazol oder Triphenylphosphin/Tetrachlorkohlenstoff, oder eines die Aminogruppe aktivierenden Mittels, z.B. Phosphortrichlorid, und gegebenenfalls in Gegenwart einer anorganischen Base wie Natriumcarbonat oder einer tertiären organischen Base wie Triethylamin oder Pyridin, welche gleichzeitig als Lösungsmittel dienen können, bei Temperaturen zwischen -25° C und 250° C, vorzugsweise jedoch bei Temperaturen zwischen -I0° C und der Siedetemperatur des verwendeten Lösungsmittels, durchgeführt.

i) Zur Herstellung von Verbindungen der allgemeinen Formel I, in der $R_4$ eine gegebenenfalls durch eine Phenyl-, Carboxy-, Alkoxycarbonyl- oder Cyanogruppe oder in 2- oder 3-Stellung durch eine Hydroxygruppe substituierte Alkylgruppe oder eine Alkenylgruppe darstellt:
Umsetzung einer Verbindung der allgemeinen Formel

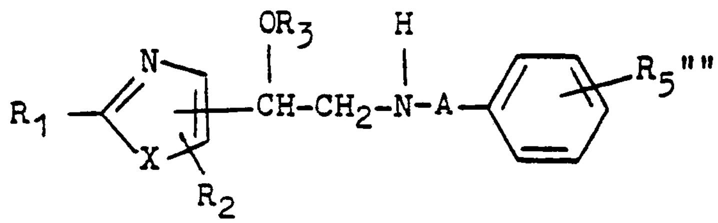

, (XV)

in der
A, X und $R_1$ bis $R_3$ wie eingangs definiert sind und
$R_5''''$ die für $R_5$ eingangs erwähnten Bedeutungen besitzt, wobei jedoch eine im Rest $R_5$ enthaltene Amino- oder Alkylaminogruppe erforderlichenfalls durch einen Schutzrest geschützt sein kann, mit einer Verbindung der allgemeinen Formel

$Z_5$ - $R_4'''$ ,(XVI)

in der
$R_4'''$ mit Ausnahme des Wasserstoffatomes die für $R_4$ eingangs erwähnten Bedeutungen besitzt und
$Z_5$ eine nukleophile Austrittsgruppe wie ein Halogenatom oder eine Sulfonyloxygruppe, z.B. ein Chlor-, Brom- oder Jodatom, die Methansulfonyloxy-, p-Toluolsulfonyloxy- oder Ethoxysulfonyloxygruppe, oder $Z_5$ zusammen mit einem α- oder β-Wasserstoffatom des Alkylrestes $R_4'''$ ein Sauerstoffatom bedeuten, und gegebenenfalls anschließende Abspaltung eines Schutzrestes.

Die Alkylierung wird in einem geeigneten Lösungsmittel wie Methanol, Ethanol, Diethylether, Aceton, Methylenchlorid, Tetrahydrofuran, Dioxan, Dimethylformamid oder Dimethylsulfoxid, gegebenenfalls in Gegenwart einer Base wie Natriumcarbonat, Kalium-tert.butylat, Triethylamin oder Pyridin, wobei die beiden letzteren gleichzeitig auch als Lösungsmittel dienen können, mit einem geeigneten Alkylierungsmittel wie Methyljodid, Dimethylsulfat, Ethylbromid, Diethylsulfat, Benzylchlorid, n-Propylbromid, Isopropylbromid, Allylbromid, Ethylenoxid, 2-Hydroxy-ethylbromid, 2-Cyano-ethylbromid oder Formaldehyd/Ameisensäure oder in Gegenwart von Natriumcyanborhyrid, falls eine entsprechende Carbonylverbindung eingesetzt wird, bei Temperaturen zwischen 0 und I00° C, durchgeführt.

Die gegebenenfalls anschließende Abspaltung eines verwendeten Schutzrestes erfolgt vorzugsweise hydrolytisch in einem wässrigen Lösungsmittel, z.B. in Wasser, Isopropanol/Wasser,

Tetrahydrofuran/Wasser oder Dioxan/Wasser, in Gegenwart einer Säure wie Salzsäure oder Schwefelsäure oder in Gegenwart einer Alkalibase wie Natriumhydroxid oder Kaliumhydroxid bei Temperaturen zwischen 0 und 100° C, vorzugsweise bei der Siedetemperatur des Reaktionsgemisches. Die Abspaltung eines Benzyl- oder Benzyloxycarbonylrestes erfolgt jedoch vorzugsweise hydrogenolytisch, z.B. mit Wasserstoff in Gegenwart eines Katalysators wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 50° C, vorzugsweise jedoch bei Raumtemperatur, und einem Wasserstoffdruck von 1 bis 7 bar, vorzugsweise jedoch von 3 bis 5 bar.

k) Zur Herstellung von Verbindungen der allgemeinen Formel I, in der $R_3$ und $R_4$ zusammen eine Ethylengruppe darstellen:

Reduktion einer gegebenenfalls im Reaktionsgemisch gebildeten Verbindung der allgemeinen Formel

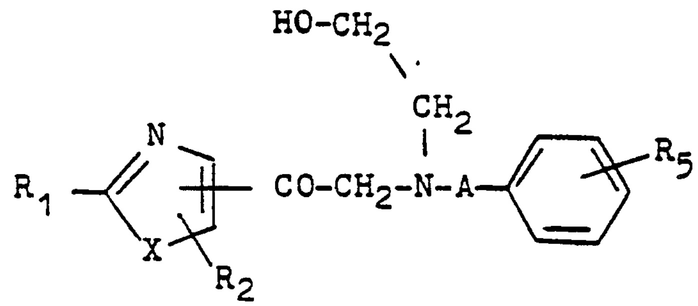

,(XVII)

in der

A, X, $R_1$, $R_2$ und $R_5$ wie eingangs definiert sind, oder dessen cyclischen Halbacetals.

Die Umsetzung wird vorzugsweise in einem Lösungsmittel wie Methylenchlorid, Chloroform oder Trifluoressigsäure mit einem geeigneten Hydrid wie einem komplexen Metallhydrid, z.B. mit Natriumborhydrid, mit katalytisch angeregtem Wasserstoff, z.B. mit Wasserstoff in Gegenwart von Platin, oder einem Trialkylsilan, z.B. mit Triethylsilan, gegebenenfalls in Gegenwart einer Säure wie Bortrifluorid, z.B. in Gegenwart von Bortrifluorid-Etherat, bei Temperaturen zwischen 0 und 60° C, vorzugsweise jedoch in Trifluoressigsäure als Lösungsmittel und bei Raumtemperatur, durchgeführt.

l) Zur Herstellung von Verbindungen der allgemeinen Formel I in der $R_3$ und $R_4$ zusammen eine Ethylengruppe, in der die zum N- oder O-Atom benachbarte Methylengruppe durch eine Carbonylgruppe ersetzt ist, bedeuten:

Umsetzung einer Verbindung der allgemeinen Formel

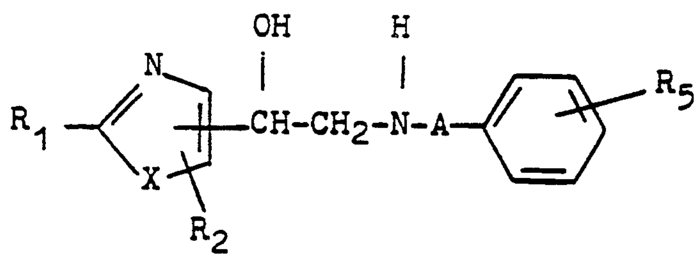

,(XVIII)

in der

A, X, $R_1$, $R_2$ und $R_5$ wie eingangs definiert sind, mit einem Halogenacetylhalogenid oder Halogenessigester.

Die Umsetzung wird zweckmäßigerweise in einem Lösungsmittel wie Methylenchlorid, Tetrahydrofuran, Dioxan, Benzol oder Toluol oder einem Überschuß des eingesetzten Acylierungsmittels gegebenenfalls in Gegenwart eines säurebindenden Mittels wie Kaliumcarbonat, einem Alkalimetallhydrid wie Natriumhydrid oder in Gegenwart einer tertiären organischen Base wie Triethylamin oder Pyridin, wobei die beiden letzteren gleichzeitig auch als Lösungsmittel dienen können, bei Temperaturen zwischen 0 und 100° C, vorzugsweise jedoch bei Temperaturen zwischen der Raumtemperatur und der Siedetemperatur des Reaktionsgemisches, durchgeführt. Wird bei der Umsetzung ein Halogenessigester in Gegenwart von Kaliumkarbonat verwendet, so erhält man bevorzugt ein entsprechendes Lacton.

m) Zur Herstellung von Verbindungen der allgemeinen Formel I, in der $R_3$ und $R_4$ zusammen eine Ethylengruppe bedeuten:
Reduktion einer Verbindung der allgemeinen Formel

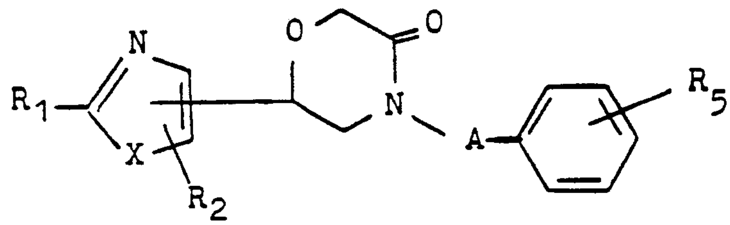

, (XIX)

in der
A, X, $R_1$, $R_2$ und $R_5$ wie eingangs definiert sind.

Die Reduktion wird in einem geeigneten Lösungsmittel wie Diethylether oder Tetrahydrofuran mit einem Reduktionsmittel wie einem Metallhydrid, z.B. mit Lithiumaluminiumhydrid oder Natriumborhydrid in Gegenwart von Eisessig oder Trifluoressigsäure, vorzugsweise jedoch mit Phosphoroxychlorid/Natriumborhydrid, Diboran oder Diboran/Dimethylsulfid bei Temperaturen zwischen 0 und 50°C, vorzugsweise jedoch bei Temperaturen zwischen 10 und 25°C, durchgeführt. Bei der Reduktion kann eine gegebenenfalls im Rest $R_5$ vorhandene Carbonylfunktion gleichzeitig zu einer Methylengruppe mitreduziert werden.

n) Zur Herstellung von Verbindungen der allgemeinen Formel I, in der $R_3$ und $R_4$ zusammen eine Ethylengruppe, in welcher die zum O-Atom benachbarte Methylengruppe durch eine Carbonylgruppe ersetzt ist, darstellen:
Cyclisierung einer gegebenenfalls im Reaktionsgemisch gebildeten Verbindung der allgemeinen Formel

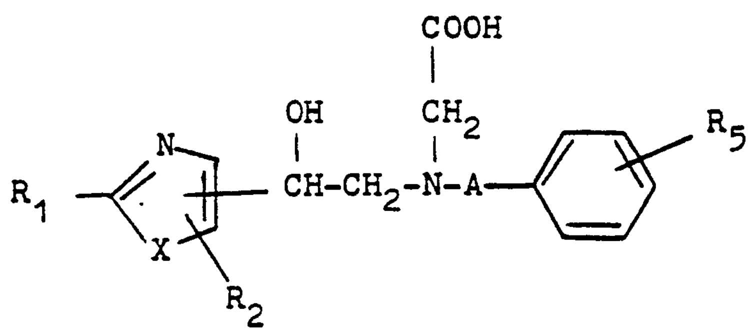

, (XX)

in der
A, X, $R_1$, $R_2$ und $R_5$ wie eingangs definiert sind, oder deren reaktionsfähige Derivate wie deren Ester oder Halogenide.

Die Cyclisierung wird in einem geeigneten Lösungsmittel wie Methylenchlorid, Chloroform, Benzol, Toluol, Tetrahydrofuran, Aceton, Methylethylketon oder Dioxan gegebenenfalls in Gegenwart eines wasserentziehenden Mittels wie Thionylchlorid, Phosphortrichlorid, N,N'-Dicyclohexylcarbodiimid, N,N'-Carbonyldimidazol oder N,N'-Thionyldiimidazol bei Temperaturen zwischen 0 und 100°C, vorzugsweise jedoch bei der Siedetemperatur des Reaktionsgemisches, durchgeführt.

o) Zur Herstellung von Verbindungen der allgemeinen Formel I, in der $R_1$ ein Wasserstoff- oder Halogenatom, eine Trifluormethyl- oder Alkylgruppe und $R_3$ ein Wasserstoffatom darstellen:
Umsetzung einer Verbindung der allgemeinen Formel

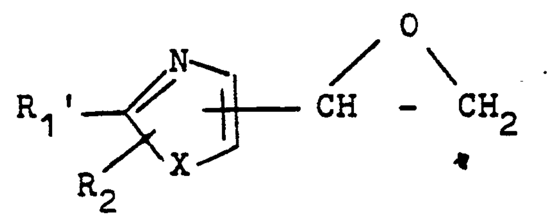

, (XXI)

in der
X und $R_2$ wie eingangs definiert sind und
$R_1'$ ein Wasserstoff- oder Halogenatom, eine Trifluormethyl- oder Alkylgruppe darstellt, mit einem Amin der allgemeinen Formel

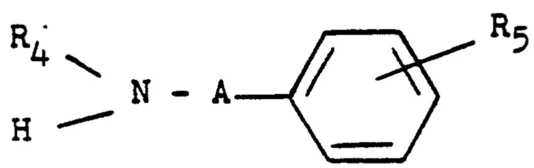

, (XXII)

in der
A, $R_4$ und $R_5$ wie eingangs definiert sind.

Die Umsetzung wird in einem geeigneten Lösungsmittel, z.B. in einem polaren Lösungsmittel wie Ethanol oder Isopropanol oder in einem aprotischen Lösungsmittel wie Dimethylformamid oder Dimethylsulfoxid, bei Temperaturen zwischen 0 und 150° C, vorzugsweise jedoch bei Temperaturen zwischen 50 und 100° C, durchgeführt.

Erhält man erfindungsgemäß eine Verbindung der allgemeinen Formel I, in der $R_5$ eine Alkoxycarbonyl-, Aminocarbonyl-, Alkylaminocarbonyl- oder Dialkylaminocarbonylgruppe darstellt oder enthält und/oder $R_1$ eine durch eine Alkanoyl-oder Benzoylgruppe substituierte Aminogruppe darstellt, so kann diese mittels Hydrolyse in eine entsprechende Verbindung der allgemeinen Formel I, in der $R_5$ eine Carboxygruppe darstellt oder enthält und/oder $R_1$ eine Aminogruppe darstellt, übergeführt werden oder
eine Verbindung der allgemeinen Formel I, in der $R_5$ eine durch eine Carboxy-, Alkoxycarbonyl-, Aminocarbonyl-, Alkylaminocarbonyl- oder Dialkylaminocarbonylgruppe substituierte Alkoxygruppe darstellt, so kann diese mittels Reduktion durch ein geeignetes Metallhydrid in eine Verbindung der allgemeinen Formel I, in der der vorstehend erwähnte substituierte Alkoxyrest anstelle der Carbonylgruppe eine Methylengruppe enthält, übergeführt werden oder
eine Verbindung der allgemeinen Formel I, in der $R_3$ eine Alkylgruppe und/oder $R_5$ eine der eingangs erwähnten Alkoxygruppen darstellt, so kann diese mittels Etherspaltung in eine entsprechende Verbindung der allgemeinen Formel I, in der $R_3$ ein Wasserstoffatom und/oder $R_5$ eine Hydroxygruppe oder eine durch eine Hydroxygruppe substituierte Alkoxygruppe darstellt, übergeführt werden.

Die nachträgliche Hydrolyse wird entweder in Gegenwart einer Säure wie Salzsäure, Schwefelsäure, Phosphorsäure oder Trichloressigsäure oder in Gegenwart einer Base wie Natriumhydroxid oder Kaliumhydroxid in einem geeigneten Lösungsmittel wie Wasser, Methanol, Äthanol, Äthanol/Wasser, Wasser/Isopropanol oder Wasser/Dioxan bei Temperaturen zwischen -10° C und 120° C, z.B. bei Temperaturen zwischen Raumtemperatur und der Siedetemperatur des Reaktionsgemisches, durchgeführt.

Die nachträgliche Reduktion wird in einem geeigneten Lösungsmittel wie Diethylether oder Tetrahydrofuran mit einem geeigneten Metallhydrid, z.B. mit Lithiumaluminiumhydrid, Diboran oder Diboran/Dimethylsulfid, vorzugsweise jedoch mit Natriumborhydrid in Gegenwart von Eisessig oder Trifluoressigsäure, bei Temperaturen zwischen 0 und 50° C, vorzugsweise jedoch bei Temperaturen zwischen 10 und 25° C, durchgeführt.

Die nachträgliche Etherspaltung wird in Gegenwart einer Säure wie Chlorwasserstoff, Bromwasserstoff, Schwefelsäure oder Bortribromid in einem geeigneten Lösungsmittel wie Methanol, Ethanol, Wasser/Isopropanol, Methylenchlorid, Chloroform oder Tetrachlorkohlenstoff bei Temperaturen zwischen -30° C und der Siedetemperatur des Reaktionsgemisches durchgeführt.

Bei der nachträglichen Hydrolyse, Reduktion oder Etherspaltung können Verbindungen der allgemeinen Formel I, in der $R_3$ und $R_4$ zusammen eine Ethylengruppe, in welcher die zum O-Atom benachbarte Methylengruppe durch eine Carbonylgruppe ersetzt ist, bedeutet, gleichzeitig gespalten werden.

Wie bereits eingangs erwähnt, können die neuen Verbindungen in Form ihrer Enantiomeren, Enantiomerengemische oder Racemate, oder sofern sie mindestens 2 asymmetrische Kohlenstoffatome enthalten, auch in Form ihrer Diastereomeren bzw. Diastereomerengemische vorliegen.

So lassen sich die erhaltenen Verbindungen der allgemeinen Formel I, welche nur ein optisch aktives Zentrum enthalten, nach an sich bekannten Methoden (siehe Allinger N. L. und Elich W. L. in "Topics in Stereochemistry", Vol. 6, Wiley Interscience, 1971) in ihre optischen Antipoden auftrennen, z.B. durch Umkristallisieren aus einem optisch aktiven Lösungsmittel oder durch Umsetzen mit einer, mit der racemischen Verbindung Salze bildenden optisch aktiven Substanz, insbesondere Säuren, und Trennen des auf diese Weise erhaltenen Salzgemisches, z.B. auf Grund von verschiedenen Löslichkeiten, in die diastereomeren Salze, aus denen die freien Antipoden durch Einwirkung geeigneter Mittel freigesetzt werden können. Besonders gebräuchliche, optisch aktive Säuren sind z.B. die D- und L-Formen von Weinsäure, Di-o-Toluolweinsäure, Äpfelsäure, Mandelsäure, Camphersulfonsäure, Glutaminsäure, Asparaginsäure oder Chinasäure.

Desweiteren lassen sich die erhaltenen Verbindungen der allgemeinen Formel I mit mindestens 2 asymmetrischen Kohlenstoffatomen auf Grund ihrer physikalischen-chemischen Unterschiede nach an sich bekannten Methoden, z.B. durch Chromatographie und/oder fraktionierte Kristallisation, in ihre Diastereomeren auftrennen. Ein so erhaltenes Enantiomerenpaar läßt sich anschließend in seine optischen Antipoden, wie oben beschrieben, auftrennen. Enthält beispielsweise eine Verbindung der allgemeinen Formel I zwei optisch aktive Kohlenstoffatome, so erhält man die entsprechenden (R R′, S S′)- und (R S′, S R′)-Formen.

Die als Ausgangsstoffe verwendeten Verbindungen, welche selbstverständlich auch in ihren optisch reinen Formen verwendet werden können, erhält man nach literaturbekannten Verfahren (siehe "Thiazole and its Derivatives" in Heterocyclic Compounds, Vol. 34, und Advances in Heterocyclic Chemistry, Vol 17, ab S. 100 (1974)) bzw. sind literaturbekannt. Diese liegen teilweise nur im Reaktionsgemisch vor und können daher teilweise nicht isoliert werden.

Die als Ausgangsstoffe verwendeten Verbindungen der allgemeinen Formel II, in der $Z_1$ eine nukleophile Austrittsgruppe darstellt, erhält man durch Reduktion einer entsprechenden Acetylverbindung, z.B. mit einem komplexen Metallhydrid, und gegebenenfalls anschließender Alkylierung.

Eine Verbindung der allgemeinen Formel IV, welche nicht isoliert werden muß, erhält man durch Umsetzung einer Verbindung der allgemeinen Formel

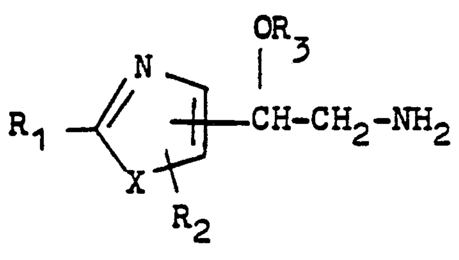

,(XXIII)

mit einer Carbonylverbindung der allgemeinen Formel

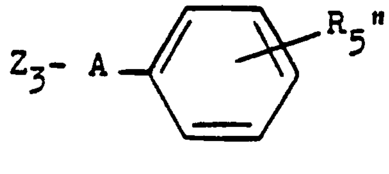

,(V)

oder durch Umsetzung eines Amins der allgemeinen Formel

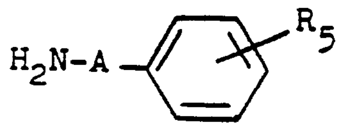
, (XXIV)

mit einem Glyoxal der allgemeinen Formel

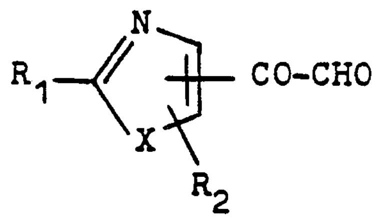
, (XXV)

in denen
$R_1$ bis $R_3$, $R_5$, A und X wie eingangs definiert sind,
$R_5''$ die für $R_5$ eingangs erwähnten Bedeutungen besitzt, wobei jedoch eine im Rest $R_5$ enthaltene Amino- oder Alkylaminogruppe durch einen Schutzrest geschützt sein kann, und
$Z_3$ zusammen mit einem Wasserstoffatom des benachbarten Kohlenstoffatomes des Restes A ein Sauerstoffatom darstellen, in Gegenwart von Natriumcyanborhydrid in einem geeigneten Lösungsmittel wie Methylenchlorid, Chloroform, Dioxan, Benzol oder Toluol und gegebenenfalls in Gegenwart eines wasserentziehenden Mittels wie p-Toluolsulfonsäure oder einem Molekularsieb bei Temperaturen zwischen 0°C und der Siedetemperatur des verwendeten Lösungsmittels, vorzugsweise jedoch in Benzol oder Toluol durch azeotrope Destillation des Reaktionsgemisches.

Eine für die Herstellung der Ausgangsverbindungen erforderliche Verbindung der allgemeinen Formel

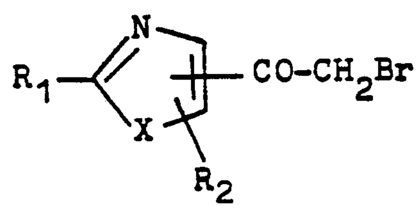
, (XXVI)

in der
$R_1$, $R_2$ und X wie eingangs definiert sind, erhält man durch Bromierung der entsprechenden Acetylverbindungen in Eisessig oder Bromwasserstoff/Eisessig bei Temperaturen zwischen 20 und 100°C oder auch, wenn $R_2$ in 5-Stellung ein Wasserstoffatom und X ein Schwefelatom darstellen, durch Ringschluß der entsprechenden Thioamide mit Dibromdiacetyl in einem Lösungsmittel wie Diethylether oder Acetonitril. Ferner erhält man beispielsweise die oben erwähnten Acetylverbindungen, wenn $R_2$ in 4-Stellung eine Methylgruppe und X ein Schwefelatom darstellt, durch Umsetzung eines entsprechenden Thioamids mit 3-Chlor-acetylaceton in Wasser, Ethanol, Wasser/Ethanol oder in der Schmelze (siehe Z. Chem. 9, 187 (1969)) oder, wenn $R_2$ in 5-Stellung eine Methylgruppe und X ein Sauerstoff- oder Schwefelatom darstellen, durch Umsetzung eines entsprechenden Acylamino-acetylacetons mit wasserabspaltenden Mitteln (siehe Chem. Ber. 84, 96 (1951) und Chem. Ber. 93, 1998 (1960)) bzw. mit Phosphorpentasulfid oder mit 2,4-Bis(4-methoxyphenyl)-1,3-dithia-2,4-diphosphetan-2,4-disulfid.

Ein Aminoketon der allgemeinen Formel VII erhält man im Reaktionsgemisch durch Umsetzung eines entsprechenden Bromacetylderivates mit einem entsprechenden Amin bzw. mit Urotropin und anschließender Hydrolyse.

Die als Ausgangsstoffe verwendeten Verbindungen der allgemeinen Formeln VIII, X, XIII, XV und XX erhält man zweckmäßigerweise durch Alkylierung eines entsprechenden Amins.

Die als Ausgangsstoffe verwendeten Verbindungen der allgemeinen Formel XII erhält man durch Acylierung eines entsprechenden Amins.

Eine als Ausgangsstoff verwendete Verbindung der allgemeinen Formel XXI erhält man beispielsweise durch Umsetzung eines entsprechenden Bromhydrins mit wässriger Kalilauge oder durch Umsetzung eines entsprechenden Aldehyds mit Dimethylsulfoniummethylid bei 0 °C in Dimethylsulfoxid/Tetrahydrofuran.

Ein entsprechendes Glyoxal erhält man beispielsweise durch Umsetzung einer entsprechenden Bromacetylverbindung der allgemeinen Formel XXVI mit Dimethylsulfoxid bei Raumtemperatur.

Desweiteren können die erhaltenen Verbindungen der allgemeinen Formel I in ihre Säureadditionssalze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Fumarsäure, Bernsteinsäure, Milchsäure, Zitronensäure, Weinsäure oder Maleinsäure in Betracht.

Wie bereits eingangs erwähnt, stellen die neuen Verbindungen der allgemeinen Formel I, in denen $R_3$ und $R_4$ zusammen eine Ethylengruppe, in der die mit dem N-Atom verknüpfte Methylengruppe durch eine Carbonylgruppe ersetzt ist, bedeuten, wertvolle Zwischenprodukte zur Herstellung der Morpholine der allgemeinen Formel I dar.

Die übrigen neuen Verbindungen der allgemeinen Formel I (ausgenommen die Lactame), deren Enantiomeren, Enantiomerengemische oder Racemate, oder sofern sie mindestens 2 asymmetrische Kohlenstoffatome enthalten, auch deren Diastereomeren bzw. Diastereomerengemische und deren Säureadditionssalze, insbesondere für die pharmazeutische Anwendung deren physiologisch verträgliche Säureadditionssalze, weisen wertvolle pharmakologische Eigenschaften auf, neben einer Hemmwirkung auf die Plättchenaggregation insbesondere eine Wirkung auf den Stoffwechsel, vorzugsweise eine blutzuckersenkende und körperfettreduzierende Wirkung, sowie eine senkende Wirkung auf die atherogenen $\beta$-Lipoproteine VLDL und LDL. Außerdem weisen einige der vorstehend erwähnten Verbindungen auch eine anabole Wirkung auf. - Hierbei hat sich dasjenige Diastereomer, falls $R_1$ eine Trifluormethylgruppe darstellt, als besonders bevorzugt erwiesen, dessen Proton in der 5-Stellung des Thiazolringes in der Morpholinreihe im $CDCl_3/CD_3OD$-NMR-Spektrum sowie in der Ethanolaminreihe im $CDCl_3$-NMR-Spektrum bei tieferem Feld liegt.

Beispielsweise wurden die nachfolgenden Verbindungen auf ihre biologischen Eigenschaften wie folgt untersucht:

A = N-[2-(4-Carbomethoxyphenyl)-l-methylethyl]-2-hydroxy-2-(2-trifluormethyl-thiazol-4-yl)ethanamin,

B = N-[2-(4-Carbomethoxymethoxyphenyl)-l-methylethyl]-2-hydroxy-2-(2-trifluormethyl-thiazol-4-yl)-ethanamin,

C = N-[2-(4-Aminocarbonylmethoxyphenyl)-l-methylethyl]-2-hydroxy-2-(2-trifluormethyl-thiazol-4-yl)-ethanamin,

D = N-[2-(4-Methylaminocarbonylmethoxyphenyl)-l-methylethyl]-2-hydroxy-2-(2-trifluormethyl-thiazol-4-yl)ethanamin,

E = N-[2-(4-Carboxyphenyl)-l-methylethyl]-2-hydroxy-2-(2-trifluormethyl-thiazol-4-yl)ethanamin,

F = N-[2-(4-Carbomethoxymethoxyphenyl)-l-methylethyl]-2-(2-trifluormethyl-thiazol-4-yl)morpholin,

G = 3-[2-(4-Carbomethoxyphenyl)-l-methylethyl]-5-(2-trifluormethyl-thiazol-4-yl)-2-oxazolidincarbonsäure-methylester (Diastereomerenpaare A und B),

H = 3-[2-(4-Carbomethoxyphenyl)-l-methylethyl]-5-(2-trifluormethyl-thiazol-4-yl)-2-oxazolidincarbonsäure-methylester (Diastereomerenpaare C und D),

I = 3-[2-(4-Carbomethoxymethoxyphenyl)-l-methylethyl]-5-(2-trifluormethyl-thiazol-4-yl)-2-oxazolidincarbonsäure-methylester (Diastereomerenpaare A und B),

J = 3-[2-(4-Carbomethoxymethoxyphenyl)-l-methylethyl]-5-(2-trifluormethyl-thiazol-4-yl)-2-oxazolidincarbonsäure-methylester (Diastereomerenpaare C und D),

K = N-[2-(4-Carbomethoxymethoxyphenyl)-l-methylethyl]-N-methyl-2-hydroxy-2-(2-trifluormethyl-thiazol-4-yl)ethanamin,

L = N-[2-(4-Aminocarbonylmethoxyphenyl)-l-methylethyl]-2-hydroxy-2-(2-methyl-thiazol-4-yl)-ethanamin,

M = N-[2-(4-Carbomethoxymethoxyphenyl)-l-methylethyl]-2-hydroxy-2-(2-methyl-thiazol-4-yl)-ethanamin,

N = N-[2-(4-Carbomethoxymethoxyphenyl)-l-methylethyl]-2-hydroxy-2-(thiazol-4-yl)ethanamin,

O = N-[2-(4-Carboxymethoxyphenyl)-l-methylethyl]-2-(2-trifluormethyl-thiazol-4-yl)morpholin,

P = N-[2-(4-Carbomethoxymethoxyphenyl)-l-methylethyl]-2-hydroxy-2-(2-chlor-thiazol-4-yl)-ethanamin,

Q = N-[2-(4-(2-Hydroxy-ethoxy)phenyl)-l-methylethyl]-2-hydroxy-2-(2-trifluormethyl-thiazol-4-yl)-ethanamin und

R = 3-[2-(4-(2-Carbomethoxy-l-methylethenyl)phenyl)-l-methylethyl]-5-(2-trifluormethyl-thiazol-4-yl)-2-

oxazolidincarbonsäure-methylester

I. Antidiabetische Wirkung:

Die antidiabetische Wirkung der erfindungsgemäßen Verbindungen läßt sich als eine blutzuckersenkende Wirkung bei Versuchstieren messen. Die zu untersuchenden Substanzen wurden dazu in 1,5%iger Methylzellulose suspendiert und weiblichen Mäusen eigener Zucht mittels Schlundsonde appliziert. 30 Minuten später wurde 1 g Glukose pro kg Körpergewicht in Wasser gelöst und subkutan appliziert. Weitere 30 Minuten später wurde aus dem retroorbitalen Venenplexus Blut entnommen. Aus dem Serum wurde Glukose mit der Hexokinase-Methode mit Hilfe eines Analysenphotometers bestimmt.

In der nachstehenden Tabelle sind die bei dieser Versuchsanordnung beobachteten Blutzuckersenkungen in % einer mitgeführten Kontrollgruppe zusammengestellt. Die statistische Auswertung erfolgt nach dem t-Test nach Student mit p = 0,05 als Signifikanzgrenze.

| Verbindung | % Änderung vom Wert der Kontrollgruppe Dosis [mg/kg] | | | | |
|---|---|---|---|---|---|
| | 0,3 | 1 | 3 | 10 | 30 |
| A | | -40 | | | -63 |
| B | | -64 | | -73 | |
| C | | | -68 | | -76 |
| D | | -65 | | | -68 |
| E | | | -57 | | -70 |
| F | | | -61 | -64 | |
| G | | | -56 | | |
| H | | | -59 | | |
| I | | -68 | | -72 | |
| J | -36 | | | -60 | |
| K | | -42 | | | -60 |
| L | | | -61 | | |
| M | | -32 | | -58 | |
| N | | | -23 | | |
| O | | | -60 | | |
| P | -52 | | | | |
| Q | | -70 | | | |
| R | | | -56 | | |

2. Antiadipöse Wirkung:

Die antiadipöse Wirkung der erfindungsgemäßen Verbindungen wurde in zwei Versuchsanordnungen nachgewiesen:

a) In der ersten Versuchsanordnung wurde die Steigerung der Lipolyse am Anstieg des Glyzerins im

Serum gemessen. Der Versuchsablauf ist identisch mit der zur Testung auf blutzuckersenkende Wirkung vorstehend beschriebenen Versuchsanordnung. Glyzerin wurde in einem kombinierten enzymatischkolorimetrischen Test mit Hilfe eines Analysenphotometers bestimmt. Die Ergebnisse sind in der nachfolgenden Tabelle in % einer mitgeführten Kontrollgruppe aufgeführt.

| Verbindung | % Änderung vom Wert der Kontrollgruppe Dosis [mg/kg] | | | | |
|---|---|---|---|---|---|
| | 0,3 | 1 | 3 | 10 | 30 |
| A | | | 88 | | |
| B | | 312 | | 326 | |
| C | | | 504 | | 732 |
| D | | 433 | | | 439 |
| E | | | 226 | | 305 |
| F | | | 413 | 430 | |
| G | | | 209 | | |
| H | | | 168 | | |
| I | | 204 | | 228 | |
| J | 260 | | | 391 | |
| K | | 283 | | | 416 |
| L | | | 220 | | |
| M | | 307 | | 453 | |
| N | | | (8) | | |
| O | | | 336 | | |
| P | 217 | | | | |
| Q | | 221 | | | |
| R | | | 750 | | |

( ) = nicht signifikant ($p \geq 0.05$)

b) In der zweiten Versuchsanordnung zur Erfassung antiadipöser Wirkung der erfindungsgemäßen Verbindungen wurde die Abnahme des Fettgewebes am Beispiel des Ovarfettgewebes gemessen. Die Verbindungen wurden zu diesem Zwecke Mäusen einmal täglich mittels Schlundsonde in 1,5%iger Methylzellulosesuspension appliziert. Am fünften Tag wurde das Ovarfettgewebe herauspräpariert und gewogen. In der nachfolgenden Tabelle sind die Ergebnisse in % einer mitgeführten Kontrollgruppe wiedergegeben.

| Verbindung | % Änderung vom Wert der Kontrollgruppe Dosis: 10 [mg/kg] |
|---|---|
| A | -36 |
| B | -60 |
| C | -35 |
| D | -30 |
| F | -47 |
| G | -33 |
| H | -20 |
| I | -28 |
| J | -11 |
| K | -12 |
| L | -53 |
| M | -48 |

3. Kardiale Nebenwirkungen:

Das Auftreten von unerwünschten Nebenwirkungen auf das Herz konnte für die erfindungsgemäßen Verbindungen für den therapeutisch angestrebten stoffwechselaktiven Dosisbereich ausgeschlossen werden. Als Nachweis diente die Messung der Herzfrequenz an Mäusen während der Testung auf blutzuckersenkende Wirkung (s.o.). Eine Stunde nach der oralen Applikation der Verbindungen wurde die Herzfrequenz mittels EKG-getriggertem Tachographen bestimmt. Die nachfolgende Tabelle gibt die Änderung der Herzfrequenz in % der Kontrollgruppe wieder.

| Verbindung | % Änderung vom Wert der Kontrollgruppe Dosis [mg/kg] 0,3 | 1 | 3 |
|---|---|---|---|
| A | | (21) | |
| B | | (0) | |
| C | | | (11) |
| D | | 17 | |
| E | | | 17 |
| F | | | (9) |
| I | | (3) | |
| J | (0) | | |
| K | | (-4) | |
| M | | (-1) | |
| O | | | (7) |

( ) = nicht signifikant ($p > 0.05$)

Desweiteren konnten bei den vorstehend beschriebenen Untersuchungen der erfindungsgemäßen Substanzen bei den applizierten Dosen keine toxischen Nebenwirkungen beobachtet werden. Diese sind daher gut verträglich.

Auf Grund ihrer pharmakologischen Eigenschaften eignen sich daher die neuen Verbindungen der allgemeinen Formel I sowie deren physiologisch vertägliche Säureadditionssalze mit anorganischen oder organischen Säuren zur Behandlung sowohl des Diabetes mellitus als auch der Adipositas, insbesondere also zur Behandlung des adipösen Diabetikers. Außerdem können die neuen Verbindungen zur Prophylaxe und zur Behandlung atherosklerotischer Veränderungen der Gefäße, die vor allem bei Diabetikern und Obesen gehäuft auftreten, verwendet werden. Hierbei kann die erforderliche Dosis ganz auf den stoffwechselphysiologischen Bedarf des einzelnen Patienten abgestimmt werden, da diese über einen großen Dosisbereich frei von einer Herz/Kreislaufwirkung sind. Beim Erwachsenen liegen daher die Tagesdosis zwischen 1 und 3000 mg, vorzugsweise jedoch 1 bis 1000 mg, verteilt auf 1 bis 4 Dosen pro Tag. Hierzu lassen sich die obenerwähnten Verbindungen mit einem oder mehreren inerten Trägerstoffen und/oder Verdünnungsmitteln, gegebenenfalls in Kombination mit anderen Wirksubstanzen, in die üblichen galenischen Zubereitungsformen wie Pulver, Tabletten, Dragées, Kapseln, Suppositorien oder Suspensionen einarbeiten.

Desweiteren können die vorstehend erwähnten Verbindungen zur Behandlung fettsüchtiger Tiere wie Hunde und als Folge ihrer körperfettreduzierenden (lipolytischen) Wirkung zur Reduktion unerwünschter Fetteinlagerungen bei der Mastzucht, also zur Verbesserung der Fleischqualität von Masttieren wie Schweinen, Rindern, Schafen und Geflügel eingesetzt werden. Bei den Tieren kann die Applikation der oben genannten Verbindungen oral oder auch nicht-oral, z.B. als Futterzusatz oder durch Injektion oder auch über implantierte Minipumpen erfolgen. Die Tagesdosis liegen hierbei zwischen 0,01 und 100 mg/kg, vorzugsweise jedoch zwischen 0,1 bis 10 mg/kg Körpergewicht.

Die nachfolgenden Beispiele sollen die Erfindung näher erläutern:

Beispiel A

2-Trifluormethyl-4-bromacetyl-thiazol

9,2 g (0,071 Mol) Trifluormethylthioacetamid, gelöst in 200 ml Acetonitril, werden in 2,5 Stunden zu einer

siedenden Lösung von 17,4 g (0,071 Mol) Dibromdiacetyl in 200 ml Acetonitril zugetropft. Das Lösungsmittel wird abdestilliert und das zurückbleibende Produkt mit Cyclohexan extrahiert. Der Extrakt wird eingeengt und der verbleibende ölige Rückstand über eine Kieselgelsäule mit Toluol/Cyclohexan als Eluens gereinigt.
Ausbeute: 8,2 g (42,7 % der Theorie),
Schmelzpunkt: 36-37 °C

Beispiel B

2,4-Dimethyl-5-acetyl-thiazol

Eine Mischung von 34 g (0,25 Mol) 3-Chloracetylaceton und 19 g (0,25 Mol) Thioacetamid werden unter Rühren langsam erwärmt. Bei ca. 60 °C setzt eine exotherme Reaktion ein. Man entfernt das Heizbad, rührt 1 Stunde nach und läßt das Produkt dabei abkühlen. Das ausgefallene Hydrobromid wird mit Petrolether/Ethanol = 5/1 verrieben und abgesaugt. Zum Überführen in die freie Base wird das Salz in Wasser gelöst und mit Natriumbicarbonat alkalisch gestellt. Anschließend wird mehrfach mit Methylenchlorid extrahiert, die organische Phase über Natriumsulfat getrocknet, eingeengt und der Rückstand über eine Kieselgelsäule mit Toluol als Eluens gereinigt.
Ausbeute: 19,7 g (51 % der Theorie)
$^1$H-NMR-Spektrum ($CDCl_3$): $\delta$ = 2,50 ppm (s, $CH_3$)

Beispiel C

2,4-Dimethyl-5-bromacetyl-thiazol

Eine Lösung von 20,8 g (0,13 Mol) Brom in 50 ml Eisessig wird zu einer zum Rückfluß erhitzten Lösung von 20,2 g (0,13 Mol) 2,4-Dimethyl-5-acetyl-thiazol in Eisessig langsam zugetropft. Nach 1 Stunde wird zur Trockne eingeengt, in Wasser gelöst und mit einer Natriumcarbonatlösung neutralisiert. Anschließend wird mehrfach mit Methylenchlorid extrahiert, die organische Phase getrocknet und eingeengt. Das so erhaltene Öl wurde ohne weitere Reinigung für weitere Umsetzungen verwendet.
Ausbeute: 24 g (79 % der Theorie).

Beispiel D

2-Phenyl-4-formyl-thiazol-cyanhydrin

16,4 g (0,0607 Mol) 2-Phenyl-4-formyl-thiazol-hydrobromid werden in 230 ml Wasser und 150 ml Dioxan am Dampfbad auf 30 °C erwärmt, wobei sich ca. 80-90 % der Substanz löst. Anschließend wird im Eisbad auf 20 °C abgekühlt und unter kräftigem Rühren 22 g (0,162 Mol) Kaliumdihydrogenphosphat in Portionen zugesetzt. Anschließend werden 6,1 g (0,124 Mol) Natriumcyanid in Portionen eingetragen und die Mischung bei Raumtemperatur 1,5 Stunden gerührt. Das ausgefallene Produkt wird mit Ether extrahiert, die organische Phase über Natriumsulfat getrocknet, eingeengt und der Rückstand im Vakuum getrocknet.
Ausbeute: 13 g (100 % der Theorie),
Schmelzpunkt: 140-141 °C

| | | | | |
|---|---|---|---|---|
| Ber.: | C 62,20 | H 3,72 | N 12,95 | S 14,82 |
| Gef.: | 62,35 | 3,91 | 12,89 | 14,93 |

Beispiel E

2-(2-Trifluormethyl-thiazol-4-yl)glyoxal

3 g (0,011 Mol) 2-Trifluormethyl-4-bromacetyl-thiazol werden in 30 ml Dimethylsulfoxyd gelöst und für 70 Stunden bei Raumtemperatur gehalten. Danach wird die Lösung auf 100 g Eis gegossen, mehrfach mit Ether extrahiert, die organische Phase über Natriumsulfat getrocknet, eingeengt und der Rückstand über eine Kieselgelsäule mit Toluol/Essigester = 7/3 als Eluens gereinigt.
Ausbeute: 1,3 g (56 % der Theorie).

Beispiel F

2-Trifluormethyl-5-methyl-4-bromacetyl-oxazol

a) 3-Trifluoracetamino-acetylaceton

10 g (0,0662 Mol) 3-Amino-acetylaceton-hydrochlorid werden vorsichtig mit 56 g (0,266 Mol) Trifluoressigsäureanhydrid versetzt. Das Reaktionsgemisch schäumt stark und es bildet sich dann eine klare Lösung. Die Mischung wird für 30 Minuten am Rückfluß gekocht, anschließend das Lösungsmittel abgezogen, der Rückstand in 500 ml Ether aufgenommen und dreimal mit je 100 ml gesättigter Natriumbicarbonatlösung verschüttelt. Die Ether-Phase wird über Natriumsulfat getrocknet und eingeengt.
Ausbeute: 12 g (86 % der Theorie),
Schmelzpunkt: 44-46 °C

| | | | |
|---|---|---|---|
| Ber.: | C 39,81 | H 3,81 | N 6,63 |
| Gef.: | 39,80 | 3,67 | 6,87 |

b) 2-Trifluormethyl-5-methyl-4-acetyl-oxazol

5 g (0,0237 Mol) 3-Trifluoracetamino-acetylaceton werden mit 5 ml Trifluoressigsäureanhydrid für 4 Stunden am Dampfbad erhitzt. Die Lösung verfärbt sich dabei und das Trifluoressigsäureanhydrid verdampft fast vollständig. Der Rückstand wird in 100 ml Chloroform gelöst und dreimal mit je 50 ml gesättigter Natriumbicarbonatlösung verschüttelt. Die Chloroform-Phase wird über Natriumsulfat getrocknet und eingedampft.
Ausbeute: 3,2 g (70 % der Theorie),
$^1$H-NMR-Spektrum ($CDCl_3$) :
$\delta$ = 2,52 ppm (s, $CH_3$);
2,65 ppm (s, $CH_3$).

c) 2-Trifluormethyl-5-methyl-4-bromacetyl-oxazol

3,8 g (0,02 Mol) 2-Trifluormethyl-5-methyl-4-acetyl-oxazol werden in 30 ml Eisessig gelöst und mit 3 ml 33 %iger Bromwasserstofflösung in Eisessig versetzt. Die Mischung wird auf 80 °C erhitzt und im Laufe von einer Stunde mit einer Lösung von 3,2 g (0,02 Mol) Brom in 10 ml Eisessig versetzt. Nach weiteren 30 Minuten wird der Eisessig abgezogen. Als Rückstand verbleibt ein dunkles Öl, welches als Rohprodukt weiter umgesetzt wird.
Ausbeute: 4,4 g (81 % der Theorie),
$^1$H-NMR-Spektrum ($CDCl_3/CD_3OD$):
$\delta$ = 2,72 ppm (s, $CH_3$)
4,52 ppm (s, $CH_2$)

Beispiel G

2,5-Dimethyl-4-bromacetyl-oxazol

2,8 g (0,02 Mol) 2,5-Dimethyl-4-acetyl-oxazol werden in 30 ml Eisessig gelöst und mit 3 ml 35 %iger Bromwasserstofflösung in Eisessig versetzt. Die Mischung wird auf 100 °C erhitzt und im Laufe von 1 Stunde mit einer Lösung von 3,2 g (0,02 Mol) Brom in 10 ml Eisessig versetzt. Nach weiteren 2 Stunden wird das Lösungsmittel abgezogen, der Rückstand mit 50 ml Ether verrieben und abgesaugt.
Ausbeute: 4,4 g (74 % der Theorie),
Schmelzpunkt: 175-176 °C

| | | | | |
|---|---|---|---|---|
| Ber.: | C 28,12 | H 3,03 | N 4,68 | Br 53,45 |
| Gef.: | 28,29 | 2,93 | 4,78 | 53,37 |

Beispiel H

2-Methyl-4-formyl-thiazol-cyanhydrin

Hergestellt analog Beispiel D durch Umsetzung von 2-Methyl-4-formyl-thiazol mit Natriumcyanid und Kaliumdihydrogenphosphat in Dimethylformamid/Wasser = 1/1. Nach Extrahieren mit Essigester, Trocknen des Extrakts mit Natriumsulfat wird über eine Kieselgelsäule mit Toluol/Essigester = 8/2 als Eluens

gereinigt.
Ausbeute: 41 % der Theorie,
Schmelzpunkt: 116-117 °C
$^{1}$H-NMR-Spektren (DMSO/$CD_3OD$): $\delta$ = 7,580 ppm (s,1H)

Beispiel I

4-Formyl-thiazol-cyanhydrin

Hergestellt analog Beispiel D durch Umsetzung von 4-Formylthiazol-hydrobromid mit Natriumcyanid und Kaliumdihydrogenphosphat in Wasser. Der ausgefallene Niederschlag wird abgesaugt und mit Wasser gewaschen. Nach dem Trocknen im Vakuumexsikkator erhält man ein fast farbloses Kristallisat, welches ohne weitere Reinigung umgesetzt wird.
Ausbeute: 53 % der Theorie,
Schmelzpunkt: 113-115 °C

Beispiel K

2-Trifluormethyl-5-methyl-4-bromacetyl-thiazol

a) 2-Trifluormethyl-5-methyl-4-acetyl-thiazol

5,8 g (0,0275 Mol) 3-Trifluoracetamino-acetylaceton werden mit 6,4 g (0,0158 Mol) 2,4-Bis-(4-methoxyphenyl)-1,3-dithia-2,4-diphosphetan-2,4-disulfid in 35 ml absolutem Toluol unter Rühren und Stickstoff 10 Stunden lang auf 100 °C erhitzt. Die entstandene klare Lösung wird eingeengt und der Rückstand über eine Kieselgelsäule mit Methylenchlorid als Eluens gereinigt.
Ausbeute: 3,1 g (54 % der Theorie),

| | | | | |
|---|---|---|---|---|
| Ber.: | C 40,19 | H 2,88 | N 6,69 | S 15,32 |
| Gef.: | 40,45 | 2,69 | 6,87 | 15,33 |

b) 2-Trifluormethyl-5-methyl-4-bromacetyl-thiazol

3 g (0,0144 Mol) 2-Trifluormethyl-5-methyl-4-acetyl-thiazol werden in 30 ml Eisessig gelöst und 2,1 ml 33%ige Bromwasserstofflösung in Eisessig zugesetzt. Die Mischung wird auf 80 °C erwärmt und im Laufe von 30 Minuten mit einer Lösung von 2,3 g (0,0144 Mol) Brom in 15 ml Eisessig versetzt. Nach weiteren 20 Minuten wird der Eisessig abgezogen. Als Rückstand verbleibt ein dunkles Öl, welches als Rohprodukt weiter umgesetzt wird.
Ausbeute: 3,8 g (92 % der Theorie),
1H-NMR-Spektrum ($CDCl_3$/$CD_3OD$):
= 2,90 (S,$CH_3$);
4,70 (S,$CH_2$).

Beispiel L

2-Hydroxy-2-(2-propyl-thiazol-4-yl)ethanamin

Hergestellt analog Beispiel P durch Reduktion von 4-(α-Cyano-α-hydroxy-methyl)-2-propyl-thiazol (Schmelzpunkt: 68-70 °C) mit Natriumborhydrid in einem Tetrahydrofuran/Trifluoressigsäure-Gemisch. Zur Aufarbeitung wird unter Kühlung mit Wasser versetzt, bis zur Lösung des gebildeten Niederschlages gerührt, mit konz. Salzsäure angesäuert und 30 Minuten bei 20 °C und 60 Minuten bei 100 °C erhitzt. Nach Abkühlen und Versetzen mit Wasser wird die organische Phase abgetrennt und verworfen. Die saure wäßrige Phase wird noch einmal mit Ethylacetat extrahiert, welche verworfen wird. Danach wird unter Eiskühlung mit 6N Natronlauge stark alkalisch gestellt. Durch mehrfache Extraktion mit Chloroform sowie Trocknen, Filtrieren und Eindampfen der Chloroform-Lösung im Vakuum kristallisiert man den Eindampfrückstand aus Ether.
Ausbeute: 75 % der Theorie,
Schmelzpunkt: 73-75 °C.

Beispiel M

2-Hydroxy-2-(2-isopropyl-thiazol-4-yl)ethanamin

Hergestellt analog Beispiel L durch Reduktion von 4-(α-Cyano-α-hydroxy-methyl)-2-isopropyl-thiazol (Schmelzpunkt: 56-58° C) mit Natriumborhydrid in einem Tetrahydrofuran/Trifluoressigsäure-Gemisch.
Ausbeute: 72 % der Theorie,
Schmelzpunkt: 82-85° C

Beispiel N

2-Hydroxy-2-(2-trifluormethyl-thiazol-4-yl)ethylamin

4,6 g (0,033 Mol) Urotropin werden zu einer Lösung von 9 g (0,033 Mol) 2-Trifluormethyl-4-bromacetyl-thiazol in 30 ml Methylenchlorid bei l0° C unter Rühren und Kühlen zugegeben. Nach wenigen Sekunden fällt ein dicker Kristallbrei aus. Es wird auf 0-3° C abgekühlt, nach 20 Minuten der Niederschlag abgesaugt und mit Ether gewaschen. Nach dem Trocknen bei 40° C werden farblose Kristalle erhalten.
Ausbeute: ll,l g (8l,3 % der Theorie),
Schmelzpunkt: l34-l37° C
Dieses Urotropinsalz wird in 330 ml Ethanol gelöst und zusammen mit einer Lösung von 70 ml konzentrierter Salzsäure in 600 ml Wasser für 2 Stunden zum Sieden erhitzt. Anschließend wird zur Trockne eingeengt. Der so erhaltene feste Rückstand wird in 300 ml Methanol gelöst, auf 0° C abgekühlt und nacheinander mit 2,4 g Natriumhydrogencarbonat und in kleinen Portionen mit 4,2 g Natriumborhydrid versetzt. Nach 2 Stunden setzt man 30 ml 30 %ige Natronlauge zu und rührt 20 Minuten nach. Nach Verdünnen mit 200 ml Wasser und mehrfaches Ausschütteln mit Methylenchlorid wird die organische Phase über Natriumsulfat getrocknet, eingeengt und über eine Kieselgelsäule mit Methanol als Eluens gereinigt.
Ausbeute: 2,9 g (5l % der Theorie),
$^1$H-NMR-Spektrum ($CDCl_3/CD_3OD$): δ = 7,675 ppm (s, lH)

Beispiel O

2-(2-Trifluormethyl-thiazol-4-yl)morpholin

Eine Lösung von 0,9 g (0,036 Mol) 2-(2-Trifluormethyl-thiazol-4-yl)morpholin-5-on in 40 ml Tetrahydrofuran wird bei 3° C mit l,35 g (0,036 Mol) Natriumborhydrid versetzt. Bei 5-8° C werden sodann sehr langsam unter kräftigem Rühren 2,43 g (0,036 Mol) Eisessig, gelöst in 20 ml Tetrahydrofuran, zugetropft. Nach 2 Stunden wird die Kühlung entfernt und l6 Stunden bei Raumtemperatur gerührt. Nach dem Einengen zur Trockene wird der erhaltene Rückstand mit l5 ml 20%iger Salzsäure versetzt und für 30 Minuten auf 90° C erhitzt. Danach wird zur Trockene eingeengt, das erhaltene Produkt in Wasser aufgenommen und mit Natriumcarbonatlösung alkalisch gestellt. Anschließend wird mehrfach mit Methylenchlorid extrahiert, die organische Phase über Natriumsulfat getrocknet, eingeengt und der Rückstand über eine Kieselgelsäule mit Essigester/Methanol = 8:2 als Eluens gereinigt.
Ausbeute: 0,56 g (65 % der Theorie),
$^1$H-NMR-Spektrum ($CDCl_3/CD_3OD$): δ = 4,750 ppm (dd, =C$\underline{H}$-O)

Beispiel P

2-Hydroxy-2-(2-phenyl-thiazol-4-yl)ethanamin

Zu einer Suspension von ll,4 g (0,3 Mol) Natriumborhydrid in 200 ml absolutem Tetrahydrofuran werden unter Eiskühlung 34,2 g (0,3 Mol) Trifluoressigsäure in 60 ml absolutem Tetrahydrofuran zugetropft. Anschließend werden l3 g (0,06 Mol) 2-Phenyl-4-formyl-thiazol-cyanhydrin in Portionen eingetragen und dann über 20 Stunden bei Raumtemperatur gerührt. Das Lösungsmittel wird abgezogen, der Rückstand vorsichtig mit l00 g Eis versetzt, mit verdünnter Salzsäure angesäuert und l Stunde am Dampfbad erhitzt. Die Mischung wird auf Raumtemperatur abgekühlt, mit Ammoniaklösung alkalisch gestellt und mit Chloroform extrahiert. Der Extrakt wird über Natriumsulfat getrocknet, eingeengt und über eine Kieselgelsäule mit Methanol als Eluens gereinigt.
Ausbeute: l0,2 g (77,3 % der Theorie),

Schmelzpunkt: 92-94 °C
Ber.: C 59,97 H 5,49 N
Gef.: 60,15 5,61 12,83

Beispiel Q

2-Hydroxy-2-(thiazol-4-yl)-ethanamin

Hergestellt analog Beispiel P durch Umsetzung von 4-Formylthiazol mit Natriumborhydrid und Trifluoressigsäure in Tetrahydrofuran. Das durch Extraktion mit Methylenchlorid gewonnene Produkt wird über eine Kieselgelsäule mit Methanol als Eluens gereinigt.
Ausbeute: 19 % der Theorie
$^1$H-NMR-Spektrum ($CDCl_3$/$CD_3OD$): $\delta$ = 4,880 ppm (dd, =C$\underline{H}$OH)

Beispiel R

2-Hydroxy-2-(2-methyl-thiazol-4-yl)ethanamin

Hergestellt analog Beispiel P durch Umsetzung von 2-Methyl-4-formyl-thiazol mit Natriumborhydrid und Trifluoressigsäure in Tetrahydrofuran.
Ausbeute: 63 % der Theorie,
$^1$H-NMR-Spektrum ($CDCl_3$/$DD_3OD$): $\delta$ = 7,150 ppm (s, 1H)

Beispiel S

2-(2-Trifluormethyl-thiazol-4-yl)morpholin-5-on

0,3 g (0,0064 Mol) einer Natriumhydrid-Dispersion (50-55 % in Öl) werden bei 30 °C in kleinen Portionen unter Rühren zu einer Lösung von 1 g (0,0047 Mol) 2-Hydroxy-2-(2-trifluormethyl-thiazol-4-yl)-ethylamin in 15 ml Toluol zugegeben. Nach 1 Stunde wird eine Lösung von 0,55 g (0,0045 Mol) Chloressigsäureethylester in 2 ml Toluol zugetropft. Nach 2 Stunden werden erst 1 ml Ethanol und danach 4 ml Wasser zugetropft. Anschließend wird mit Salzsäure sauer gestellt, mehrmals mit Methylenchlorid extrahiert und der Extrakt über Natriumsulfat getrocknet. Das erhaltene Produkt wird über eine Kieselgelsäule mit Essigester/Methanol = 1/1 als Eluens gereinigt und nach dem Einengen zur Trockene mit wenig Ether digeriert.
Ausbeute: 0,36 g (32 % der Theorie),
Schmelzpunkt: 139-141 °C

Beispiel T

2-(2-Methyl-thiazol-4-yl)morpholin-5-on

Hergestellt analog Beispiel S durch Umsetzung von 2-Hydroxy-2-(2-methyl-thiazol-4-yl)ethylamin mit Chloressigsäureethylester und Reinigung über eine Kieselgelsäule mit Essigester/Methanol = 19:1 als Eluens.
Ausbeute: 29 % der Theorie,
Schmelzpunkt: 125-126 °C
Ber.: C 48,47 H 5,08 N 14,13 S 16,17
Gef.: 48,63 5,07 14,10 16,47

Beispiel U

2-(2-Methyl-thiazol-4-yl)morpholin

Hergestellt analog Beispiel O durch Reduktion von 2-(2-Methyl-thiazol-4-yl)morpholin-5-on mit Lithiumaluminiumhydrid und Reinigung der Base über eine Kieselgelsäule mit Chloroform/Methanol/Ammoniak = 93:7:0,7 als Eluens.
Ausbeute: 46 % der Theorie,

Ber.: C 52,15 H 6,56 N 15,20 S 17,40
Gef.: 52,37 6,52 15,27 17,32

Beispiel V

2-Hydroxy-2-(2-methyl-thiazol-5-yl)ethanamin

a) 2-Methyl-5-formyl-thiazol-cyanhydrin

0,5 g (0,004 Mol) 2-Methyl-5-formyl-thiazol werden in 3 ml Wasser gelöst und auf 15° C abgekühlt. Es werden nacheinander 1 g Kaliumdihydrogenphosphat und 0,4 g Natriumcyanid zugegeben. Fast sofort fällt ein farbloses Produkt aus, welches noch 25 Minuten bei 10° C gerührt und dann abgesaugt wird. Das erhaltene rohe Produkt wird ohne weitere Reinigung weiter umgesetzt.
Ausbeute: 0,5 g (81 % der Theorie)

b) 2-Hydroxy-2-(2-methyl-thiazol-5-yl)ethanamin

Hergestellt analog Beispiel P durch Umsetzung von 2-Methyl-5-formyl-thiazol-cyanhydrin in Tetrahydrofuran mit Natriumborhydrid und Trifluoressigsäure. Die Base wird über eine Kieselgelsäule mit Methanol als Eluens gereinigt und als Rohprodukt für die weiteren Umsetzungen verwendet.
Ausbeute: 87 % der Theorie

Beispiel W

2-(N,N-Dimethylamino)-4-bromacetyl-thiazol

133,5 g Dibromdiacetyl werden in 2,5 l Ether über 2,5 Tage mit 57 g N,N-Dimethylthioharnstoff in einer Soxhletapparatur am Rückfluß gekocht. Nach Abkühlung des Reaktionsgemisches und Einengen wird der erhaltene Rückstand in Wasser aufgenommen, mit gesättigter Natriumbicarbonatlösung neutralisiert und mehrmals mit Methylenchlorid extrahiert. Die vereinigten Extrakte werden getrocknet, filtriert, eingeengt und anschließend über eine Kieselgelsäule mit Toluol/Essigester = 8:2 als Eluens gereinigt.
Ausbeute: 50 g (27,7 % der Theorie),
Schmelzpunkt: 113-115° C
Ber.: C 33,75 H 3,64 N 11,26 S 12,87 Br 32,08
Gef.: 33,90 3,63 11,25 12,69

Beispiel X

2-Hydroxy-2-(2-methyl-oxazol-4-yl)ethanamin

a) 2-Methyl-4-formyl-oxazol-cyanhydrin

Hergestellt analog Beispiel V durch Umsetzung von 6 g (0,0543 Mol) 2-Methyl-4-formyl-oxazol mit 5,45 g (0.112 Mol) Natriumcyanid und 14,4 g (0,1 g Mol) Kaliumdihydrogenphosphat in 180 ml Wasser und 60 ml Dioxan.
Ausbeute: 6,2 g (83 % der Theorie),
Die Substanz ist ölig.
Ber.: C 52,17 H 4,37 N 20,28
Gef.: 52,08 4,50 19,98

b) 2-Hydroxy-2-(2-methyl-oxazol-4-yl)ethanamin

Hergestellt analog Beispiel P durch Umsetzung von 6 g (0,0435 Mol) 2-Methyl-4-formyl-oxazol-cyanhydrin mit 8,25 g (0,217 Mol) Natriumborhydrid in Tetrahydrofuran und 24,6 g (0,214 Mol) Trifluoressigsäure.
Ausbeute: 2,7 g (44 % der Theorie),
Die Substanz ist ölig.
Ber.: C 50,69 H 7,09 N 19,70

Gef.: 50,32 7,22 19,68

$^1$H-NMR-Spektrum (80 MHZ) ($CDCl_3$): $\delta$ = 7.55 ppm (s,1H)

Beispiel Y

2-Chlor-4-bromacetyl-thiazol

7,6 g (6,0344 Mol) 2-Amino-4-bromacetyl-thiazol werden in 20 ml Wasser und 50 ml konzentrierter Salzsäure gelöst. Bei 0°C wird nun unter Rühren eine Lösung von 3,44 g (0,0499 Mol) Natriumnitrit in 15 ml Wasser zugetropft. Anschließend wird die entstandene Diazoniumsalz-Lösung unter kräftigem Rühren in eine kalte Lösung von 4,93 g (0,0449 Mol) Kupfer(I)chlorid in 15 ml konzentrierter Salzsäure portionsweise eingetragen und die Mischung bei Raumtemperatur über 20 Stunden gerührt. Dann wird mit 100 ml Wasser verdünnt und mit Ether extrahiert. Der Etherextrakt wird über Natriumsulfat getrocknet und eingedampft. Zur Reinigung wird das Rohprodukt über eine Kieselgelsäule mit Methylenchlorid als Eluens gereinigt.

Ausbeute: 4 g (48 % der Theorie),

Schmelzpunkt: 72°C

Ber.: C 24,96 H 1,25 N 5,82

Gef.: 25,12 1,30 6,00

Beispiel Z

2-Piperidino-4-bromacetyl-thiazol

7,2 g (0,05 Mol) Piperidino-thioharnstoff werden in einer Soxhlet-Apparatur für 10 Stunden mit einer Lösung von 12,2 g (0,05 Mol) Dibromdiacetyl in 1 l Ether zum Rückfluß erhitzt. Die ausgefallene gelbe Verbindung wird abgesaugt, in 500 ml Chloroform gelöst und die Chloroformlösung mit 400 ml gesättigter Natriumbicarbonatlösung extrahiert. Anschließend wird die Chloroformlösung über Natriumsulfat getrocknet und eingedampft. Das Rohprodukt wird über eine Kieselgelsäule mit Toluol als Eluens gereinigt.

Ausbeute: 7,5 g (52 % der Theorie),

Schmelzpunkt: 78-80°C

Ber.: C 41,53 H 4,52 N 9,68 Br 27,63

Gef.: 41,80 4,47 9,40 27,57

Beispiel ZA

2-Methoxy-2-(2-methyl-thiazol-4-yl)ethanamin

Hergestellt analog Beispiel L durch Reduktion von 4-(α-Cyano-α-methoxy-methyl)-2-methyl-thiazol [erhalten aus 4-(Dimethoxymethyl)-2-methyl-thiazol durch Umsetzung mit Trimethylsilylcyanid in Ether in Gegenwart von Bortrifluorid-etherat] mittels Natriumborhydrid in einem Tetrahydrofuran/Trifluoressigsäure-Gemisch.

Ausbeute: 80 % der Theorie (Öl),

Massenspektrum: Ber. $(M + H^+)$ = 173

Gef. $(M + H^+)$ = 173

Beispiel ZB

2-(2-Trifluormethyl-thiazol-4-yl)ethylenoxid

a) 1-(2-Trifluormethyl-thiazol-4-yl)-1-hydroxy-2-bromethan

6 g (0,022 Mol) 2-Trifluormethyl-4-bromacetyl-thiazol werden in 150 ml Methanol gelöst, auf 10°C gekühlt und mit 0,63 g Natriumborhydrid versetzt. Nach 15 Minuten wird mit Eis versetzt, mit Salzsäure angesäuert, mit Ammoniak alkalisch gestellt und mit Methylenchlorid ausgeschüttelt. Die organische Phase wird über Natriumsulfat getrocknet, filtriert und eingeengt. Man erhält ein Öl, welches als Rohprodukt weiter umgesetzt wird.

Ausbeute: 5,4 g (89 % der Theorie)

$^1$H-NMR-Spektrum (80 MHz) ($CDCl_3$): $\delta$ = 7,7 ppm (s, 1H)

b) 2-(2-Trifluormethyl-thiazol-4-yl)ethylenoxid

5 g (0,018 Mol) 1-(2-Trifluormethyl-thiazol-4-yl)-1-hydroxy-2-bromethan werden in 4 ml 50%iger Natronlauge suspendiert und 5 Minuten lang gerührt. Anschließend wird mit Eiswasser verdünnt und mit Methylenchlorid ausgeschüttelt. Die organische Phase wird über Natriumsulfat getrocknet, im Vakuum bei 20 °C eingeengt und das erhaltene Öl über eine Kieselgelsäule mit Methylenchlorid als Eluens gereinigt.
Ausbeute: 2,15 (67 % der Theorie),
$^{1}$-NMR-Spektrum (80 MHz) ($CDCl_3$): $\delta$ 7,5 ppm (s, 1H)

Beispiel ZC

(2-Methyl-thiazol-4-yl)ethylenoxid

10,2 g (0,048 Mol) Trimethylsulfoniumjodid werden in 42 ml Dimethylsulfoxid gelöst und unter Rühren zu einer auf 0 °C gekühlten Lösung von 1,14 g Natriumhydrid in 50 ml Dimethylsulfoxid/Tetrahydrofuran(1:1) zugetropft. Nach 60 Minuten tropft man bei 0 °C eine Lösung von 6,1 g 2-Methyl-4-formylthiazol in 25 ml Tetrahydrofuran zu und rührt 3 Stunden bei Raumtemperatur. Anschließend wird auf 0 °C abgekühlt, 9,6 ml Wasser zugetropft und mit Ether extrahiert. Die etherische Phase wird 2 x mit Wasser ausgeschüttelt, getrocknet und eingeengt. Das erhaltene Öl wird über eine Kieselgelsäule mit Toluol/Essigester (65:35) als Eluens gereinigt.
Ausbeute: 1,6 g (24 % der Theorie),
$^{1}H$-NMR-Spektrum (80 MHz) ($CDCl_3$): $\delta$ = 7,1 ppm (s, 1H)

Beispiel 1

N-[2-(4-Carbomethoxyphenyl)-1-methylethyl]-N-benzyl-2-hydroxy-2-(2-benzoylamino-thiazol-4-yl)ethanamin

3,5 g (0,011 Mol) 2-Benzoylamino-4-bromacetyl-thiazol werden in 20 ml Dimethylformamid gelöst und unter Rühren bei Raumtemperatur zu einer Lösung von 2,6 g (0,092 Mol) N-Benzyl-2-(4-carbomethoxyphenyl)-1-methyl-ethylamin und 1,1 g (0,011 Mol) Triethylamin, gelöst in 40 ml Dimethylformamid, zugetropft. Nach 1,5 Stunden wird mit Eis/Wasser versetzt und mit Methylenchlorid extrahiert. Der Extrakt wird über Natriumsulfat getrocknet und eingeengt. Das erhaltene Aminoketon wird in 100 ml Methanol aufgenommen und bei Raumtemperatur mit 0,6 g Natriumborhydrid versetzt. Nach 1 Stunde wird zur Trockene eingeengt, mit Wasser versetzt und mit Salzsäure sauer gestellt. Nach 10 Minuten wird mit Ammoniak alkalisch gestellt und mit Methylenchlorid extrahiert. Der Extrakt wird über Natriumsulfat getrocknet, eingeengt und über eine Kieselgelsäule mit Toluol/Essigester = 8:2 als Eluens gereinigt.
Ausbeute: 1,4 g (29 % der Theorie),

| | | | |
|---|---|---|---|
| Ber.: | C 68,03 | H 5,90 | N 7,93 |
| Gef.: | 68,13 | 6,11 | 7,88 |

$^{1}H$-NMR-Spektrum ($CDCl_3$/$CD_3OD$): $\delta$ = 6,875 ppm (s, 1H)

Beispiel 2

N-[2-(4-Carboxyphenyl)-1-methylethyl]-N-benzyl-2-hydroxy-2-(2-amino-thiazol-4-yl)ethanamin

13,6 ml (0,0136 Mol) 1 N Natronlauge werden bei Raumtemperatur zu einer Lösung von 1,5 g (0,0034 Mol) N-[2-(4-Carbomethoxyphenyl)-1-methylethyl]-N-benzyl-2-hydroxy-2-(2-aminothiazol-4-yl)ethanamin in Dioxan/Methanol = 1:1 innerhalb von 10 Minuten unter Rühren zugetropft. Nach 1 Stunde werden 20 ml Wasser so langsam eingetropft, daß immer eine Lösung erhalten bleibt. Nach 16 Stunden fügt man 13,6 ml (0,0136 Mol) 1 N Salzsäure hinzu und extrahiert mit Methylenchlorid, trocknet über Natriumsulfat, engt zur Trockene ein und reinigt die erhaltene Base über eine Kieselgelsäule mit Essigester/Methanol = 9:1.
Ausbeute: 0,36 g (26 % der Theorie),

| | | | |
|---|---|---|---|
| Ber.: | C 64,21 | H 6,12 | N 10,21 |
| Gef.: | 64,12 | 6,12 | 9,99 |

$^{1}H$-NMR-Spektrum (DMSO): $\delta$ = 6,250 ppm (s, 1H)

Beispiel 3

N-[2-(4-Carboethoxyphenyl)ethyl]-2-hydroxy-2-(2-benzoylaminothiazol-4-yl)ethanamin

9,6 g (0,03 Mol) 2-Benzoylamino-4-bromacetyl-thiazol werden in 100 ml Methylenchlorid gelöst und unter Rühren bei Raumtemperatur zu einer Lösung von 11,5 g (0,06 Mol) 2-(4-Carboethoxyphenyl)ethylamin in 150 ml Methylenchlorid zugetropft. Nach 1,5 Stunden wird auf 5°C abgekühlt, mit 200 ml Methanol verdünnt und zur Reduktion des erhaltenen Aminoketons bei 0-5°C mit 3 g Natriumborhydrid in kleinen Portionen versetzt. Nach 3 Stunden wird die Lösung zur Trockene eingeengt, mit Eis/Wasser versetzt und mit Salzsäure sauer gestellt. Nach 10 Minuten wird mit Natriumbicarbonatlösung alkalisch gestellt und mit Methylenchlorid extrahiert. Der Extrakt wird über Natriumsulfat getrocknet, eingeengt und über eine Kieselgelsäule mit Chloroform/Methanol = 9:1 unter Zusatz von 2 % mit Ammoniak gesättigtem Ethanol als Eluens gereinigt. Das erhaltene Produkt wird mit Petrolether zur Kristallisation gebracht.
Ausbeute: 3,4 g (26 % der Theorie),
Schmelzpunkt: 83-85°C

| | | | |
|---|---|---|---|
| Ber.: | C 62,85 | H 5,73 | N 9,56 |
| Gef.: | 62,75 | 5,78 | 9,41 |

Beispiel 4

N-[2-(4-Carboxyphenyl)ethyl]-2-hydroxy-2-(2-benzoylaminothiazol-4-yl)ethanamin

Hergestellt analog Beispiel 2 durch Umsetzung von N-[2-(4-Carboethoxyphenyl)ethyl]-2-hydroxy-2-(2-benzoylamino-thiazol-4-yl)-ethanamin mit 1 N Natronlauge und anschließender Reinigung des Rohproduktes über eine Kieselgelsäule mit Chloroform/Methanol = 1:1 und Verreiben mit Wasser/Ethanol = 9:1.
Ausbeute: 36 % der Theorie,
Schmelzpunkt: 143-145°C (Zers.)

| | | | |
|---|---|---|---|
| Ber. | C 61,29 | H 5,14 | N 10,14 |
| Gef.: | 61,19 | 5,18 | 10,06 |

Beispiel 5

N-[2-(4-Carboethoxyphenyl)ethyl]-2-hydroxy-2-(2-benzoylamino-4-methyl-thiazol-5-yl)ethanamin

Hergestellt analog Beispiel 3 durch Umsetzung von 2-(4-Carboethoxyphenyl)ethylamin und stöchiometrischen Mengen Triethylamin mit 2-Benzoylamino-4-methyl-5-bromacetyl-thiazol, anschließender Reduktion und Reinigung der Base über eine Kieselgelsäule mit Chloroform/Methanol = 9:1 als Eluens und Verreiben mit Petrolether.
Ausbeute: 33 % der Theorie,
Schmelzpunkt: 96-98°C

| | | | |
|---|---|---|---|
| Ber.: | C 63,55 | H 6,00 | N 9,26 |
| Gef.: | 63,45 | 5,85 | 9,19 |

Beispiel 6

N-[2-(4-Carboethoxyphenyl)ethyl]-2-hydroxy-2-(2-acetylamino-4-methyl-thiazol-5-yl)ethanamin

Hergestellt analog Beispiel 3 durch Umsetzung von 2-(4-Carboethoxyphenyl)ethylamin und stöchiometrischen Mengen Triethylamin mit 2-Acetylamino-4-methyl-5-bromacetyl-thiazol, anschließender Reduktion und Reinigung der Base über eine Kieselgelsäule mit Essigester/Methanol = 19:1 als Eluens und Verreiben mit Ether.
Ausbeute: 33 % der Theorie,
Schmelzpunkt: 97-99°C

| | | | |
|---|---|---|---|
| Ber.: | C 58,29 | H 6,44 | N 10,73 |
| Gef.: | 58,40 | 6,58 | 10,61 |

Beispiel 7

N-[2-(4-Carboxyphenyl)ethyl]-2-hydroxy-2-(2-acetylamino-4-methyl-thiazol-5-yl)ethanamin

Hergestellt analog Beispiel 2 durch Umsetzung von N-[2-(4-Carboethoxyphenyl)ethyl]-2-hydroxy-2-(2-acetylamino-4-methyl-thiazol-5-yl)ethanamin mit l N Natronlauge. Nach Neutralisieren mit l N Salzsäure wird zur Trockene eingeengt und der Rückstand aus l0 ml Wasser umkristallisiert.
Ausbeute: 80 % der Theorie,
Schmelzpunkt: l56-l58 °C
Ber.: C 56,l8 H 5,83 N ll,56
Gef.: 56,20 5,95 ll,6l

Beispiel 8

N-[2-(4-Carboethoxyphenyl)ethyl]-2-hydroxy-2-(2-amino-4-methyl-thiazol-5-yl)ethanamin-dihydrochlorid

3 g (0,0l3 Mol) 2-Amino-4-methyl-5-bromacetyl-thiazol werden unter Rühren bei Raumtemperatur in kleinen Portionen zu einer Lösung von 5,2 g (0,026 Mol) Triethylamin in 300 ml Tetrahydrofuran zugegeben. Nach 2 Stunden wird zur Trockene eingeengt. Der erhaltene Rückstand wird in Ethanol gelöst und zur Reduktion des gebildeten Aminoketons werden in kleinen Portionen l,5 g Natriumborhydrid bei l5 °C unter Rühren zugegeben. Nach l6 Stunden wird die Lösung zur Trockene eingeengt, mit Wasser versetzt und mit Salzsäure angesäuert. Danach stellt man mit Ammoniak alkalisch und extrahiert mit Methylenchlorid. Der Extrakt wird über Natriumsulfat getrocknet, eingeengt und über eine Kieselgelsäule mit Chloroform/Methanol = 8:2 als Eluens gereinigt. Die erhaltene Base wird in Ethanol gelöst, mit isopropanolischer Salzsäure und Aceton in das Dihydrochlorid übergeführt und mit Ether gewaschen.
Ausbeute: 3,5 g (64 % der Theorie),
Schmelzpunkt: l60 °C
Ber.: C 48,34 H 5,97 N 9,95
Gef.: 48,23 6,20 9,97

Beispiel 9

N-[2-(4-Carboxyphenyl)ethyl]-2-hydroxy-2-(2-amino-4-thiazol-4-yl)ethanamin-dihydrochlorid

l,5 g (0,0043 Mol) N-[2-(4-Carboxyphenyl)ethyl]-2-hydroxy-2-(2-acetylamino-thiazol-4-yl)ethanamin werden in 30 ml l N Salzsäure gelöst und für 2,5 Stunden auf 90 °C erhitzt und anschließend zur Trockene eingeengt. Der verbleibende Rückstand wird aus einem Gemisch von 50 ml Ethanol und 3 ml Wasser umkristallisiert und das erhaltene Kristallisat mit Ether gewaschen.
Ausbeute: l,4 g (85 % der Theorie),
Schmelzpunkt: 2l8-2l9 °C
Ber.: C 44,2l H 5,04 N ll,05
Gef.: 44,40 5,l7 l0,94

Beispiel l0

N-[2-(4-Carbomethoxyphenyl)-l-methylethyl]-2-hydroxy-2-(2-acetylamino-thiazol-4-yl)ethanamin-hydrochlorid

Hergestellt analog Beispiel 3 durch Umsetzung von 2-(4-Carbomethoxyphenyl)-l-methylethylamin und Triethylamin in Tetrahydrofuran mit 2-Acetylamino-4-bromacetyl-thiazol, anschließender Reduktion und Reinigung der Base über eine Kieselgelsäule mit Essigester/Methanol = 9:l als Eluens und Ausfällen des Hydrochlorids mit isopropanolischer Salzsäure aus Ether.
Ausbeute: 36 % der Theorie,
Schmelzpunkt: ll8-l20 °C
Ber.: C 52,23 H 5,84 N l0,l5
Gef.: 5l,98 6,0l 9,97
Laut $^1$H-NMR-Spektrum (400 MHz) liegt ein ca. 35:65-Diasteromerengemisch vor.

Beispiel ll

N-[2-(4-Carboxyphenyl)-l-methylethyl]-2-hydroxy-2-(2-amino-thiazol-4-yl)ethanamin-dihydrochlorid

Hergestellt analog Beispiel 9 durch Umsetzung von N-[2-(4-Carboxyphenyl)-l-methylethyl]-2-hydroxy-2-(2-acetylamino-thiazol-4-yl)ethanamin und I N Salzsäure.
Ausbeute: 79 % der Theorie,
Schmelzpunkt: I67 °C
Ber.: C 45,69 H 5,37 N I0,66
Gef.: 45,49 5,5I I0,54
Laut $^1$H-NMR-Spektrum (400 MHz) liegt ein ca. 40:60-Diasteromerengemisch vor.

Beispiel I2

N-[2-(4-Carbomethoxyphenyl)-I-methylethyl]-2-hydroxy-2-(2-trifluormethyl-thiazol-4-yl)ethanamin

I,3 g (0,0062 Mol) 2-(2-Trifluormethyl-thiazol-4-yl)glyoxal und I,2 g (0,0057 Mol) 2-(4-Carbomethoxyphenyl)-I-methylethylamin werden bei 50 °C zusammengegeben und bei Raumtemperatur für 4 Stunden gerührt. Zur Reduktion der entstandenen Schiff'schen Base wird mit 0,75 g Natriumborhydrid in Portionen bei 20 °C versetzt und für I6 Stunden gerührt. Anschließend wird auf Eis gegossen, mit Salzsäure angesäuert, mit Natriumbicarbonatlösung alkalisch gestellt und mit Methylenchlorid extrahiert. Der Extrakt wird über Natriumsulfat getrocknet, eingeengt und über eine Kieselgelsäule mit Methylenchlorid/Methanol = 40:I als Eluens gereinigt.
Ausbeute: I,5 g (68 % der Theorie),
Ber.: C 52,57 H 4,93 N 7,2I
Gef.: 52,7I 5,08 7,30
$^1$H-NMR-Spektrum ($CDCl_3$):
$\delta$ = 4,735 ppm (dd, =CH-OH);
4,895 ppm (dd, =CH-OH)
Laut $^1$H-NMR-Spektrum (400 MHz) liegt ein ca. 60:40-Diasteromerengemisch vor.

Beispiel I3

N-[2-(4-Carbomethoxymethoxyphenyl)-I-methylethyl]-2-hydroxy-2-(2-phenyl-thiazol-4-yl)ethanamin

I,32 g (0,006 Mol) 2-Hydroxy-2-(2-phenyl-thiazol-4-yl)ethanamin und I,33 g (0,006 Mol) I-(4-Carbomethoxymethoxyphenyl)propan-2-on werden in 40 ml absolutem Methanol gelöst, mit 0,34 ml (0,006 Mol) Eisessig und 0,37 g (0,006 Mol) Natriumcyanborhydrid versetzt und 20 Stunden bei Raumtemperatur gerührt. Anschließend wird auf Eis gegossen, mit Salzsäure angesäuert, mit Natriumbicarbonatlösung alkalisch gestellt und mit Chloroform extrahiert. Der Extrakt wird über Natriumsulfat getrocknet, eingeengt und über eine Kieselgelsäule mit Essigester/Methanol = 9:I als Eluens gereinigt.
Ausbeute: 2,2 g (86 % der Theorie),
Ber.: C 64,76 H 6,I4 N 6,56 S 7,5I
Gef.: 64,50 6,42 6,39 7,30
$^1$H-NMR-Spektrum ($CDCl_3$):
$\delta$ = 4,88 ppm (dd, =CH-OH);
4,93 ppm (dd, =CH-OH)
Laut $^1$H-NMR-Spektrum (400 MHz) liegt ein ca. 50:50-Diasteromerengemisch vor.

Beispiel I4

N-[2-(4-Aminocarbonylmethoxyphenyl)-I-methylethyl]-2-hydroxy-2-(2-methyl-thiazol-4-yl)ethanamin

I g (0,0027 Mol) N-[2-(4-Carbomethoxymethoxyphenyl)-I-methylethyl]-2-hydroxy-2-(2-methyl-thiazol-4-yl)ethanamin werden in 5 ml Methanol gelöst und mit 5 ml konzentriertem Ammoniak 3 Stunden lang bei Raumtemperatur gerührt. Anschließend wird mit Wasser verdünnt und mit Methylenchlorid extrahiert. Der Extrakt wird über Natriumsulfat getrocknet, eingeengt und über eine Kieselgelsäule mit Essigester/Methanol = 9:I als Eluens gereinigt.
Ausbeute: 0,76 g (8I % der Theorie),
Schmelzpunkt: I48 °C
Ber.: C 58,43 H 6,63 N I2,02
Gef.: 58,62 6,69 I2,00
Laut $^1$H-NMR-Spektrum (400 MHz) liegt ein ca. 25:75-Diasteromerengemisch vor.

Beispiel 15

N-[2-(4-Methylaminocarbonylmethoxyphenyl)-1-methylethyl]-2-hydroxy-2 (2-trifluormethyl-thiazol-4-yl)-ethanamin

Hergestellt analog Beispiel 3 durch Umsetzung von 2-Trifluormethyl-4-bromacetyl-thiazol und 2-(4-Methylaminocarbonylmethoxyphenyl)-1-methylethylamin, anschließender Reduktion und Reinigung der Base über eine Kieselgelsäule mit Methylenchlorid/Methanol = 9:1 als Eluens.
Ausbeute: 10 % der Theorie,
Ber.: C 51,79 H 5,31 N 10,17
Gef.: 51,79 5,55 10,91
$^1$H-NMR-Spektrum ($CDCl_3/CD_3OD$):
$\delta$ = 7,575 ppm (s, 1H),
7,595 ppm (s, 1H);
Laut $^1$H-NMR-Spektrum (400 MHz) liegt ein ca. 50:50-Diasteromerengemisch vor.

Beispiel 16

N-[2-(4-Carboxyphenyl)-1-methylethyl]-2-hydroxy-2-(2-trifluormethyl-thiazol-4-yl)ethanamin

Hergestellt analog Beispiel 2 durch Umsetzung von N-[2-(4-Carbomethoxyphenyl)-1-methylethyl]-2-hydroxy-2-(2-trifluormethyl-thiazol-4-yl)ethanamin mit 1 N Natronlauge. Nach Neutralisieren mit Salzsäure wird zur Trockene eingeengt und der Rückstand mit einer Mischung von Essigester/Ethanol = 6:1 behandelt. Der Extrakt wird zur Trockene eingeengt und mit 5 ml Wasser verrieben. Nach Abdenkatieren des Wassers wird der verbleibende Rückstand in Methanol aufgenommen, zur Trockene gebracht, mit Ether verrieben und abgesaugt.
Ausbeute: 52 % der Theorie,
Schmelzpunkt: 107-109° C,
Ber.: C 51,33 H 4,58 N 7,48
Gef.: 51,41 4,74 7,42
Laut $^1$H-NMR-Spektrum (400 MHz) liegt ein ca. 60:40-Diasteromerengemisch vor.

Beispiel 17

N-[2-(4-Carbomethoxymethoxyphenyl)-1-methylethyl]-2-(2-trifluormethyl-thiazol-4-yl)morpholin

Hergestellt analog Beispiel 13 durch Umsetzung von 2-(2-Trifluormethyl-thiazol-4-yl)morpholin mit 1-(4-Carbomethoxymethoxyphenyl)propan-2-on und anschließender Reinigung der Base über eine Kieselgelsäule mit Toluol/Essigester = 8:2 als Eluens.
Ausbeute: 54 % der Theorie,
Ber.: C 54,04 H 5,22 N 6,30
Gef.: 54,28 5,24 6,46
$^1$H-NMR-Spektrum ($CDCl_3$):
$\delta$ = 7,610 ppm (s, 1H);
7,575 ppm (s, 1H)
Laut $^1$H-NMR-Spektrum (400 MHz) liegt ein ca. 50:50-Diasteromerengemisch vor.

Beispiel 18

3-[2-(4-Carbomethoxymethoxyphenyl)-1-methylethyl]-5-(2-trifluormethyl-thiazol-4-yl)-2-oxazolidincarbonsäure-methylester

0,52 g (0,0012 Mol) N-[2-(4-Carbomethoxymethoxyphenyl)-1-methylethyl]-2-hydroxy-2-(2-trifluormethyl-thiazol-4-yl)ethanamin und 0,211 g (0,0024 Mol) Methylglyoxylat werden in 25 ml Toluol 10 Minuten auf 100° C erhitzt und anschließend 1 Stunde am Wasserabscheider gekocht. Die Lösung wird nach dem Abkühlen mit 50 ml Essigester versetzt und mit 30 ml Wasser geschüttelt. Die organische Phase wird abgetrennt, über Natriumsulfat getrocknet und eingeengt. Der Rückstand wird über eine Labor-Fertigsäule, Größe B (Firma Merck) mit Toluol/Essigester = 20:1,5 als Eluens gereinigt.

Fraktion A (Diastereomerenpaare A und B):
Ausbeute: 90 mg (15,4 % der Theorie),
$^1$H-NMR-Spektrum ($CDCl_3/CD_3OD$):
$\delta$ = 5,10 ppm (s, 1H);
5,24 ppm (s, 1H)
Laut $^1$H-NMR-Spektrum (400 MHz) liegt ein ca. 50:50-Diasteromerengemisch vor.
Fraktion B (Diastereomerenpaare C und D):
Ausbeute: 110 mg (18,8 % der Theorie),
$^1$H-NMR-Spektrum ($CDCl_3/CD_3OD$):
$\delta$ = 4,97 ppm (s, 1H);
5,21 ppm (s, 1H)
Laut $^1$H-NMR-Spektrum (400 MHz) liegt ein ca. 50:50-Diasteromerengemisch vor.

Beispiel 19

3-[2-(4-Carbomethoxyphenyl)-1-methylethyl]-5-(2-trifluormethyl-thiazol-4-yl)-2-oxazolidincarbonsäuremethylester

Hergestellt analog Beispiel 18 durch Umsetzung von N-[2-(4-Carbomethoxyphenyl)-1-methylethyl]-2-hydroxy-2-(2-trifluormethyl-thiazol-4-yl)ethanamin mit Methylglyoxylat. Fraktion A (Diastereomerenpaare A und B):
Ausbeute: 25 % der Theorie),
$^1$H-NMR-Spektrum ($CDCl_3/CD_3OD$):
$\delta$ = 5,07 ppm (s, 1H);
5,26 ppm (s, 1H)
Laut $^1$H-NMR-Spektrum (400 MHz) liegt ein ca. 45:55-Diasteromerengemisch vor.
Fraktion B (Diastereomerenpaare C und D):
Ausbeute: 23 % der Theorie),
$^1$H-NMR-Spektrum ($CDCl_3/CD_3OD$):
$\delta$ = 5,08 ppm (s, 1H);
5,22 ppm (s, 1H)
Laut $^1$H-NMR-Spektrum (400 MHz) liegt ein ca. 35:65-Diasteromerengemisch vor.

Beispiel 20

N-[2-(4-Carbomethoxymethoxyphenyl)-1-methylethyl]-2-hydroxy-2-(2-trifluormethyl-thiazol-4-yl)ethanamin

Hergestellt analog Beispiel 3 durch Umsetzung von 2-(4-Carbomethoxymethoxyphenyl)-1-methylethylamin mit 2-Trifluormethyl-4-bromacetyl-thiazol, anschließender Reduktion und Reinigung der Base über eine Kieselgelsäule mit Methylenchlorid/Methanol = 20:1 als Eluens.
Ausbeute: 51 % der Theorie,
Ber.: C 51,66 H 5,06 N 6,70
Gef.: 51,40 5,14 6,64
$^1$H-NMR-Spektrum ($CDCl_3$):
$\delta$ = 4,835 ppm (dd, =CH-OH);
4,895 ppm (dd, =CH-OH)
Laut $^1$H-NMR-Spektrum (400 MHz) liegt ein ca. 60:40-Diasteromerengemisch vor.

Beispiel 21

N-[2-(4-Carbomethoxyphenyl)-1-methylethyl]-2-hydroxy-2-(4-methyl-oxazol-5-yl)ethanamin-dihydrochlorid

2,6 g (0,009 Mol) 4-Methyl-5-bromacetyl-oxazol und 1,16 g (0,009 Mol) N,N-Diisopropyl-ethylamin werden in 50 ml Methylenchlorid gelöst. Diese Lösung wird zu 3,47 g (0,018 Mol) 2-(4-Carbomethoxyphenyl)-1-methylethylamin in 100 ml Methylenchlorid innerhalb von 20 Minuten zugetropft und die Mischung für 2 Stunden bei Raumtemperatur und eine Stunde bei 35 °C gerührt. Die Reaktionslösung wird im Eisbad abgekühlt und mit 150 ml Methanol versetzt. Zur Reduktion des entstandenen Aminoketons werden dann 1 g Natriumborhydrid in Portionen über 30 Minuten zugesetzt, 20 Stunden bei Raumtemperatur gerührt und

eingedampft. Anschließend wird der Rückstand mit Eiswasser versetzt, mit Salzsäure angesäuert, mit wässerigem konzentreirtem Ammoniak alkalisch gestellt und mit Chloroform extrahiert. Der Extrakt wird über Natriumsulfat getrocknet, eingeengt und über eine Kieselgelsäule mit Essigester/Methanol = 9:l als Eluens gereinigt. Anschließend wird das Hydrochlorid mit etherischer Salzsäure gefällt.
Ausbeute: l,l g (3l % der Theorie),
Schmelzpunkt: 80 ° C (Zers.)

| | | | | |
|---|---|---|---|---|
| Ber.: | C 52,l7 | H 6,l8 | N 7,l3 | Cl l8,l4 |
| Gef.: | 52,00 | 6,l0 | 6,90 | l7,90 |

Laut $^1$H-NMR-Spektrum (400 MHz) liegt ein ca. 50:50-Diasteromerengemisch vor.

Beispiel 22

N-[2-(4-Carbomethoxyphenyl)-l-methylethyl]-2-hydroxy-2-(thiazol-4-yl)ethanamin

Hergestellt analog Beispiel 3 durch Umsetzung von 2-(4-Carbomethoxyphenyl)-l-methylethylamin mit 4-Bromacetyl-thiazol, anschließender Reduktion und Reinigung der Base über eine Kieselgelplatte mit Essigester/Methanol = 8:2 als Eluens.
Ausbeute: 9 % der Theorie,

| | | | |
|---|---|---|---|
| Ber.: | C 59,97 | H 6,29 | N 8,75 |
| Gef.: | 60,09 | 6,0l | 8,56 |

$^1$H-NMR-Spektrum ($CDCl_3$): δ = 4,900-4,970 ppm (m, =CH-OH);
Laut $^1$H-NMR-Spektrum (400 MHz) liegt ein ca. 60:40-Diasteromerengemisch vor.

Beispiel 23

N-[2-(4-Carbomethoxymethoxyphenyl)-l-methylethyl]-N-methyl-2-hydroxy-2-(2-trifluormethyl-thiazol-4-yl)-ethanamin

Hergestellt analog Beispiel 3 durch Umsetzung von 2-Trifluormethyl-4-bromacetyl-thiazol und N-Methyl-2-(4-carbomethoxymethoxyphenyl)-l-methylethylamin, anschließender Reduktion und Reinigung der Base über eine Kieselgelsäule mit Essigester/Methanol = 40:l als Eluens.
Ausbeute: 53 % der Theorie,

| | | | |
|---|---|---|---|
| Ber.: | C 52,77 | H 5,36 | N 6,48 |
| Gef.: | 53,00 | 5,06 | 6,64 |

$^1$H-NMR-Spektrum ($CDCl_3$/$CD_3OD$):
δ = 7,555 ppm (s, lH);
7,575 ppm (s, lH)
Laut $^1$H-NMR-Spektrum (400 MHz) liegt ein ca. 50:50-Diasteromerengemisch vor.

Beispiel 24

N-[2-(4-Aminocarbonylmethoxyphenyl)-l-methylethyl]-2-hydroxy-2-(2-trifluormethyl-thiazol-4-yl)ethanamin

Hergestellt analog Beispiel l4 durch Umsetzung von N-[2-(4-Carbomethoxymethoxyphenyl)-l-methylethyl]-2-hydroxy-2-(2-trifluormethyl-thiazol-4-yl)ethanamin mit konzentriertem Ammoniak und anschließender Reinigung der Base über eine Kieselgelsäule mit Methylenchlorid/Methanol = 9:l als Eluens.
Ausbeute: 65 % der Theorie,

| | | | |
|---|---|---|---|
| Ber.: | C 50,6l | H 5,00 | N l0,42 |
| Gef.: | 50,43 | 5,l9 | l0,37 |

$^1$H-NMR-Spektrum ($CDCl_3$):
δ = 4,850 ppm (dd, =CH-OH);
4,775 ppm (dd, =CH-OH)
Laut $^1$H-NMR-Spektrum (400 MHz) liegt ein ca. 60:40-Diasteromerengemisch vor.

Beispiel 25 N-[2-(4-Carbomethoxymethoxyphenyl)-l-methylethyl]-2-hydroxy-2-(2-methyl-thiazol-4-yl)ethanamin

Hergestellt analog Beispiel I3 durch Umsetzung von 2-Hydroxy-2-(2-methyl-thiazol-4-yl)ethylamin mit I-(4-Carbomethoxymethoxyphenyl)propan-2-on und anschließender Reinigung der Base über eine Kieselgelsäule mit Essigester/Methanol = 9:I als Eluens.
Ausbeute: 55 % der Theorie,
Ber.: C 59,32 H 6,64 N 7,69
Gef.: 59,20 6,45 7,9I
$^1$H-NMR-Spektrum ($CDCl_3$):
δ = 4,825 ppm (dd, =CH-OH);
4,775 ppm (dd, =CH-OH)
Laut $^1$H-NMR-Spektrum (400 MHz) liegt ein ca. 50:50-Diasteromerengemisch vor.

Beispiel 26

N-[2-(4-Carbomethoxymethoxyphenyl)-I-methylethyl]-2-hydroxy-2-(thiazol-4-yl)ethanamin

Hergestellt analog Beispiel I3 durch Umsetzung von 2-Hydroxy-2-(thiazol-4-yl)ethylamin mit I-(4-Carbomethoxymethoxyphenyl)propan-2-on und anschließender Reinigung der Base über eine Kieselgelsäule mit Essigester/Methanol = 8:2 als Eluens.
Ausbeute: 48 % der Theorie,
Ber.: C 58,26 H 6,33 N 8,00
Gef.: 58,4I 6,36 8,22
$^1$H-NMR-Spektrum ($CDCl_3$):
δ = 4,945 ppm (dd, =CH-OH);
4,900 ppm (dd, =CH-OH)
Laut $^1$H-NMR-Spektrum (400 MHz) liegt ein ca. 50:50-Diasteromerengemisch vor.

Beispiel 27

N-[2-(4-Carbomethoxyphenyl)-I-methylethyl]-2-hydroxy-2-(2,5-dimethyl-oxazol-4-yl)ethanamin-dihydrochlorid

Hergestellt analog Beispiel 2I durch Umsetzung von 2,5-Dimethyl-4-bromacetyl-oxazol mit 2-(4-Carbomethoxyphenyl)-I-methylethylamin, anschließender Reduktion und Fällung des Dihydrochlorids mit etherischer Salzsäure.
Ausbeute: 47 % der Theorie,
Schmelzpunkt: 78 °C (Zers.)
Ber.: C 53,33 H 6,46 N 6,90 Cl I7,5I
Gef.: 53,I0 6,50 6,80 I7,32
Laut $^1$H-NMR-Spektrum (400 MHz) liegt ein ca. 50:50-Diasteromerengemisch vor.

Beispiel 28

N-[2-(4-Carbomethoxymethoxyphenyl)-I-methylethyl]-2-hydroxy-2-(2,5-dimethyl-oxazol-4-yl)ethanamin-dihydrochlorid

Hergestellt analog Beispiel 2I durch Umsetzung von 2,5-Dimethyl-4-bromacetyl-oxazol mit 2-(4-Carbomethoxymethoxyphenyl)-I-methylethylamin, anschließender Reduktion und Fällung des Dihydrochlorids mit etherischer Salzsäure.
Ausbeute: 47 % der Theorie,
Schmelzpunkt: 82 °C (Zers.)
Ber.: C 52,4I H 6,48 N 6,43 Cl I6,28
Gef.: 52,2I 6,55 6,50 I6,40
Laut $^1$H-NMR-Spektrum (400 MHz) liegt ein ca. 50:50-Diasteromerengemisch vor.

Beispiel 29

N-[2-(4-Carbomethoxymethoxyphenyl)-I-methylethyl]-2-hydroxy-2-(2,4-dimethyl-thiazol-5-yl)ethanamin

Hergestellt analog Beispiel 3 durch Umsetzung von 2,4-Dimethyl-5-bromacetyl-thiazol mit 2-(4-Carbo-

methoxymethoxyphenyl)-I-methylethylamin, anschließender Reduktion und Reinigung der Base über eine Kieselgelsäule mit Essigester/Methanol = I7:3.
Ausbeute: 45 % der Theorie,
Ber.: C 60,29 H 6,92 N 7,40
Gef.: 60,53 6,85 7,60
$^1$H-NMR-Spektrum ($CDCl_3/CD_3OD$):
δ = 4,890-4,970 ppm (m, =CH-OH);
Laut $^1$H-NMR-Spektrum (400 MHz) liegt ein ca. 53:47-Diasteromerengemisch vor.

Beispiel 30

N-[ 2-( 4-Carbomethoxymethoxyphenyl) -1-methylethyl]-N-( 2-hydroxyethyl ) -2-hydroxy-2-( 2-trifluormethyl-thiazol-4-yl) -ethanamin

Hergestellt analog Beispiel 3 durch Umsetzung von 2-Trifluormethyl-4-bromacetyl-thiazol und N-(2-Hydroxyethyl)-2(4-carbomethoxymethoxyphenyl)-1-methylethylamin. Vor der Reduktion mit Natriumborhydrid wird zur Vervollständigung der Reaktion 3 Stunden zum Sieden erhitzt. Anschließend wird die Base über eine Kieselgelsäule mit Essigsäureethylester als Fließmittel gereinigt.
Ausbeute: 50 % der Theorie,
Ber.: C 51,94 H 5,45 N 6,06
Gef.: 52,00 5,33 6,09

$^1$H-NMR-Spektrum ($CDCl_3$): δ = 0,930 ppm (d, -CH-);
|
$CH_3$
0,970 ppm (d, -CH-)
|
$CH_3$

Beispiel 3I

N-[2-(4-Carbomethoxymethoxyphenyl)-I-methylethyl]-2-hydroxy-2-(2-trifluormethyl-5-methyl-oxazol-4-yl)-ethanamin-hydrochlorid

Hergestellt analog Beispiel 2I durch Umsetzung von 2-Trifluormethyl-5-methyl-4-bromacetyl-oxazol mit 2-(4-Carbomethoxymethoxyphenyl)-I-methylethylamin und anschließender Reduktion. Die Verbindung wird über eine Kieselgelsäule mit Essigester/Methanol = 20:I als Eluens gereinigt und anschließend das Hydrochlorid mit etherischer Salzsäure gefällt.
Ausbeute: 0,43 g (I0 % der Theorie),
Schmelzpunkt: 58° C
Ber.: C 50,38 H 5,34 N 6,I8 Cl 7,82
Gef.: 50,58 5,33 5,93 8,20

Beispiel 32

N-[2-(4-Carbomethoxymethoxyphenyl)-I-methylethyl]-2-(2-trifluormethyl-thiazol-4-yl)morpholin

Hergestellt analog Beispiel 3 durch Umsetzung von II,6 g (0,043 Mol) 2-Trifluormethyl-4-bromacetyl-thiazol mit 23 g (0,086 Mol) N-(2-Hydroxyethyl)-2-(4-carbomethoxymethoxyphenyl)-I-methylethylamin. Zur vollständigen Umsetzung wird 6 Stunden zum Sieden erhitzt. Anschließend wird im Vakuum eingeengt, der erhaltene Rückstand in 85 ml Trifluoressigsäure gelöst und mit 7 g (0,06 Mol) Triethylsilan bei Raumtemperatur versetzt. Nach 90 Stunden wird die Lösung auf Eis gegossen, mit konzentriertem Ammoniak versetzt und mehrmals mit Methylenchlorid extrahiert. Die organische Phase wird über Natriumsulfat getrocknet, eingeengt und der Rückstand über eine Kieselgelsäule mit Toluol/Essigester = 8/2 als Eluens gereinigt.
Ausbeute: II g (58 % der Theorie),

$^1$H-NMR-Spektrum: δ = 7,6l0 ppm (s, lH); 7,578 ppm (s, lH)
Laut $^1$H-NMR-Spektrum (400 MHZ) liegt ein ca. 43:57-Diastereomerengemisch vor.

Beispiel 33

N-[2-(4-Carbomethoxymethoxyphenyl)-l-methylethyl]-2-hydroxy-2-(2-trifluormethyl-5-methyl-thiazol-4-yl)-ethanamin

Hergestellt analog Beispiel 3 durch Umsetzung von 2-Trifluormethyl-5-methyl-4-bromacetyl-thiazol mit 2-(4-Carbomethoxymethoxyphenyl)-l-methylethylamin und anschließender Reduktion. Die Verbindung wird über eine Kieselgelsäule mit Essigester/Methanol = 9/l als Eluens gereinigt.
Ausbeute: 8 % der Theorie,
Ber.: C 52,77 H 5,36 N 6,47
Gef.: 52,60 5,44 6,55
$^1$H-NMR-Spektrum ($CDCl_3$):
δ = 4,765 ppm (dd, =CH-OH);
4,8l0 ppm (dd, =CH-OH)
Laut $^1$H-NMR-Spektrum (400 MHZ) liegt ein ca. 54:46-Diastereomerengemisch vor.

Beispiel 34

N-[3-(4-Carboxamidophenyl)-l-methylpropyl]-2-hydroxy-2-(2-trifluormethyl-thiazol-4-yl)ethanamin

Hergestellt analog Beispiel l3 durch Umsetzung von 2-Hydroxy-2-,2-trifluormethyl-thiazol-4-yl)ethanamin mit l-(4-Carboxamidophenyl)butan-3-on und anschließender Reinigung der Base über eine Kieselgelsäule mit Essigester/Methanol = 5/l als Eluens.
Ausbeute: 26 % der Theorie,
Schmelzpunkt: ll9-l2l °C
Ber.: C 52,70 H 5,20 N l0.85
Gef.: 52,6l 5,35 l0,84
Laut $^1$H-NMR-Spektrum (400 MHZ) liegt ein ca. 50:50-Diastereomerengemisch vor.

Beispiel 35

N-[2-[4-Carboxymethoxyphenyl)-l-methylethyl]-2-(2-trifluormethyl-thiazol-4-yl)morpholin

Hergestellt analog Beispiel l6 durch Umsetzung von N-[2-(4-Carbomethoxymethoxyphenyl)-l-methylethyl]-2-(2-trifluormethyl-thiazol-4-yl)morpholin in Methanol mit ln Natronlauge. Nach Neutralisieren mit ln Salzsäure wird mit Methylenchlorid ausgeschüttelt, der Extrakt zur Trockene eingeengt und der verbleibende Rückstand mit Petrolether verrieben und abgesaugt.
Ausbeute: 94 % der Theorie,
Schmelzpunkt: 86 °C
Ber.: C 53,0l H 4,92 N 6,5l
Gef.: 53,l4 4,85 6,54

Beispiel 36

N-[2-(4-Carboxymethoxyphenyl)-l-methylethyl]-2-hydroxy-2-(2-trifluormethyl-thiazol-4-yl)ethanamin

Hergestellt analog Beispiel l6 durch Umsetzung von N-[2-(4-Carbomethoxymethoxyphenyl)-l-methylethyl]-2-(2-trifluormethyl-thiazol-4-yl)ethanamin in Methanol mit ln Natronlauge. Nach Neutralisieren mit ln Salzsäure wird mit Methylenchlorid ausgeschüttelt, der Extrakt zur Trockene eingeengt und der verbleibende Rückstand mit Petroläther verrieben und abgesaugt.
Ausbeute: 50 % der Theorie,
Schmelzpunkt: 80-82 °C (Zers.)
Ber.: C 50,49 H 4,74 N 6,93
Gef.: 50,60 4,6l 7,04

Beispiel 37

N-[2-(4-Carbomethoxymethoxyphenyl)-l-methylethyl]-2-hydroxy-2-(2-chlor-thiazol-4-yl)ethanamin-hydrochlorid

Hergestellt analog Beispiel l3 durch Umsetzung von 2-Hydroxy-2-(2-chlor-thiazol-4-yl)ethanamin mit l-(4-Carbomethoxymethoxyphenyl)propan-2-on und anschließender Fällung des Hydrochlorids mit etherischer Salzsäure.
Ausbeute: 43 % der Theorie,
Schmelzpunkt: 58 °C (Zers.)

| | | | | | |
|---|---|---|---|---|---|
| Ber.: | C 48,45 | H 5,26 | N 6,64 | Cl l6,82 | S 7,60 |
| Gef.: | 48,48 | 5,23 | 6,6l | l6,67 | 7,87 |

Beispiel 38

N-[2-(4-(2-Carbomethoxy-l-methylethenyl)phenyl)-l-methylethyl]-2-hydroxy-2-(2-trifluormethyl-thiazol-4-yl)-ethanamin

Hergestellt analog Beispiel l3 durch Umsetzung von 2-Hydroxy-2-(2-trifluormethyl-thiazol-4-yl)ethanamin mit l-[4-(2-Carbomethoxy-l-methylethenyl)phenyl]propan-2-on und anschließender Reinigung der Base über eine Kieselgelsäule mit Chloroform/Methanol/methanolischem Ammoniak = 9,5/0,4/0,l als Eluens.
Ausbeute: 43 % der Theorie,

| | | | | |
|---|---|---|---|---|
| Ber.: | C 56,00 | H 5,4l | N 6,54 | S 7,48 |
| Gef.: | 56,00 | 5,57 | 6,37 | 7,76 |

Laut $^1$H-NMR-Spektrum (400 MHz) liegt ein ca. 3:4-Diastereomerengemisch vor.

Beispiel 39

N-[2-(4-(2-Carbomethoxy-l-methylethenyl)phenyl)-l-methylethyl]-2-(2-trifluormethyl-thiazol-4-yl)morpholin

Hergestellt analog Beispiel l3 durch Umsetzung von 2-(2-Trifluormethyl-thiazol-4-yl)morpholin mit l-[4-(2-Carbomethoxy-l-methylethenyl)phenyl]propan-2-on und anschließender Reinigung der Base über eine Kieselgelsäule mit Chloroform/Essigester = l9:l als Eluens.
Ausbeute: 29% der Theorie,

| | | | | |
|---|---|---|---|---|
| Ber.: | C 58,l4 | H 5,54 | N 6,l6 | S 7,05 |
| Gef.: | 58,38 | 5,49 | 5,96 | 7,40 |

Laut $^1$H-NMR-Spektrum (400 MHz) liegt ein ca. 50:50-Diastereomerengemisch vor.

Beispiel 40

N-[2-(4-(2-Carbomethoxy-l-methylethenyl)phenyl)-l-methylethyl]-2-hydroxy-2-(2-methyl-thiazol-4-yl)-ethanamin

Hergestellt analog Beispiel l3 durch Umsetzung von 2-Hydroxy-2-(2-methyl-thiazol-4-yl)ethanamin mit l-[4-(2-Carbomethoxy-l-methylethenyl)phenyl]propan-2-on und anschließender Reinigung der Base über eine Kieselgelsäule mit Toluol/Methanol = l9:l als Eluens.
Ausbeute: 32% der Theorie,

| | | | | |
|---|---|---|---|---|
| Ber.: | C 65,97 | H 7,05 | N 6,99 | S 8,0l |
| Gef.: | 65,70 | 7,l6 | 6,88 | 8,05 |

Laut $^1$H-NMR-Spektrum (400 MHz) liegt ein ca. 50:50-Diastereomerengemisch vor.

Beispiel 4l

N-[2-(4-(2-Carbomethoxy-l-methylethenyl)phenyl)-l-methylethyl]-2-(2-methyl-thiazol-4-yl)morpholin

Hergestellt analog Beispiel l3 durch Umsetzung von 2-(2-Methyl-thiazol-4-yl)morpholin mit l-[4-(2-Carbomethoxy-l-methylethenyl)phenyl]propan-2-on und anschließender Reinigung der Base über eine Kieselgelsäule mit Chloroform/Methanol/Ammoniak = 9:l:0,l als Eluens.

Ausbeute: 59% der Theorie,

| | | | | |
|---|---|---|---|---|
| Ber.: | C 64,13 | H 7,00 | N 7,48 | S 8,56 |
| Gef.: | 63,90 | 6,86 | 7,20 | 8,28 |

Laut $^1$H-NMR-Spektrum (400 MHz) liegt ein ca. 50:50-Diastereomerengemisch vor.

Beispiel 42

N-[2-(4-Hydroxyphenyl)-l-methylethyl]-2-(2-methyl-thiazol-4-yl)morpholin

Hergestellt analog Beispiel 13 durch Umsetzung von 2-(2-Methyl-thiazol-4-yl)morpholin mit 1-(4-Hydroxyphenyl)-propan-2-on und anschließender Reinigung der Base über eine Kieselgelsäule mit Toluol/Methanol = 9:1 als Eluens.

Ausbeute: 46 % der Theorie,

| | | | | |
|---|---|---|---|---|
| Ber.: | C 64,12 | H 6,96 | N 8,80 | S 10,07 |
| Gef.: | 63,90 | 7,03 | 8,73 | 9,83 |

Laut $^1$H-NMR-Spektrum (400 MHz) liegt ein ca. 50:50-Diastereomerengemisch vor.

Beispiel 43

N-[2-(4-Carbomethoxymethoxyphenyl)ethyl]-2-hydroxy-2-(2-trifluormethyl-thiazol-4-yl)ethanamin

Hergestellt analog Beispiel 3 durch Umsetzung von 2-Trifluormethyl-4-bromacetyl-thiazol mit 2-(4-Carbomethoxymethoxyphenyl)ethanamin und anschließender Reduktion.

Ausbeute: 15 % der Theorie,

Schmelzpunkt: 91-92 °C

| | | | | |
|---|---|---|---|---|
| Ber.: | C 50,49 | H 4,74 | N 6,93 | S 7,93 |
| Gef.: | 50,74 | 4,94 | 6,84 | 8,10 |

Beispiel 44

N-[3-(4-Carboxamidophenyl)-l-methylpropyl]-2-hydroxy-2-(2-methyl-thiazol-4-yl)ethanamin

Hergestellt analog Beispiel 13 durch Umsetzung von 2-Hydroxy-2-(2-methyl-thiazol-4-yl)ethanamin mit 1-(4-Carboxamidophenyl)butan-3-on.

Ausbeute: 46 % der Theorie,

Schmelzpunkt: 94-95 °C

| | | | | |
|---|---|---|---|---|
| Ber.: | C 61,24 | H 6,95 | N 12,60 | S 9,61 |
| Gef.: | 61,50 | 7,15 | 12,34 | 9,65 |

Laut $^1$H-NMR-Spektrum (400 MHz) liegt ein 50:50-Gemisch der Diastereomeren vor.

Beispiel 45

N-[3-(4-Carboxamidophenyl)-l-methylpropyl]-2-(2-trifluormethyl-thiazol-4-yl)morpholin

Hergestellt analog Beispiel 32 durch Umsetzung von N-(2-Hydroxyethyl)-3-(4-carboxamidophenyl)-1-methylpropylamin mit 2-Trifluormethyl-4-bromacetyl-thiazol und anschließender Reduktion mit Triethylsilan.

Ausbeute: 19,7 % der Theorie,

Schmelzpunkt: 95-105 °C

| | | | | |
|---|---|---|---|---|
| Ber.: | C 55,19 | H 5,36 | N 10,16 | S 7,76 |
| Gef.: | 55,20 | 5,45 | 9,98 | 7,91 |

$^1$H-NMR-Spektrum ($CDCl_3$):

$\delta$ = 4.74 ppm (dd, 1H)

$\delta$ = 4,815 ppm (dd, 1H)

Laut $^1$H-NMR-Spektrum (400 MHz) liegt ein 48:52-Gemisch der Diastereomeren vor.

Beispiel 46

N-[2-(4-Carbomethoxymethoxyphenyl)ethyl]-2-hydroxy-2-(2-methyl-thiazol-4-yl)ethanamin-hydrochlorid

a) N-(4-Hydroxyphenyl-acetyl)-2-hydroxy-2-(2-methyl-thiazol-4-yl)ethanamin

3,16 g (20 mMol) 2-Hydroxy-2-(2-methyl-thiazol-4-yl)ethanamin werden in 80 ml absolutem Tetrahydrofuran gelöst und nacheinander mit 3,84 g (20 mMol) 4-Hydroxy-phenylessigsäure, 6,3 g (24 mMol) Triphenylphosphin, 5,6 ml (40 mMol) Triethylamin und 2 ml (20 mMol) Tetrachlorkohlenstoff versetzt. Nach Rühren über Nacht engt man ein, nimmt in 2N Salzsäure auf und extrahiert dreimal mit Methylenchlorid. Anschließend wird die wässrige Phase mit 2N Natronlauge auf pH 7 eingestellt und zur Trockne eingeengt. Der Einengungsrückstand wird mit einem Gemisch aus Chloroform/Methanol (1:1) mehrmals ausgekocht. Die Extrakte werden eingeengt und der Rückstand über eine Kieselgelsäule mit Essigsäureethylester/Methanol = 50:1 als Eluens gereinigt.
Ausbeute: 4,4 g Öl (75,9 % der Theorie),

| | | | |
|---|---|---|---|
| Ber.: | C 57,51 | H 5,52 | N 9,58 |
| Gef.: | 57,63 | 5,59 | 9,41 |

b) N-(2-(4-Hydroxyphenyl)ethyl]-2-hydroxy-2-(2-methyl-thiazol-4-yl)ethanamin-hydrochlorid

4,2 g (14,4 mMol) N-(4-Hydroxyphenyl-acetyl)-2-hydroxy-2-(2-methyl-thiazol-4-yl)ethanamin werden in 30 ml absolutem Tetrahydrofuran gelöst und einer am Rückfluß siedenden Suspension von 1,37 g (36 mMol) Lithiumaluminiumhydrid in 30 ml absolutem Tetrahydrofuran zugetropft. Nach 1 Stunde wird abgekühlt, mit 2N Natronlauge zersetzt und eingeengt. Dann wird mit 20 ml 2N Salzsäure aufgenommen und mit wässrigem Ammoniak alkalisch gestellt. Nach dem erneuten Einengen wird mit Chloroform/Methanol = 10:1 heiß extrahiert. Die Extrakte werden eingeengt und über eine Kieselgelsäule mit Essigsäureethylester/Methanol = 4:1 als Eluens gereinigt. Anschließend wird in Essigsäureethylester mit etherischer Salzsäure das Hydrochlorid gefällt.
Ausbeute: 760 mg (19 % der Theorie),
Schmelzpunkt: 110-113 °C

| | | | | |
|---|---|---|---|---|
| Ber.: | C 53,41 | H 6,08 | N 8,90 | Cl 11,26 |
| Gef.: | 53,12 | 6,04 | 8,80 | 11,31 |

c) N-[2-(4-Carbomethoxymethoxyphenyl)ethyl]-2-hydroxy-2-(2-methyl-thiazol-4-yl)ethanamin hydrochlorid

Zu 280 mg (1 mMol) N-[2-(4-Hydroxyphenyl)ethyl]-2-hydroxy-2-(2-methyl-thiazol-4-yl)ethanamin-hydrochlorid, gelöst in 7 ml absolutem Dimethylformamid, gibt man 100 mg (2,1 mMol) Natriumhydrid-Dispersion (50%ig in Paraffinöl). Nach 15 Minuten Rühren bei Raumtemperatur tropft man eine Lösung von 153 mg (1 mMol) Bromessigsäure-ethylester in 3 ml absolutem Dimethylformamid rasch zu. Anschließend wird über Nacht gerührt, mit 20 ml gesättigter Natriumbicarbonatlösung versetzt und mit Methylenchlorid extrahiert. Die Extrakte werden getrocknet, eingeengt und nach Lösen in Ether/Methanol mit etherischer Salzsäure das Hydrochlorid gefällt.
Ausbeute: 20 mg (21 % der Theorie),
Schmelzpunkt: 163-165 °C (Zers.)

| | | | |
|---|---|---|---|
| Ber.: | C 52,78 | H 5,99 | N 7,24 |
| Gef.: | 52,41 | 5,76 | 7,32 |

Beispiel 47

N-[2-(4-Carbomethoxymethoxyphenyl)ethyl]-2-(2-trifluormethylthiazol-4-yl)morpholin

a) N-[2-(4-Methoxy-phenyl)ethyl]-2-(2-trifluormethyl-thiazol-4-yl)morpholin

Hergestellt analog Beispiel 32 durch Umsetzung von N-(2-Hydroxyethyl)-2-(4-methoxyphenyl)ethanamin mit 2-Trifluormethyl-4-bromacetyl-thiazol und anschließender Reduktion mit Triethylsilan.
Ausbeute: 24 % der Theorie, Öl,

| | | | | |
|---|---|---|---|---|
| Ber.: | C 54,82 | H 5,14 | N 7,52 | S 8,61 |
| Gef.: | 55,00 | 5,24 | 7,42 | 8,86 |

b) N-[2-(4-Carbomethoxymethoxyphenyl)ethyl]-2-(2-trifluormethyl-thiazol-4-yl)morpholin

0,6 g (1,6 mMol) N-[2-(4-Methoxyphenyl)ethyl]-2-(2-trifluormethyl-thiazol-4-yl)morpholin werden mit 6 ml

48%iger wässriger Bromwasserstoffsäure 3 Stunden auf dem Dampfbad erhitzt. Dann wird eingeengt, mit Toluol versetzt und erneut zur Trockne eingeengt. Der schaumige Rückstand wird in I5 ml Aceton mit I,2 g (8,7 mMol) Kaliumcarbonat und 0,3 ml (I,65 mMol) Bromessigsäure-methylester eine Stunde am Rückfluß gekocht. Anschließend wird filtriert, das Filtrat eingeengt und der Rückstand über eine Kieselgelsäule mit Toluol/Aceton = 4:I gereinigt.
Ausbeute: 0,5 g (72 % der Theorie),

| | | | | |
|---|---|---|---|---|
| Ber.: | C 53,0I | H 4,92 | N 6,5I | S 7,45 |
| Gef.: | 53,07 | 4,88 | 6,72 | 7,62 |

Beispiel 48

N-[2-(4-Carboethoxymethoxyphenyl)-I-methylethyl]-2-(2-trifluormethyl-thiazol-4-yl)morpholin

a) N-[2-(4-Hydroxyphenyl)-I-methylethyl]-2-(2-trifluormethylthiazol-4-yl)morpholin

Hergestellt analog Beispiel 32 durch Umsetzung von 2-Trifluormethyl-4-bromacetyl-thiazol mit N-[2-(4-Hydroxyphenyl)-I-methylethyl]-2-hydroxy-ethanamin und anschließender Reinigung der Base über eine Kieselgelsäule mit Chloroform/Essigester = 3:I als Eluens.
Ausbeute: 29 % der Theorie,

| | | | |
|---|---|---|---|
| Ber.: | C 54,83 | H 5,I4 | N 7,52 |
| Gef.: | 53,83 | 5,07 | 6,93 |

Laut $^1$H-NMR-Spektrum (400 MHz) liegt ein 50:50-Gemisch der Diastereomeren vor.

b) N-[2-(4-Carboethoxymethoxyphenyl)-I-methylethyl]-2-(2-trifluormethyl-thiazol-4-yl)morpholin

280 mg (0,75 mMol) N-[2-(4-Hydroxyphenyl)-I-methylethyl]-2-(2-trifluormethyl-thiazol-4-yl)morpholin werden mit I0I mg (0,82 mMol) Chloressigsäure-ethylester und II3 mg (0,82 mMol) Kaliumkarbonat in 2 ml wasserfreiem Aceton 6 Stunden am Rückfluß gekocht. Danach wird abgesaugt, der Rückstand 2 mal mit Aceton gewaschen und das Filtrat eingeengt. Die rohe Base wird über eine Kieselgelsäule mit Chloroform/Methanol = 20:I als Eluens gereinigt.
Ausbeute: 50 % der Theorie,

| | | | | |
|---|---|---|---|---|
| Ber.: | C 55,0I | H 5,50 | N 6,II | S 6,99 |
| Gef.: | 55,48 | 5,62 | 5,85 | 6,62 |

Beispiel 49

N-[2-(4-Carbomethoxymethoxyphenyl)-I-methylethyl]-2-(2-isopropyl-thiazol-4-yl)morpholin-6-on-hydrochlorid

Eine Lösung von 0,50 g (I,27 mMol) N-[2-(4-Methoxycarbonylmethoxy-phenyl)-I-methylethyl]-2-hydroxy-2-(2-isopropyl-thiazol-4-yl)ethanamin in 5 ml wasserfreiem Dimethylformamid wird nacheinander mit 0,I94 g (I,27 mMol) Bromessigsäure-methylester, 0,I75 g (I,27 mMol) Kaliumkarbonat und einem kleinen Kristall Kaliumjodid versetzt. Nach 4 Stunden Rühren bei 20 °C wird das Dimethylformamid im Vakuum abdestilliert und der Rückstand zwischen Chloroform und Wasser verteilt. Der getrocknete und filtrierte Chloroform-Extrakt wird im Vakuum eingeengt und der Rückstand durch Säulenchromatographie an Kieselgel (Toluol/Aceton = 4/I) gereinigt.
Ausbeute: 0,20 g (36 % der Theorie), viskoses Öl.
Laut $^1$H-NMR-Spektrum (400 MHz, $CDCl_3/CD_3OD$) liegt ein 50:50-Gemisch der Diastereomeren vor.
$\delta$ = 5,59 ppm (dd, =CH-O-CO-)
$\delta$ = 5,62 ppm (dd, =C$\underline{H}$-O-CO-)

Durch Behandeln der Base mittels Chlorwasserstoff/Ether und anschließendes Trocknen bei 20 °C und 0,I Torr erhält man ein schaumartiges Hydrochlorid.
Schmelzbereich: 40-50 °C.

| | | | |
|---|---|---|---|
| Ber.: (x I,2 HCl) | C 54,44 | H 6,27 | Cl 8,77 |
| Gef.: | 54,70 | 6,57 | 8,48 |

Beispiel 50

N-[2-(4-Carbomethoxymethoxyphenyl)-l-methylethyl]-2-(2-isopropyl-thiazol-4-yl)morpholin

a) N-[2-(4-Carbomethoxymethoxyphenyl)-l-methylethyl]-2-(2-isopropyl-thiazol-4-yl)morpholin-5-on

Zur gerührten Lösung von 3,l0 g (7,89 mMol) N-[2-(4-Methoxycarbonylmethoxyphenyl)-l-methylethyl]-2-hydroxy-2-(2-isopropyl-thiazol-4-yl)ethanamin und von l,l0 ml (7,89 mMol) Triäthylamin in 20 ml Chloroform wird bei 25° C Innentemperatur 0,60 ml (7,89 mMol) Chloracetylchlorid zugetropft. Nach Stehen über Nacht bei 20° C wird im Vakuum eingeengt und der Rückstand in 30 ml wasserfreiem Dimethylformamid gelöst. Dazu gibt man 0,888 g (l5,78 mMol) einer 55%igen Dispersion von Natriumhydrid in Öl, wobei kurzes Aufschäumen beobachtet wird. Es wird 3 Stunden bei 20° C gerührt, im Vakuum eingeengt und der Rückstand zwischen Wasser und Ether verteilt. Die Ether-Lösung wird nach Trocknen und Filtrieren im Vakuum eingeengt und der ölige Rückstand über Kieselgel (Toluol/Aceton = 4:l) gereinigt.
Ausbeute: l,40 g (4l % der Theorie),
Schmelzbereich: 60-70° C

| | | | | |
|---|---|---|---|---|
| Ber.: | C 6l,l0 | H 6,53 | N 6,48 | S 7,4l |
| Gef.: | 6l,20 | 6,57 | 6,77 | 7,58 |

Laut $^1$H-NMR-Spektrum (400 MHz, $CDCl_3$) liegt ein 50:50-Gemisch der Diastereomeren vor.
δ = 4,72 ppm (dd, =CH-O-)
δ = 4,85 ppm (dd, =CH-O-)

b) N-[2-(4-Carbomethoxymethoxyphenyl)-l-methylethyl]-2-(2-isopropyl-thiazol-4-yl)morpholin

Zu 0,425 ml (4,56 mMol) Phosphoroxychlorid werden 0,200 g (0,462 mMol) N-[2-(4-Carbomethoxymethoxyphenyl)-l-methylethyl]-2-(2-isopropyl-thiazol-4-yl)morpholin-5-on gegeben. Nach l5-minütigem Rühren bei 20° C wird im Vakuum eingeengt und der Rückstand in 4 ml l,2-Dimethoxy-ethan gelöst. Nach Zugabe von 0,052 g (l,39 mMol) Natriumborhydrid und Rühren über Nacht bei 20° C wird im Vakuum eingeengt und der Rückstand zwischen Chloroform und Wasser verteilt. Nach Trocknen und Filtrieren und Einengen der Chloroform-Lösung wird eine Stunde bei l00° C mit Chlorwasserstoff-Lösung erhitzt. Nach Einengen im Vakuum wird der Rückstand zwischen Chloroform und wäßriger Soda-Lösung verteilt und der Chloroform-Extrakt über Kieselgel (Toluol/Aceton = 3:l) gereinigt.
Ausbeute: 0,028 g (l4,5 % der Theorie),
Ber.: Molpeak m/e = 4l8
Gef.: Basepeak m/e = 239
Laut $^1$H-NMR-Spektrum (400 MHz, $CDCl_3/CD_3OD$) liegt ein 50:50-Gemisch der Diastereomeren vor.
δ = 4,72 ppm (dd, =CH-O-)
δ = 4,74 ppm (dd, =CH-O-)

Beispiel 5l

N-[2-(4-Carbomethoxymethoxyphenyl)-l-methylethyl]-2-hydroxy-2-(2-isopropyl-thiazol-4-yl)ethanamin-dihydrochlorid

Hergestellt analog Beispiel l3 durch Umsetzung von 2-Hydroxy-2-(2-isopropyl-thiazol-4-yl)ethanamin mit l-(4-Carbomethoxymethoxyphenyl)-propan-2-on und anschließende Reinigung der Base durch Säulenchromatographie an Kieselgel (Chloroform/Methanol = l0:l).
Ausbeute: 89 % der Theorie,
Ber.: Molpeak m/e = 4l8
Gef.: Basepeak m/e = 239
$^1$H-NMR-Spektrum (400 MHz, $CDCl_3$):
δ = 4,80 ppm (dd, =CH-OH)
δ = 4,84 ppm (dd, =CH-OH)
Laut $^1$H-NMR-Spektrum liegt ein ca. 50:50-Gemisch der Diastereomeren vor.
Zur Überführung in das Dihydrochlorid wird die Base mit Chlorwasserstoff/Diethylether behandelt. Nach Abdampfen des Ethers im Vakuum wird drei Tage bei 20° C und 0,l Torr getrocknet.
Schmelzbereich: 55-70° C

| | | | | |
|---|---|---|---|---|
| Ber.: | C 5l,6l | H 6,49 | N 6,02 | S 6,88 |
| Gef.: | 5l,40 | 6,64 | 5,86 | 6,88 |

Beispiel 52

N-[2-(4-Carbomethoxymethoxyphenyl)-l-methylethyl]-2-hydroxy-2-(2-propyl-thiazol-4-yl)ethanamin-dihydrochlorid

Hergestellt analog Beispiel l3 durch Umsetzung von 2-Hydroxy-2-(2-propyl-thiazol-4-yl)ethanamin mit l-(4-Carbomethoxymethoxy-phenyl)propan-2-on und anschließende Reinigung der Base durch Säulenchromatographie an Kieselgel (Chloroform/Methanol = l0:l).
Ausbeute: 52 % der Theorie,
$^1$H-NMR-Spektrum (400 MHz, $CDCl_3/CD_3OD$):
$\delta$ = 4,72 ppm (dd, =CH-OH)
$\delta$ = 4,78 ppm (dd, =C$\underline{H}$-OH)
Laut $^1$H-NMR-Spektrum liegt ein ca. 50:50-Gemisch der Diastereomeren vor.
Zur Überführung in das Dihydrochlorid wird die viskose Base mit Chlorwasserstoff/Methanol behandelt. Nach Abdampfen des Methanols im Vakuum wird zunächst bei 0,l Torr und 40 bis 50 °C getrocknet und anschließend über Nacht bei 35 °C und 0,l Torr über Phosphorpentoxid getrocknet.
Schmelzbereich: 50-70 °C

| | | | | |
|---|---|---|---|---|
| Ber.: | C 5l,6l | H 6,49 | N 6,02 | S 6,88 |
| Gef.: | 5l,38 | 6,l3 | 6,28 | 7;00 |

Beispiel 53

N-[2-(4-Carboxymethoxyphenyl)-l-methylethyl]-2-hydroxy-2-[2-propyl-thiazol-4-yl)ethanamin

Hergestellt analog Beispiel l6 durch Umsetzung von N-[2-(4-Carbomethoxymethoxyphenyl)-l-methylethyl]-2-hydroxy-2-(2-propyl-thiazol-4-yl)ethanamin in Methanol und lN Natronlauge. Nach Neutralisieren mit lN Salzsäure wird im Vakuum eingeengt und dann zwischen Chloroform und Wasser verteilt. Der Chloroform-Extrakt wird über Natriumsulfat getrocknet, filtriert und im Vakuum eingedampft. Der Eindampfrückstand ergibt beim Verreiben mit Ether eine pulvrige Festsubstanz, die bei 50 °C und 0,l Torr 6 Stunden getrocknet wird.
Ausbeute: 79 % der Theorie,
Schmelzbereich: 79-85 °C

| | | | | |
|---|---|---|---|---|
| Ber. (x 0,75 $H_2O$): | C 58,23 | H 7,07 | N 7,l5 | S 8,l8 |
| Gef. : | 58,l0 | 6,75 | 6,9l | 8,56 |

Laut $^1$H-NMR-Spektrum (400 MHz, $CDCl_3/CD_3OD$) liegt ein 50:50-Gemisch der Diastereomeren vor.
$\delta$ = 5,l4 ppm (dd, =CH-OH)
$\delta$ = 5,l7 ppm (dd, =C$\underline{H}$-OH)

Beispiel 54

N-[2-(4-Carboxymethoxyphenyl)-l-methylethyl]-2-hydroxy-2-(2-isopropyl-thiazol-4-yl)ethanamin-hydrochlorid

Hergestellt analog Beispiel l6 durch Umsetzung von N-[2-(4-Carbomethoxymethoxyphenyl) l-methylethyl]-2-hydroxy-2-(2-isopropyl-thiazol-4-yl)ethanamin in Methanol mit lN Natronlauge. Nach Zugabe von lN Salzsäure bis zum Erreichen von pH=6 wird im Vakuum eingeengt und dann zwischen Chloroform und Wasser verteilt. Der getrocknete und filtrierte Chloroform-Extrakt wird im Vakuum eingedampft. Beim Verreiben des Eindampfrückstandes mit Ether erhält man eine schaumartige Festsubstanz, die selbst nach mehrstündigem Trocknen bei 30 °C und 0,l Torr über Phosphorpentoxid noch ca. 5 % Chloroform enthält.
Ausbeute: 22 % der Theorie,
Schmelzbereich: 80-90 °C

| | | | | |
|---|---|---|---|---|
| Ber. (+ 5 % $CHCl_3$): | C 54,35 | H 6,48 | N 6,66 | Cl 9,69 |
| Gef. : | 54,l9 | 6,27 | 6,53 | 9,26 |

Laut $^1$H-NMR-Spektrum (400 MHz, $d_6$-DMSO) liegt ein ca. 50:50-Gemisch der Diastereomeren vor.
$\delta$ = 5,00 ppm (dd, =CH-OH)
$\delta$ = 5,04 ppm (dd, =C$\underline{H}$-OH)

Beispiel 55

N-[2-(4-Methoxyphenyl)-l-methylethyl]-2-hydroxy-2-(2-trifluorthiazol-4-yl)ethanamin-hydrochlorid

Hergestellt analog Beispiel l3 durch Umsetzung von 2-Hydroxy-2-(2-trifluormethyl-thiazol-4-yl)ethanamin mit l-(4-Methoxyphenyl)-propan-2-on, anschließender Reinigung der Base über eine Kieselgelsäule mit Essigester/Methanol = 92:8 als Eluens und Fällung des Hydrochlorids mit etherischer Salzsäure.
Ausbeute: 57 % der Theorie,
Schmelzpunkt: l47-l49° C (Zers.)
Ber.: C 48,42 H 5,08 N 7,06 Cl 8,93
Gef.: 48,65 5,39 7,ll 9,l9
Laut $^1$H-NMR-Spektrum (400 MHz) liegt ein l:l-Gemisch der Diastereomeren vor.

Beispiel 56

3-[2-(4-Methoxyphenyl)-l-methylethyl]-5-(2-trifluormethylthiazol-4-yl)-2-oxazolidincarbonsäure-methylester

Hergestellt analog Beispiel l8 durch Umsetzung von N-[2-(4-methoxyphenyl)-l-methylethyl]-2-hydroxy-2-(2-trifluormethylthiazol-4-yl)ethanamin mit Methylglyoxylat und anschließender Reinigung der Base über eine Kieselgelsäule mit Chloroform/Petroläther/Essigester = 5:4,5:0,5 als Eluens.
Ausbeute: 25 % der Theorie,
Ber.: C 53,02 H 4,92 N 6,5l S 7,45
Gef.: 53,29 4,86 6,32 7,54

Beispiel 57

N-[2-(4-Hydroxyphenyl)-l-methylethyl]-2-hydroxy-2-(2-trifluorthiazol-4-yl)ethanamin

Hergestellt analog Beispiel l3 durch Umsetzung von 2-Hydroxy-2-(2-trifluormethyl-thiazol-4-yl)ethanamin mit l-(4-Hydroxyphenyl)-propan-2-on und anschließender Reinigung der Base über eine Kieselgelsäule mit Chloroform/Methanol = 9:l als Eluens.
Ausbeute: 63 % der Theorie,
Schmelzpunkt: ab 77° C, klare Schmelze ab 97° C
Ber.: C 52,0l H 4,95 N 8,09 S 9,26
Gef.: 52,05 4,98 8,l7 9,l9
Laut $^1$H-NMR-Spektrum (400 MHz) liegt ein l:l-Gemisch der Diastereomeren vor.

Beispiel 58

N-[2-(4-Hydroxyphenyl)-l-methylethyl]-2-hydroxy-2-(2-methyl-thiazol-4-yl)ethanamin

Hergestellt analog Beispiel l3 durch Umsetzung von 2-Hydroxy-2-(2-methyl-thiazol-4-yl)ethanamin mit l-(4-Hydroxyphenyl)propan-2-on und anschließender Reinigung der Base über eine Kieselgelsäule mit Chloroform/Methanol/Ammoniak = 9:l:l als Eluens.
Ausbeute: 52 % der Theorie,
Schmelzpunkt: l46-l54° C
Ber.: C 6l,62 H 6,89 N 9,58 S l0,97
Gef.: 6l,96 6,90 9,65 ll,24
Laut $^1$H-NMR-Spektrum (400 MHz) liegt ein 70:30-Gemisch der Diastereomeren vor.

Beispiel 59

N-[2-(4-Methoxyphenyl)-l-methylethyl]-2-(2-trifluormethyl-thiazol-4-yl)morpholin

Hergestellt analog Beispiel l3 durch Umsetzung von 2-(2-Trifluormethyl-thiazol-4-yl)morpholin mit 4-Methoxyphenyl-propan-2-on und anschließender Reinigung der Base über eine Kieselgelsäule mit Chloroform/Essigester = 9:l als Eluens.
Ausbeute: 80 % der Theorie,
Schmelzpunkt: l46-l54° C
Ber.: C 55,95 H 5,48 N 7,25

Gef.: 56,09 5,62 6,83
Laut $^1$H-NMR-Spektrum (400 MHz) liegt ein 50:50-Gemisch der Diastereomeren vor.

Beispiel 60

3-[2-(4-(2-Carbomethoxy-l-methylethenyl)phenyl)-l-methyl-ethyl]-5-(2-trifluormethyl-thiazol-4-yl)-2-oxazolidincarbonsäure-methylester

Hergestellt analog Beispiel l8 durch Umsetzung von N-[2-(4-(2-Carbomethoxy-l-methylethenyl)phenyl)-l-methyl-ethyl]-2-hydroxy-2-(2-trifluormethyl-thiazol-4-yl)ethanamin mit Methylglyoxylat und anschließender Reinigung der Base über eine Kieselgelsäule mit Chloroform/Petrolether/Essigester = 5:4,5:0,5 als Eluens.
Ausbeute: 66 % der Theorie,
Ber.: C 55,4l H 5,05 N 5,62 S 6,43
Gef.: 55,33 5,23 4,96 6,64
$^1$H-NMR-Spektrum ($CDCl_3/CD_3OD$):
$\delta$ = 5,l0 ppm (s, 2H)
5,23 ppm (s, lH)
5,27 ppm (s, lH)
Laut $^1$H-NMR-Spektrum (400 MHz) liegt ein l:l:l:l-Gemisch der Diastereomeren vor.

Beispiel 6l

3-[2-(4-Hydroxyphenyl)-l-methylethyl]-5-(2-trifluormethylthiazol-4-yl)-2-oxazolidincarbonsäure-methylester

Hergestellt analog Beispiel l8 durch Umsetzung von N-[2-(4-Hydroxyphenyl)-l-methylethyl]-2-hydroxy-2-(2-trifluormethyl-thiazol-4-yl)ethanamin mit Methylglyoxylat, anschließender Reinigung der Base über eine Kieselgelsäule mit Chloroform/Essigester = 9:l als Eluens und Fällung des Hydrochlorids mit etherischer Salzsäure.
Ausbeute: 37 % der Theorie,
Schmelzpunkt: ab l58° C (Zers.)
Ber.: C 47,74 H 4,45 N 6,l9 S 7,08 Cl 7,88
Gef.: 47,49 4,72 6,38 7,22 7,98

Beispiel 62

3-[2-(4-(2-Carbomethoxy-l-methylethenyl)phenyl)-l-methylethyl]-5-(2-methyl-thiazol-4-yl)-2-oxazolidincarbonsäuremethylester

Hergestellt analog Beispiel l8 durch Umsetzung von N-[2-(4-(2-Carbomethoxy-l-methylethenyl)phenyl)-l-methylethyl]-2-hydroxy-2-(2-methyl-thiazol-4-yl)ethanamin mit Methylglyoxylat und anschließender Reinigung der Base über eine Kieselgelsäule mit Petrolether/Essigester = 7:3 als Eluens.
Ausbeute: l8 % der Theorie, Öl,
Ber.: C 62,l4 H 6,35 N 6,30 S 7,2l
Gef.: 6l,90 6,60 6,34 7,03

Beispiel 63

3-[2-(4-Hydroxyphenyl)-l-methylethyl]-5-(2-methyl-thiazol-4-yl)-2-oxazolidincarbonsäure-methylester

Hergestellt analog Beispiel l8 durch Umsetzung von N-[2-(4-Hydroxyphenyl)-l-methylethyl]-2-hydroxy-2-(2-methyl-thiazol-4-yl)ethanamin mit Methylglyoxylat und anschließender Reinigung der Base über eine Kieselgelsäule mit Ether/Petrolether = 8:2 als Eluens.
Ausbeute: 25 % der Theorie, Öl,
Ber.: C 59,65 H 6,l2 N 7,73 S 8,85
Gef.: 60,00 5,98 7,23 8,97

Beispiel 64

N-[2-(4-Carbomethoxymethoxyphenyl)-l-methylethyl]-2-(2-trifluormethyl-thiazol-4-yl)morpholin-6-on

2,l g (0,005 Mol) N-[2-(4-Carbomethoxymethoxyphenyl)-l-methylethyl]-2-hydroxy-2-(2-trifluormethyl-thiazol-4-yl)ethanamin werden in 70 ml Aceton gelöst und unter Rühren 5 ml Bromessigsäuremethylester und 5 g Kaliumcarbonat zugegeben. Zunächst wird l6 Stunden bei Raumtemperatur gerührt und anschließend 4 Stunden am Rückfluß erhitzt. Die anorganischen Produkte werden abfiltriert, das Lösungsmittel abdestilliert und der erhaltene Rückstand über eine Kieselgelsäule mit Toluol/Essigester = 8,5:l,5 als Eluens gereinigt.

Ausbeute: 0,8 g Öl (35 % der Theorie),

Ber.: C 52,39 H 4,62 N 6,ll

Gef.: 52,50 4,5l 5,85

$^1$H-NMR-Spektrum (400 MHz) ($CDCl_3$/$CD_3OD$):

$\delta$ = 7,596 ppm (d, lH)

$\delta$ = 7,686 ppm (d, lH)

Danach liegt ein 50:50-Gemisch der Diastereomeren vor.

Beispiel 65

N-[2-(4-Carboethoxymethoxyphenyl)-l-methylethyl]-2-(2-trifluormethyl-thiazol-4-yl)morpholin-hydrochlorid

0,5 g (0,00l2 Mol) N-[2-(4-Carboxymethoxyphenyl)-l-methylethyl]-2-(2-trifluormethyl-thiazol-4-yl)-morpholin werden in l50 ml Chlorofom gelöst, unter Rühren mit 2 ml Ethanol und 0,25 g konzentrierter Schwefelsäure versetzt und anschließend für l Stunde am Wasserabscheider zum Rückfluß erhitzt. Danach wird abgekühlt, mit Eiswasser versetzt, mit Ammoniak alkalisch gestellt und die Phasen getrennt. Anschließend schüttelt man erneut mit Chloroform aus, trocknet die organische Phase über Natriumsulfat, filtriert ab und engt zur Trockene ein. Der erhaltene Rückstand wird in Ether gelöst, mit etherischer Salzsäure versetzt, zur Trockene eingeengt, mit Aceton verrieben und abgesaugt.

Ausbeute: 0,37 g (63 % der Theorie),

Schmelzpunkt: l22-l23 °C

Ber.: C 50,95 H 5,30 N 5,66

Gef.: 50,85 5,49 5,68

Laut $^1$H NMR-Spektrum (400 MHz) liegt ein 50:50-Gemisch der Diastereomeren vor.

Beispiel 66

N-[2-(4-Carbomethoxymethoxyphenyl)-l-methylethyl]-2-hydroxy-2-(2-methyl-thiazol-5-yl)ethanamin-dihydrochlorid

Hergestellt analog Beispiel l3 durch Umsetzung von 2-Hydroxy-2-(2-methyl-thiazol-5-yl)ethanamin mit l-(4-Carbomethoxymethoxyphenyl)propan-2-on, Reinigen der Base über eine Kieselgelsäule mit Essigester/Ethanol = 8:2 als Eluens und anschließender Fällung des Dihydrochlorids mit etherischer Salzsäure.

Ausbeute: 32 % der Theorie,

Schmelzpunkt: l90-l92 °C

Ber.: C 49,43 H 5,99 N 6,40

Gef.: 49,43 5,90 6,49

Laut $^1$H-NMR-Spektrum (400 MHz) liegt ein ca. 50:50-Gemisch der Diastereomeren vor.

Beispiel 67

N-[2-(4-Carbomethoxymethoxyphenyl)-l-methylethyl]-2-hydroxy-2-(2-trifluormethyl-thiazol-4-yl)ethanamin (Diastereomer B)

Hergestellt analog Beispiel l3 durch Umsetzung von 2-Hydroxy-2-(2-trifluormethyl-thiazol-4-yl)ethanamin mit l-(4-Carbomethoxymethoxyphenyl)propan-2-on und Natriumcyanborhydrid in Methanol (Reaktionszeit: 5 Stunden) und anschließender Reinigung über eine Kieselgelsäule mit Methylenchlorid/Methanol = 20:l. Dabei erhält man ein 50:50 Diastereomerengemisch der Base. Diese wird aus einem Gemisch Ether/Essigester = 65:l0 umkristallisiert. Die dabei erhaltene Mutterlauge wird mit etherischer Salzsäure

versetzt und zur Trockene gebracht. Den dabei erhaltenen Rückstand kristallisiert man aus einem Gemisch Ether/Essigester/Methanol = l00:60:l um. Die nach dem Abfiltrieren der Kristalle erhaltene Mutterlauge wird zur Trockene eingeengt, durch alkalisches Ausschütteln mit Methylenchlorid wird die Base freigesetzt und über eine Kieselgelsäule mit Methylenchlorid/Methanol = 20:l als Eluens gereinigt. Dabei erhält man das Diastereomer B in ca. 92-94 % Reinheit als Öl.
Ausbeute: 4 % der Theorie,
Ber.: C 54,66 H 5,06 N 6,70
Gef.: 54,43 5,l3 6,88
$^1$H-NMR-Spektrum (400 MHz) ($CDCl_3/CD_3OD$):
$\delta$ = 7,56 ppm (s,lH)

Beispiel 68

N-[2-(4-Carbomethoxymethoxyphenyl)-l-methylethyl]-2-hydroxy-2-(2-trifluormethyl-thiazol-4-yl)ethanamin (Diastereomer A)

Hergestellt analog Beispiel l3 durch Umsetzung von 2-Hydroxy-2-(2-trifluormethyl-thiazol-4-yl)ethanamin mit l-(4-Carbomethoxymethoxyphenyl)propan-2-on und Natriumcyanborhydrid in Methanol (Reaktionszeit: 5 Stunden) und anschließender Reinigung über eine Kieselgelsäule mit Methylenchlorid/Methanol = 20:l. Dabei erhält man ein ca. 50:50 Diastereomerengemisch der Base. Dieses wird aus einem Gemisch Ether/Essigester = 65:l0, sowie weitere zweimal aus Essigester umkristallisiert. Man erhält dabei das Diastereomer A in 98-99 % Reinheit.
Ausbeute: l4 % der Theorie,
Schmelzpunkt: l04-l05 °C
Ber.: C 5l,66 H 5,06 N 6,70
Gef.: 5l,90 4,82 6,82
$^1$H NMR-Spektrum (400 MHz) ($CDCl_3/CD_3OD$):
$\delta$ = 7,59 ppm (s,lH)

Beispiel 69

N-[2-(4-Carboxymethoxyphenyl)-l-methylethyl]-2-trifluormethylthiazol-4-yl)morpholin (Diastereomer A)

Hergestellt analog Beispiel l6 durch Umsetzung von N-[2-(4-Carbomethoxymethoxyphenyl)-l-methylethyl]-2-(2-trifluormethylthiazol-4-yl)morpholin (Diastereomer A) in Methanol mit ln Natronlauge. Nach Neutralisieren mit ln Salzsäure wird mit Methylenchlorid ausgeschüttelt, der Extrakt zur Trockene eingeengt und der verbleibende Rückstand mit Petrolether verrieben und abgesaugt.
Ausbeute: 96 % der Theorie,
Schmelzpunkt: ab 70 °C (Sint.)
Ber.: C 53,0l H 4,92 N 6,5l
Gef.: 53,l5 4,97 6,53
$^1$H-NMR-Spektrum (400 MHz) ($CDCl_3/CD_3OD$):
$\delta$ = 7,757 ppm (s,lH)

Beispiel 70

N-[2-(4-Carboxymethoxyphenyl)-l-methylethyl]-2-(2-trifluormethyl-thiazol-4-yl)morpholin (Diastereomer B)

Hergestellt analog Beispiel l6 durch Umsatz von N-[2-(4-Carbomethoxymethoxyphenyl)-l-methylethyl]-2-(2-trifluormethylthiazol-4-yl)morpholin (Diastereomer B) in Methanol mit ln Natronlauge. Nach Neutralisieren mit ln Salzsäure wird mit Methylenchlorid ausgeschüttelt, der Extrakt zur Trockene eingeengt und der verbleibende Rückstand mit Petrolether verrieben und abgesaugt.
Ausbeute: 88 % der Theorie,
Schmelzpunkt: ab 70 °C (Sint.)
Ber.: C 53,0l H 4,92 N 6,5l
Gef.: 53,l9 5,l9 6,48
$^1$H-NMR-Spektrum (400 MHz) ($CDCl_3/CD_3OD$):
$\delta$ = 7,786 ppm (s,lH)

Danach enthält die Verbindung noch ca. 6-7 % Diastereomer A.

Beispiel 7l

N-[2-(4-(2-Hydroxyethoxy)phenyl)-l-methylethyl]-2-(2-trifluormethyl-thiazol-4-yl)morpholin (Diastereomer A)

l g (0,0022 Mol) N-[2-(4-Carbomethoxymethoxyphenyl)-l-methylethyl]-2-(2-trifluormethyl-thiazol-4-yl)-morpholin-5-on (Diastereomer A) werden in 6 ml absolutem Tetrahydrofuran gelöst. Bei Raumtemperatur werden l5 ml (0,0l5 Mol) einer l-molaren Lösung von Diboran in Tetrahydrofuran innerhalb l5-20 Minuten zugetropft. Die Lösung erwärmte sich dabei auf ca. 35-40 °C. Nach 30 Minuten wird zur Trockene eingeengt und der verbleibende Rückstand in 40 ml Methanol und 2 ml konzentrierter Salzsäure gelöst und für 30 Minuten stehen gelassen. Unter Eiskühlung wird mit Ammoniak alkalisch gestellt und mit Methylenchlorid mehrmals ausgeschüttelt. Die organische Phase wird über Natriumsulfat getrocknet und die Base über eine Kieselgelsäule mit Toluol/Essigester = 7,5:2,5 als Eluens gereinigt.
Ausbeute: 0,28 g (3l % der Theorie),
Schmelzpunkt: 76-78 °C
Ber.: C 54,79 H 5,57 N 6,73
Gef.: 54,90 5,7l 6,54
$^1$H-NMR-Spektrum (400 MHz) ($CDCl_3/CD_3OD$):
$\delta$ = 7,6l4 ppm (s,lH)

Beispiel 72

N-[2-(4-Carboxymethoxyphenyl)-l-methylethyl]-2-hydroxy-2-(2-trifluormethyl-thiazol-4-yl)ethanamin (Diastereomer A)

Hergestellt analog Beispiel l6 durch Umsetzung von N-[2-(4-Carbomethoxymethoxyphenyl)-l-methylethyl]-2-(2-trifluormethyl-thiazol-4-yl)ethanamin (Diastereomer A) in Methanol mit ln Natronlauge. Nach Neutralisieren mit ln Salzsäure wird mit Methylenchlorid ausgeschüttelt, der Extrakt zur Trockene eingeengt und der verbleibende Rückstand mit Petrolether verrieben und abgesaugt.
Ausbeute: 89 % der Theorie,
Schmelzpunkt: ll9-l2l °C
Ber.: C 50,49 H 4,74 N 6,93
Gef.: 50,62 4,69 6,90
$^1$H-NMR-Spektrum (400 MHz) ($CDCl_3/CD_3OD$):
$\delta$ = 7,77l ppm (s,lH)

Beispiel 73

N-[2-(4-Carboxymethoxyphenyl)-l-methylethyl]-N-(2-hydroxyethyl)-2-hydroxy-2-(2-trifluormethyl-thiazol-4-yl)-ethanamin

Hergestellt analog Beispiel l6 durch Umsetzung von N-[2-(4-Carbomethoxymethoxyphenyl)-l-methylethyl]-N-(2-hydroxyethyl)-2-hydroxy-2-(2-trifluormethyl-thiazol-4-yl)ethanamin in Methanol mit ln Natronlauge. Nach Neutralisieren mit ln Salzsäure wird zur Trockene eingeengt, mit l0 ml Ethanol behandelt und die anorganischen Rückstände abfiltriert. Die Ethanolphase wird mit 60 ml Methylenchlorid verdünnt und erneut abfiltriert. Die Mutterlauge wird zur Trockene eingeengt und der Rückstand mit Ether verrieben und abgesaugt.
Ausbeute: 90 % der Theorie,
Schmelzpunkt: 83-85 °C
Ber.: C 50,88 H 5,l7 N 6,25
Gef.: 50,70 5,44 6,ll
Laut $^1$H-NMR-Spektrum (400 MHz) liegt ein ca. 60:40 Diastereomerengemisch vor.

Beispiel 74

N-[2-(4-Carbomethoxymethoxyphenyl)-l-methylethyl]-2-(2-trifluormethyl-thiazol-4-yl)morpholin (Diastereomer A)

a) N-[2-(4-Carbomethoxymethoxyphenyl)-l-methylethyl]-2-(2-trifluormethyl-thiazol-4-yl)morpholin-5-on (Diastereomer A)

l,2 g (0,0029 Mol) N-[2-(4-Carbomethoxymethoxyphenyl)-l-methyl]-2-hydroxy-2-(2-trifluormethyl-thiazol-4-yl)ethanamin (Diastereomer A) werden in l2 ml Methylenchlorid gelöst, auf l2 °C abgekühlt und 0,4 ml (0,0029 Mol) Triethylamin zugesetzt. Unter Rühren werden 0,22 ml (0,0029 Mol) Chloracetylchlorid zugetropft. Die Temperatur steigt dabei auf 24 °C an. Nach 30 Minuten wird die organische Phase mit Wasser ausgeschüttelt, über Natriumsulfat getrocknet und zur Trockene eingeengt. Das dabei erhaltene Öl wird in l2 ml Dimethylformamid aufgenommen und unter Rühren bei 22-24 °C mit l30 mg/50%iger Natriumhydriddispersion in Öl umgesetzt. Nach einer Stunde werden zur Vervollständigung der Reaktion weitere 90 mg 50%ige Natriumhydriddispersion zugegeben. Nach insgesamt l,5 Stunden wird mit etherischer Salzsäure neutralisiert und l00 ml Methylenchlorid zugegeben. Die organische Phase wird mit Wasser ausgeschüttelt und diese weitere 2 x mit Methylenchlorid extrahiert. Nach dem Trocknen der organischen Phasen über Natriumsulfat wird zur Trockene eingeengt. Das erhaltene Öl wird über eine Kieselgelsäule mit Toluol/Essigester = 6:4 als Eluens gereinigt.
Ausbeute: l,2 g (90 % der Theorie)

b) N-[2-(4-Carbomethoxymethoxyphenyl)-l-methylethyl]-2-(2-trifluormethyl-thiazol-4-yl)morpholin (Diastereomer A)

l,2 g (0,0026 Mol) N-[2-(4-Carbomethoxymethoxyphenyl)-l-methylethyl]-2-(2-trifluormethyl-thiazol-4-yl)-morpholin-5-on (Diastereomer A) werden bei 22 °C in l0 ml absolutem Tetrahydrofuran gelöst. Zu dieser Lösung werden in Abständen von jeweils 30 Minuten dreimal jeweils 2,8 ml (0,0028 Mol) einer l molaren Lösung von Diboran in Tetrahydrofuran zugetropft. Nach l,5 Stunden wird zur Trockene eingeengt. Der erhaltene Rückstand wird in 80 ml Methanol aufgenommen und für l6 Stunden bei Raumtemperatur stehen gelassen. Es wird erneut zur Trockene eingeengt, der erhaltene Rückstand in Methylenchlorid aufgenommen und die organische Phase mit einer kalten, wässrigen Ammoniaklösung extrahiert. Die Methylenchloridphase wird 2 x mit Wasser extrahiert und die organische Phase nach dem Trocknen über Natriumsulfat eingeengt. Der erhaltene Rückstand wird über eine Kieselgelsäule mit Toluol/Essigester = 8:2 als Eluens gereinigt. Dabei erhält man ein farbloses Öl.
Ausbeute: 0,8 g (69,3 % der Theorie),
Ber.: C 54,04 H 5,22 N 6,30
Gef.: 54,20 5,53 6,4l
$^1$H-NMR-Spektrum (400 MHz) ($CDCl_3/CD_3OD$):
$\delta$ = 4,807 ppm (dd, lH)

Beispiel 75

N-[2-(4-Carbomethoxymethoxyphenyl)-l-methylethyl]-2-(2-trifluormethyl-thiazol-4-yl)morpholin (Diastereomer B)

a) N-[2-(4-Carbomethoxymethoxyphenyl)-l-methylethyl]-2-(2-trifluormethyl-thiazol-4-yl)morpholin-5-on (Diastereomer B)

Hergestellt analog Beispiel 74a durch Umsetzung von N-[2-(4-Carbomethoxymethoxyphenyl)-l-methylethyl]-2-hydroxy-2-(2-trifluormethyl-thiazol-4-yl)ethanamin (Diastereomer B) mit Chloracetylchlorid und Natriumhydrid und Reinigung der Base über eine Kieselgelsäule mit Toluol/Essigester = 6:4 als Eluens.
Ausbeute: 7l % der Theorie.

b) N-[2-(4-Carbomethoxymethoxyphenyl)-l-methylethyl]-2-(2-trifluormethyl-thiazol-4-yl)morpholin (Diastereomer B)

Hergestellt analog Beispiel 74b durch Umsetzung von N-[2-(4-Carbomethoxymethoxyphenyl)-l-methylethyl]-2-(2-trifluormethyl-thiazol-4-yl)morpholin-5-on (Diastereomer B) mit Diboran in Tetrahydrofuran sowie anschließender Reinigung der Base über eine Kieselgelsäule mit Tolul/Essigester = 8:2 als Eluens.
Ausbeute: 53 % der Theorie,
Ber.: C 54,04 H 5,22 N 6,30

Gef.: 54,3l 5,35 6,22
$^1$H-NMR-Spektrum (400 MHz) ($CDCl_3/CD_3OD$):
δ = 4,829 ppm (dd, lH)

Beispiel 76

N-[2-(4-Carbomethoxymethoxyphenyl)-l-methylethyl]-N-(carboethoxymethyl)-2-hydroxy-2-(2-trifluormethyl-thiazol-4-yl)ethanamin

0,2l g (0,005 Mol) N-[2-(4-Carbomethoxymethoxyphenyl)-l-methylethyl]-2-hydroxy-2-(2-trifluormethyl-thiazol-4-yl)ethanamin werden in l0 ml Butan-2-on gelöst, mit 0,5 ml Bromessigsäureethylester und 0,5 g Kaliumcarbonat versetzt und l6 Stunden bei Raumtemperatur gerührt. Die anorganischen Produkte werden abfiltriert und das Lösungsmittel abdestilliert. Das zurückbleibende Öl wird über eine Kieselgelsäule mit Toluol/Essigester = 85:l5 als Eluens gereinigt. Man erhält ein farbloses Öl.
Ausbeute: 0,l4 g (56 % der Theorie),
Ber.: C 52,47 H 5,4l N 5,56
Gef.: 52,7l 5,42 5,57
Laut $^1$H-NMR-Spektrum (400 MHz) liegt ein ca. 50:50-Gemisch der Diastereomeren vor.

Beispiel 77

N-[2-(4-Carbomethoxymethoxyphenyl)-l-methylethyl]-N-(carboxymethyl)-2-hydroxy-2-(2-trifluormethyl-thiazol-4-yl)ethanamin

l,4 g (0,003l Mol) N-[2-(4-Carbomethoxymethoxyphenyl)-l-methylethyl]-2-(2-trifluormethyl-thiazol-4-yl)-morpholin-6-on werden in l0 ml Methanol gelöst. Zu dieser Lösung werden unter Rühren bei Raumtemperatur 3 ml (0,003 Mol) ln Natronlauge zugegeben. Nach 5 Minuten werden l5 ml Eiswasser zugegeben und mit 3 ml ln Salzsäure neutralisiert. Die wässrige Phase wird 3 x mit Methylenchlorid ausgeschüttelt. Die organische Phase wird über Natriumsulfat getrocknet, abfiltriert und zur Trockene eingeengt. Der erhaltene Rückstand wird über eine Kieselgelsäule mit Methylenchlorid/Methanol = 20:l als Eluens gereinigt. Dabei erhält man nach dem Einengen farblose Kristalle.
Ausbeute: 0,29 g (2l % der Theorie),
Schmelzpunkt: l28° C
Ber.: C 50,4l H 4,86 N 5,88
Gef.: 50,l9 4,89 5,74
Laut $^1$H-NMR-Spektrum (400 MHz) liegt ein ca. 50:50-Gemisch der Diastereomeren vor.

Beispiel 78

N-[2-(4-(2-(l-Piperidino)ethoxy)phenyl)-l-methylethyl]-2-hydroxy-2-(2-methyl-thiazol-4-yl)ethanamin

Hergestellt analog Beispiel l3 durch Umsetzung von 2-Hydroxy-2-(2-methyl-thiazol-4-yl)ethanamin mit l-(4-(2-(l-Piperidino)ethoxy)phenyl)propan-2-on und anschließender Reinigung über eine Kieselgelsäule mit Methanol als Eluens.
Ausbeute: l3 % der Theorie,
Schmelzpunkt: l28° C
Ber.: C 65,48 H 8,24 N l0,4l S 7,94
Gef.: 65,39 8,l7 l0,29 7,79
$^1$H-NMR-Spektrum ($CDCl_3$):
δ = 4,75 (dd, =CH-OH)
δ = 4,70 (dd, =CH-OH)
Laut $^1$H-NMR-Spektrum (400 MHz) liegt ein ca. l:l-Gemisch der Diastereomeren vor.

Beispiel 79

N-[2-(4-(2-Hydroxyethoxy)phenyl)-l-methylethyl]-2-hydroxy-2-(2-methyl-thiazol-4-yl)ethanamin

Hergestellt analog Beispiel l3 durch Umsetzung von 2-Hydroxy-2-(2-methyl-thiazol-4-yl)ethanamin mit l-

[4-(2-Hydroxyethoxy)phenyl]propan-2-on und anschließender Reinigung über eine Kieselgelsäule mit Methylenchlorid/Methanol = 9:l als Eluens.
Ausbeute: 6l % der Theorie,
Ber.: C 60,69 H 7,l9 N 8,33 S 9,53
Gef.: 60,59 7,l3 8,25 9,47
$^1$H-NMR-Spektrum ($CDCl_3$):
δ = 4,78 (dd, =CH-OH)
δ = 4,83 (dd, =CH-OH)
Laut $^1$H-NMR-Spektrum (400 MHz) liegt ein ca. l:l-Gemisch der Diastereomeren vor.

Beispiel 80

N-[2-(4-Carbomethoxyphenyl)-l-methylethyl]-2-hydroxy-2-(2-dimethylamino-thiazol-4-yl)ethanamin

5 g 2-(N,N-Dimethylamino)-4-bromacetyl-thiazol werden in 250 ml Aceton mit l0 g Kaliumhydrogencarbonat und 9,5 g 2-(4-Carbomethoxyphenyl)-l-methylethylamin-hydrochlorid 3 Stunden zum Rückfluß erhitzt. Nach Abkühlen des Reaktionsgemisches werden die anorganischen Produkte abfiltriert und das Filtrat am Rotationsverdampfer eingeengt. Der erhaltene ölige Rückstand wird in l50 ml absolutem Methanol aufgenommen und bei 0-5 °C mit l,75 g Natriumborhydrid in kleinen Portionen versetzt. Anschließend wird eine Stunde bei 0-5 °C und 24 Stunden bei Raumtemperatur nachgerührt. Danach wird das Reaktionsgemisch mit Eis/Wasser versetzt, mit konzentrierter Salzsäure angesäuert, mit Ammoniak unter Eiskühlung alkalisch gestellt und mit Methylenchlorid extrahiert. Der Extrakt wird über Natriumsulfat getrocknet, eingeengt und über eine Kieselgelsäule mit Essigester/Methanol = 8:2 als Eluens gereinigt.
Ausbeute: 0,5 g (6,8 % der Theorie),
Ber.: C 59,43 H 6,93 N ll,56 S 8,82
Gef.: 59,38 7,07 ll,39 9,04
$^1$H-NMR-Spektrum ($CDCl_3/CD_3OD$):
δ = 6,39 ppm (s, lH)
δ = 6,37 ppm (s, lH)
Laut $^1$H-NMR-Spektrum (400 MHz) liegt ein ca. 2:l-Gemisch der Diastereomeren vor.

Beispiel 8l

N-[2-(Carbomethoxymethoxyphenyl)-l-methylethyl]-2-(2-methyl-thiazol-4-yl)morpholin

Hergestellt analog Beispiel l3 durch Umsetzung von 2-(2-Methyl-thiazol-4-yl)morpholin mit l-(4-Carbomethoxymethoxyphenyl)propan-2-on und anschließender Reinigung über eine Kieselgelsäule mit Toluol/Essigester = 6:4 als Eluens.
Ausbeute: 22 % der Theorie,
Ber.: C 6l,52 H 6,7l N 7,l7 S 8,2l
Gef.: 6l,68 6,89 6,98 8,32
$^1$H-NMR-Spektrum ($CDCl_3/CD_3OD$):
δ = 2,7l ppm (s, lH)
δ = 2,72 ppm (s, lH)
Laut $^1$H-NMR-Spektrum (400 MHz) liegt ein ca. l:l-Gemisch der Diastereomeren vor.

Beispiel 82

N-[2-(4-(2-Hydroxyethoxy)phenyl)-l-methylethyl]-2-hydroxy-2-(2-trifluormethyl-thiazol-4-yl)ethanamin

Hergestellt analog Beispiel l3 durch Umsetzung von 2-Hydroxy-2-(2-trifluormethyl-thiazol-4-yl)ethanamin mit l-(4-(2-Hydroxyethoxy)phenyl)propan-2-on und anschließender Reinigung über eine Kieselgelsäule mit Essigester/Methanol = 9:l als Eluens.
Ausbeute: 27,6 % der Theorie,
Ber.: C 52,30 H 5,42 N 7,l8 S 8,2l
Gef.: 52,l9 5,57 7,l3 8,40
$^1$H-NMR-Spektrum ($CDCl_3/CD_3OD$):
δ = 7,60 ppm (s, lH)

δ = 7,57 ppm (s, IH)
Laut $^1$H-NMR-Spektrum (400 MHz) liegt ein ca. I:I-Gemisch der Diastereomeren vor.

Beispiel 83

N-[2-(4-(2-Methylaminoethoxy)phenyl)-I-methylethyl]-2-hydroxy-2-(2-trifluormethyl-thiazol-4-yl)ethanamin

I,2 ml Borandimethylsulfid-Komplex werden zu einer Lösung von 0,38 g N-[2-(4-Methylaminocarbonylmethoxyphenyl)-I-methylethyl]-2-hydroxy-2-(2-trifluormethyl-thiazol-4-yl)ethanamin in 20 ml absolutem Tetrahydrofuran gegeben und 3 Stunden zum Rückfluß erhitzt. Anschließend werden 4 ml Methanol vorsichtig zum Reaktionsgemisch zugetropft und eine Stunde zum Rückfluß erhitzt. Nach Abkühlung auf Raumtemperatur wird mit etherischer Salzsäure versetzt. Die entstandene Lösung wird eingedampft, der erhaltene Rückstand in I5 ml Wasser aufgenommen, mit konzentriertem Ammoniak alkalisch gestellt und mehrfach mit Methylenchlorid extrahiert. Die vereinigten Extrakte werden mit Natriumsulfat getrocknet, eingedampft und über eine Kieselgelsäule mit Methylenchlorid/Methanol = 8:2 als Eluens gereinigt.
Ausbeute: 0,06 g (I3,6 % der Theorie),
Ber.: C 53,59 H 6,00 N I0,42
Gef.: 53,40 6,I9 I0,20
$^1$H-NMR-Spektrum ($CDCl_3/CD_3OD$):
δ = 7,60 ppm (s, IH)
δ = 7,57 ppm (s, IH)
Laut $^1$H-NMR-Spektrum (400 MHz) liegt ein ca. I:I-Gemisch der Diastereomeren vor.

Beispiel 84

N-[2-(4-Methylaminocarbonylmethoxyphenyl)-I-methylethyl]-2-(2-trifluormethyl-thiazol-4-yl)morpholin

Hergestellt analog Beispiel 32 durch Umsetzung von 2-Trifluormethyl-4-bromacetyl-thiazol mit N-(2-Hydroxyethyl)-2-(4-methylaminocarbonylmethoxyphenyl)-I-methylethylamin und Kaliumhydrogencarbonat in Aceton bei Raumtemperatur und anschließender Reduktion mit Natriumborhydrid in Trifluoressigsäure. Das erhaltene Rohprodukt wird über eine Kieselgelsäule mit Toluol/Essigester = 2:8 als Eluens gereinigt.
Ausbeute: 47,4 % der Theorie,
Schmelzpunkt: 96-98 °C
Ber.: C 54,I7 H 5,46 N 9,48 S 7,23
Gef.: 53,99 5,42 9,38 7,39
$^1$H-NMR-Spektrum ($CDCl_3/CD_3OD$):
δ = 7,6I ppm (s, IH)
δ = 7,59 ppm (s, IH)
Laut $^1$H NMR-Spektrum (400 MHz) liegt ein ca. I:I-Gemisch der Diastereomeren vor.

Beispiel 85

N-[2-(4-(2-Hydroxyethoxy)phenyl)-I-methylethyl]-2-(2-methylthiazol-4-yl)morpholin

Hergestellt analog Beispiel I3 durch Umsetzung von 2-Hydroxy-2-(2-methyl-thiazol-4-yl)morpholin mit I-[4-(2-Hydroxyethoxy)phenyl]propan-2-on und anschließender Reinigung über eine Kieselgelsäule mit Methylenchlorid als Eluens.
Ausbeute: 42 % der Theorie,
Ber.: C 63,I3 H 6,97 N 7,75
Gef.: 63,03 6,95 7,68
$^1$H-NMR-Spektrum ($CDCl_3/CD_3OD$):
δ = 7,I4 ppm (s, IH)
δ = 7,I3 ppm (s, IH)
Laut $^1$H-NMR-Spektrum (400 MHz) liegt ein ca. 2:3-Gemisch der Diastereomeren vor.

Beispiel 86

N-[2-(4-Carboxymethoxyphenyl)-I-methylethyl]-2-(2-methylthiazol-4-yl)morpholin

Hergestellt analog Beispiel l3 durch Umsetzung von 2-(2-Methyl-thiazol-4-yl)morpholin mit l-[4-Carbomethoxyphenyl]propan-2-on und anschließender Reinigung über eine Kieselgelsäule mit Methylenchlorid/Essigester/Methanol/Ammoniak = 4:4:2:l als Eluens.
Ausbeute: 28 % der Theorie,
Ber.: C 60,62 H 6,42 N 7,44 S 8,52
Gef.: 60,58 6,40 7,40 8,50
$^1$H-NMR-Spektrum ($CDCl_3/CD_3OD$):
δ = 7,l3 ppm (s, lH)
δ = 7,l4 ppm (s, lH)
Laut $^1$H-NMR-Spektrum (400 MHz) liegt ein ca. l:l-Gemisch der Diastereomeren vor.

Beispiel 87

N-[2-(4-(6-Hydroxyhexoxy)phenyl)-l-methylethyl]-2-(2-trifluormethyl-thiazol-4-yl)morpholin

Hergestellt analog Beispiel 32 durch Umsetzung von N-(2-Hydroxyethyl)-2-(4-(6-hydroxyhexoxy)phenyl)-l-methylethylamin mit 2-Trifluormethyl-4-bromacetyl-thiazol und Kaliumhydrogencarbonat in Aceton bei Raumtemperatur und anschließender Reduktion mit Natriumborhydrid in Trifluoressigsäure. Das erhaltene Rohprodukt wird über eine Kieselgelsäule mit Toluol/Essigester = 6:4 als Eluens gereinigt.
Ausbeute: l0,8 % der Theorie,
Ber.: C 58,46 H 6,6l N 5,93 S 6,78
Gef.: 58,57 6,49 5,79 6,9l
$^1$H-NMR-Spektrum ($CDCl_3/CD_3OD$):
δ = 7,6l ppm (s, lH)
δ = 7,57 ppm (s, lH)
Laut $^1$H-NMR-Spektrum (400 MHz) liegt ein ca. l:l-Gemisch der Diastereomeren vor.

Beispiel 88

N-[2-(4-Methylaminocarbonylmethoxyphenyl)-l-methylethyl]-2-(2-methyl-thiazol-4-yl)morpholin

Hergestellt analog Beispiel l3 durch Umsetzung von 2-(2-Methyl-thiazol-4-yl)morpholin mit l-(4-Methylaminocarbonylmethoxyphenyl)propan-2-on und anschließender Reinigung über eine Kieselgelsäule mit Essigester als Eluens.
Ausbeute: 22,3 % der Theorie,
Ber.: C 6l,67 H 6,99 N l0,79 S 8,23
Gef.: 6l,70 6,97 l0,67 8,42
$^1$H-NMR-Spektrum ($CDCl_3/CD_3OD$):
δ = 2,72 ppm (s, 3H)
δ = 2,7l ppm (s, 3H)
Laut $^1$H-NMR-Spektrum (400 MHz) liegt ein ca. l:l-Gemisch der Diastereomeren vor.

Beispiel 89

N-[2-(4-Aminocarbonylmethoxyphenyl)-l-methylethyl]-2-(2-trifluormethyl-thiazol-4-yl)morpholin

Hergestellt analog Beispiel 24 durch Umsetzung von N-[2-(4-Carbomethoxymethoxyphenyl)-l-methylethyl]-2-(2-trifluormethylthiazol-4-yl)morpholin mit Ammoniak und anschließender Reinigung über eine Kieselgelsäule mit Essigester/Toluol = 8:2 als Eluens.
Ausbeute: 5l,7 % der Theorie,
Ber.: C 53,l4 H 5,l6 N 9,78 S 7,47
Gef.: 53,26 5,34 9,63 7,59
$^1$H-NMR-Spektrum ($CDCl_3/CD_3OD$):
δ = 7,7l ppm (s, lH)
δ = 7,69 ppm (s, lH)
Laut $^1$H-NMR-Spektrum (400 MHz) liegt ein ca. l:l-Gemisch der Diastereomeren vor.

Beispiel 90

N-[2-(4-(2-Methylaminoethoxy)phenyl)-l-methylethyl]-2-(2-trifluormethyl-thiazol-4-yl)morpholin

Hergestellt analog Beispiel 83 durch Umsetzung von N-[2-(4-Methylaminocarbonylmethoxyphenyl)-l-methylethyl]-2-(2-trifluormethyl-thiazol-4-yl)morpholin mit Borandimethylsulfid-Komplex und anschließender Reinigung über eine Kieselgelsäule mit Essigester/Methanol (8:2) als Eluens.
Ausbeute: 26 % der Theorie,

| | | | |
|---|---|---|---|
| Ber.: | C 55,93 | H 6,I0 | N 9,78 |
| Gef.: | 56,08 | 6,2I | 9,65 |

$^1$H-NMR-Spektrum ($CDCl_3/CD_3OD$):
δ = 7,63 ppm (s, IH)
δ = 7,59 ppm (s, IH)
Laut $^1$H-NMR-Spektrum (400 MHz) liegt ein ca. I:I-Gemisch der Diastereomeren vor.

Beispiel 9I

N-[2-(4-(6-Hydroxyhexoxy)phenyl)-l-methylethyl]-2-(2-trifluormethyl-thiazol-4-yl)ethanamin

Hergestellt analog Beispiel I3 durch Umsetzung von 2-Hydroxy-2-(2-trifluormethyl-thiazol-4-yl)ethanamin mit I-(4-(6-Hydroxyhexoxy)phenyl)propan-2-on und anschließender Reinigung über eine Kieselgelsäule mit Essigester/Methanol (9,5:0,5) als Eluens.
Ausbeute: I7,4 % der Theorie,

| | | | | |
|---|---|---|---|---|
| Ber.: | C 56,49 | H 6,55 | N 6,27 | S 7,I8 |
| Gef.: | 56,32 | 6,47 | 6,34 | 7,28 |

$^1$H-NMR-Spektrum ($CDCl_3/CD_3OD$):
δ = 7,59 ppm (s, IH)
δ = 7,55 ppm (s, IH)
Laut $^1$H-NMR-Spektrum (400 MHz) liegt ein ca. I:I-Gemisch der Diastereomeren vor.

Beispiel 92

N-[2-(4-Carbomethoxymethoxyphenyl)-l-methylethyl]-2-(2-trifluormethyl-5-methyl-thiazol-4-yl)morpholin

Hergestellt analog Beispiel 32 durch Umsetzung von 4,2 g (0,0I46 Mol) 2-Trifluormethyl-5-methyl-4-bromacetyl-thiazol mit 8 g (0,03 Mol) N-(2-Hydroxyethyl)-2-(4-carbomethoxymethoxyphenyl)-l-methylethylamin in Methylenchlorid durch 20-stündiges Rühren bei Raumtemperatur und 2-stündiges Erhitzen unter Rückfluß. Die Reduktion erfolgt in 28 ml Trifluoressigsäure mit 2,4 g (0,02 Mol) Triethylsilan. Das Rohprodukt wird über eine Kieselgelsäule mit Toluol/Essigester = 7:3 als Eluens gereinigt.
Ausbeute: I,7 g (28 % der Theorie),

| | | | | |
|---|---|---|---|---|
| Ber.: | C 55,0I | H 5,49 | N 6,I0 | S 6,99 |
| Gef.: | 55,20 | 5,60 | 6,30 | 7,20 |

$^1$H-NMR-Spektrum ($CDCl_3/CD_3OD$):
δ = 4,775 ppm (t, IH)
δ = 4,8I0 ppm (t, IH)
Laut $^1$H-NMR-Spektrum (400 MHz) liegt ein ca. 3:2-Gemisch der Diastereomeren vor.

Beispiel 93

N-[2-(4-Carbomethoxymethoxyphenyl)-l-methylethyl]-2-(2-acetamino-thiazol-4-yl)morpholin

Hergestellt analog Beispiel 32 durch Umsetzung von 2,23 g (0,0085 Mol) 2-Acetylamino-4-bromacetyl-thiazol mit 2,26 g (0,0085 Mol) N-(2-Hydroxyethyl)-2-(4-carbomethoxymethoxyphenyl)-l-methylethylamin und 0,86 g (0,0085 Mol) Triethylamin in 30 ml Methylenchlorid und 30 ml Methanol innerhalb 20 Stunden bei Raumtemperatur. Die Reduktion erfolgt in 9 ml Trifluoressigsäure mit I,48 g (0,0I28 Mol) Triethylsilan. Das Rohprodukt wird über eine Kieselgelsäule mit Essigester/Methanol = 9:I als Eluens gereinigt.
Ausbeute: I g (27 % der Theorie),
Schmelzpunkt: 65-70 °C

| | | | | |
|---|---|---|---|---|
| Ber.: | C 58,I8 | H 6,27 | N 9,69 | S 7,33 |
| Gef.: | 57,90 | 6,40 | 9,49 | 7,48 |

$^1$H-NMR-Spektrum ($CDCl_3/CD_3OD$):
$\delta$ = 6,875 ppm (d, IH)
$\delta$ = 6,850 ppm (d, IH)
Laut $^1$H-NMR-Spektrum (400 MHz) liegt ein ca. 2:I-Gemisch der Diastereomeren vor.

Beispiel 94

N-[2-(4-Carbomethoxymethoxyphenyl)-I-methylethyl]-2-hydroxy-2-(2-methyl-oxazol-4-yl)ethanamin-dihydrochlorid

Hergestellt analog Beispiel I3 durch Umsetzung von 0,7 g (0,005 Mol) 2-Hydroxy-2-(2-methyl-oxazol-4-yl)ethanamin und I,I g (0,005 Mol) I-(4-Carbomethoxymethoxyphenyl)propan-2-on in 40 ml absolutem Methanol mit 0,3 g (0,005 Mol) Essigsäure und 0,32 g (0,005 Mol) Natriumcyanborhydrid. Das Rohprodukt wird über eine Kieselgelsäule mit Essigester/Methanol = 9:I als Eluens gereinigt und durch Fällung mit etherischer Salzsäure das Dihydrochlorid hergestellt.
Ausbeute: 0,6 g (28 % der Theorie),
Schmelzpunkt: I60 °C sintern, ab I68 °C Zers.

| | | | | |
|---|---|---|---|---|
| Ber.: | C 5I,30 | H 6,2I | N 6,64 | S I6,84 |
| Gef.: | 5I,50 | 6,I0 | 6,76 | I6,57 |

$^1$H-NMR-Spektrum ($CDCl_3/CD_3OD$):
$\delta$ = 7,49 ppm (d, IH)
$\delta$ = 7,5I ppm (d, IH)
Laut $^1$H-NMR-Spektrum (400 MHz) liegt ein ca. 50:50-Gemisch der Diastereomeren vor.

Beispiel 95

N-[2-(4-Carbomethoxymethoxyphenyl)-I-methylethyl]-2-(2-chlorthiazol-4-yl)morpholin

Hergestellt analog Beispiel 32 durch Umsetzung von 5 g (0,0208 Mol) 2-Chlor-4-bromacetyl-thiazol mit 5,6 g (0,02I Mol) N-(2-Hydroxyethyl)-2-(4-carbomethoxymethoxyphenyl)-I-methylethylamin in 200 ml Aceton und 6,3 g (0,063 Mol) Kaliumhydrogencarbonat innerhalb 20 Stunden bei Raumtemperatur. Die Reduktion erfolgte in 4I ml Trifluoressigsäure mit 3,5 g (0,029 Mol) Triethylsilan über 24 Stunden. Das Rohprodukt wird über eine Kieselgelsäule mit Methylenchlorid/Methanol = 20:I als Eluens gereinigt.
Ausbeute: I,2 g (I6 % der Theorie),

| | | | |
|---|---|---|---|
| Ber.: | C 55,53 | H 5,64 | N 6,8I |
| Gef.: | 55,4I | 5,70 | 6,57 |

$^1$H-NMR-Spektrum ($CDCl_3/CD_3OD$):
$\delta$ = 7,2I ppm (d, IH)
$\delta$ = 7,22 ppm (d, IH)
Laut $^1$H-NMR-Spektrum (400 MHz) liegt ein ca. 2:3-Gemisch der Diastereomeren vor.

Beispiel 96

N-[2-(4-Carboxymethoxyphenyl)-I-methylethyl]-2-(2-amino-thiazol-4-yl)morpholin-dihydrochlorid

I,6 g (0,0037 Mol) N-[2-(4-Carbomethoxymethoxyphenyl)-I-methylethyl]-2-(2-acetamino-thiazol-4-yl)-morpholin werden in I00 ml I8%iger Salzsäure über 48 Stunden unter Stickstoff am Rückfluß gekocht. Die Reaktionslösung wird mit I,5 g Aktivkohle versetzt, gut verrührt und abfiltriert. Dann wird die Lösung eingeengt und das Produkt im Vakuum über Kaliumhydroxid getrocknet.
Ausbeute: I,66 g (I00 % der Theorie),

| | | | | | |
|---|---|---|---|---|---|
| Ber.: | C 47,99 | H 9,32 | N 8,32 | S 7,II | Cl I5,76 |
| Gef.: | 48,20 | 9,45 | 8,99 | 7,I8 | I5,78 |

Laut $^1$H-NMR-Spektrum (400 MHz) liegt ein 2:I-Gemisch der Diastereomeren vor.

Beispiel 97

N-[2-(4-Carboxymethoxyphenyl)-I-methylethyl]-2-(2-chlorthiazol-4-yl)morpholin

0,55 g (0,0013 Mol) N-[2-(4-Carbomethoxymethoxyphenyl)-1-methylethyl]-2-(2-chlor-thiazol-4-yl)morpholin werden bei Raumtemperatur für 10 Minuten in 4 ml Methanol und 4 ml 1n Natronlauge gerührt. Dann wird mit 4 ml 1n Salzsäure neutralisiert und das Produkt durch Extraktion mit Methylenchlorid gewonnen.
Ausbeute: 0,52 g (100 % der Theorie),
Schmelzpunkt: 80-90 °C (Zers.)
Ber.: C 54,47 H 5,33 N 7,05 Cl 8,93
Gef.: 54,40 5,42 7,00 8,90
Laut $^1$H-NMR-Spektrum (400 MHz) liegt ein 2:3-Gemisch der Diastereomeren vor.

Beispiel 98

N-[2-(4-(2-Hydroxyethoxy)phenyl)-1-methylethyl]-2-(2-chlorthiazol-4-yl)morpholin

Hergestellt analog Beispiel 71 durch Umsetzung von N-[2-(4-Carbomethoxymethoxyphenyl)-1-methylethyl]-2-(2-chlor-thiazol-4-yl)morpholin mit Boran-Tetrahydrofuran-Komplex (1 molare Lösung in Tetrahydrofuran) über 60 Stunden.
Ausbeute: 35 % der Theorie
$^1$H-NMR-Spektrum ($CDCl_3/CD_3OD$):
$\delta$ = 7,209 ppm (d, 1H)
$\delta$ = 7,228 ppm (d, 1H)
Laut $^1$H-NMR-Spektrum (400 MHz) liegt ein 2:3-Gemisch der Diastereomeren vor.

Beispiel 99

N-[2-(4-(2-Hydroxyethoxy)phenyl)-1-methylethyl]-2-(2-hydroxy-2-(2-chlor-thiazol-4-yl)ethanamin

Hergestellt analog Beispiel 71 durch Umsetzung von N-[2-(4-Carboxymethoxyphenyl)-1-methylethyl]-2-hydroxy-2-(2-chlorthiazol-4-yl)ethanamin mit Boran-Tetrahydrofuran-Komplex (1 molare Lösung in Tetrahydrofuran) über 24 Stunden.
Ausbeute: 20 % der Theorie
$^1$H-NMR-Spektrum ($CDCl_3/CD_3OD$):
$\delta$ = 7,195 ppm (d, 1H)
$\delta$ = 7,22 ppm (d, 1H)
Laut $^1$H-NMR-Spektrum (400 MHz) liegt ein ca. 1:1-Gemisch der Diastereomeren vor.

Beispiel 100

N-[2-(4-Carboxymethoxyphenyl)-1-methylethyl]-2-hydroxy-2-(2-chlor-thiazol-4-yl)ethanamin

Hergestellt analog Beispiel 97 durch Umsetzung von N-[2-(4-Carbomethoxymethoxyphenyl)-1-methylethyl]-2-hydroxy-2-(2-chlor-thiazol-4-yl)ethanamin mit 1n-Natronlauge in Methanol.
Ausbeute: 0,19 g (100 % der Theorie)
Schmelzpunkt: 58-63 °C
Ber.: C 51,82 H 5,16 N 7,55
Gef.: 51,75 5,22 7,58

Beispiel 101

N-[2-(4-(2-Hydroxyethoxy)phenyl)-1-methylethyl]-N-(2-hydroxyethyl)-2-hydroxy-2-(2-trifluormethyl-thiazol-4-yl)ethanamin

Hergestellt analog Beispiel 71 durch Umsetzung von N-[2-(4-Carboxymethoxyphenyl)-1-methylethyl]-N-(2-hydroxyethyl)-2-hydroxy-2-(2-trifluormethyl-thiazol-4-yl)ethanamin in Tetrahydrofuran mit Diboran in Tetrahydrofuran und Reinigung der Base über eine Kieselgelsäule mit Essigester/Methanol = 20:1 als Eluens.
Ausbeute: 54 % der Theorie, Öl,
Ber.: C 52,52 H 5,80 N 6,45
Gef.: 52,19 5,72 6,39
$^1$H-NMR-Spektrum (400 MHz) ($CDCl_3/CD_3OD$):

δ = 7,828 ppm (s, IH)
δ = 7,839 ppm (s, IH)
Es liegt ein 60:40-Gemisch der Diastereomeren vor.

Beispiel I02

N-[2-(4-Carbomethoxymethoxyphenyl)-I-methylethyl]-2-(2-piperidino-thiazol-4-yl)morpholin

Hergestellt analog Beispieol 32 durch Umsetzung von 2,9 g (0,0I Mol) 2-Piperidino-4-bromacetyl-thiazol mit 3,2 g (0,0I2 Mol) N-(2-Hydroxyethyl)-2-(4-carbomethoxyphenyl)-I-methylethylamin in 200 ml Aceton in Gegenwart von 3 g (0,03 Mol) Kaliumhydrogencarbonat und anschließender Reduktion in 60 ml Trifluoressigsäure mit 2,9 g (0,076 Mol) Natriumborhydrid über 24 Stunden. Die Substanz wird über eine Kieselgelsäule mit Essigester als Eluens gereinigt, wobei ein gelbes Öl erhalten wird.
Ausbeute: 0,8 g (I7 % der Theorie),

| | | | | |
|---|---|---|---|---|
| Ber.: | C 62,7I | H 7,23 | N 9,I4 | S 6,97 |
| Gef.: | 62,50 | 7,33 | 9,40 | 6,92 |

Laut $^1$H-NMR-Spektrum (400 MHz) liegt ein ca. 33:66-Gemisch der Diastereomeren vor:
δ = 6,33 ppm (d, IH)
δ = 6,44 ppm (d, IH)

Beispiel I03

N-[2-(4-Carbomethoxymethoxyphenyl)-I-methylethyl]-2-hydroxy-2-(2-piperidino-thiazol-4-yl)ethanamin-hydrochlorid

Hergestellt analog Beispiel 3 durch Umsetzung von 2-Piperidino-4-bromacetyl-thiazol mit 2-(4-Carbomethoxymethoxyphenyl)-I-methylethylamin und anschließender Reduktion. Die Verbindung wird über eine Kieselgelsäule mit Essigester/Methanol = 9:I als Eluens gereinigt und die Base mit etherischer Salzsäure in das Hydrochlorid überführt.
Ausbeute: I3 % der Theorie,
Schmelzpunkt: ab I00 °C Zers.

| | | | | | |
|---|---|---|---|---|---|
| Ber.: | C 52,I6 | H 6,56 | N 8,29 | S 6,32 | Cl I4,0I |
| Gef.: | 5I,80 | 6,83 | 8,I7 | 6,48 | I3,72 |

Laut $^1$H-NMR-Spektrum (400 MHz) liegt ein ca. 50:50-Gemisch der Diastereomeren vor.

Beispiel I04

N-[2-(4-Carbomethoxyphenyl)-I-methylethyl]-2-hydroxy-2-(2-trifluormethyl-thiazol-4-yl)ethanamin

0,54 g (0,030 Mol) 2-(2-Trifluormethyl-thiazol-4-yl)ethylenoxid werden in 5 ml Ethanol gelöst und zu einer siedenden Lösung von 0,58 g (0,0030 Mol) 2-(4-Carbomethoxyphenyl)-I-methylethylamin in I3 ml Ethanol innerhalb von I0 Minuten zugetropft. Anschließend wird für 5 Stunden am Rückfluß gekocht, das Lösungsmittel abdestilliert und die Base über eine Kieselgelsäule mit Methylenchlorid/Methanol = 20:I als Eluens gereinigt.
Ausbeute: 0,45 g (38 % der Theorie),

| | | | |
|---|---|---|---|
| Ber.: | C 52,57 | H 4,93 | N 7,2I |
| Gef.: | 52,34 | 5,II | 7,II |

$^1$H-NMR-Spektrum (400 MHz) ($CDCl_3/CD_3OD$):
δ = 7,58 ppm (s, IH)
δ = 7,6I ppm (s, IH)
Es liegt ein ca. 50:50-Gemisch der Diastereomeren vor.

Beispiel I05

N-[2-(4-Carbomethoxymethoxyphenyl)-I-methylethyl]-2-hydroxy-2-(2-trifluormethyl-thiazol-4-yl)ethanamin

Hergestellt analog Beispiel I04 durch Umsetzung von 2-(2-Trifluormethyl-thiazol-4-yl)ethylenoxid mit 2-(4-Carbomethoxymethoxyphenyl)-l-methylethylamin und Dimethylsulfoxid bei 90 °C für l6 Stunden. Die Base wird durch Ausschütteln mit Ether und Reinigen über eine Kieselgelsäule mit Methylenchlorid/Methanol = 20:l als Eluens gereinigt.
Ausbeute: 24 % der Theorie,
Ber.: C 5l,66 H 5,06 N 6,70
Gef.: 5l,50 4,99 6,7l
$^1$H-NMR-Spektrum (400 MHz) ($CDCl_3$/$CD_3OD$):
δ = 7,57 ppm (d, lH)
δ = 7,6l ppm (d, lH)
Es liegt ein ca. 50:50-Gemisch der Diastereomeren vor.

Beispiel l06

N-[2-(4-(2-Hydroxyethoxy)phenyl)-l-methylethyl]-N-(2-hydroxyethyl)-2-hydroxy-2-(2-chlor-thiazol-4-yl)-ethanamin

Hergestellt analog Beispiel 30 durch Umsetzung von 2-Chlor-4-bromacetylthiazol mit N-(2-Hydroxyethyl)-2-(4-carbomethoxymethoxyphenyl)-l-methylethylamin und anschließender Reduktion in Methanol mit Natriumborhydrid bei Raumtemperatur. Das Rohprodukt wird über eine Kieselgelsäule mit Chloroform/Essigester/Methanol = l0:9:l als Eluens gereinigt.
Ausbeute: l5 % der Theorie,
Ber.: C 53,92 H 6,29 N 6,99 S 8,00 Cl 8,84
Gef.: 53,68 6,30 6,57 7,6l 8,72
$^1$H-NMR-Spektrum (400 MHz) ($CDCl_3$/$CD_3OD$):
δ = 7,22 ppm (d, lH)
δ = 7,ll ppm (d, lH)
Laut $^1$H-NMR-Spektrum (400 MHz) liegt ein ca. l:l-Gemisch der Diastereomeren vor.

Beispiel l07

N-[2-(4-Methoxycarbonylmethoxyphenyl)-l-methylethyl]-(2-methoxy-2-(2-methyl-thiazol-4-yl)ethanamin-dihydrochlorid × l,5 $H_2O$

Hergestellt analog Beispiel l3 durch Umsetzung von 2-Methoxy-2-(2-methyl-thiazol-4-yl)ethanamin mit l-(4-Methoxycarbonylmethoxyphenyl)propan-2-on und anschließender Reinigung der Base durch Säulenchromatographie an Kieselgel mit Chloroform/Methanol = l0/l als Eluens.
Ausbeute: 58 % der Theorie,
$^1$H-NMR-Spektrum (400 MHz) ($CDCl_3$)
δ = 4,40 ppm (dd, =CH-OMe)
δ = 4,44 ppm (dd, =CH-OMe)
Laut $^1$H-NMR-Spektrum liegt ein ca. 50:50-Gemisch der Diastereomeren vor.
Die ölige Base wird mittels Chlorwasserstoff/Methanol in ein schaumartiges Dihydrochlorid übergeführt.
Schmelzpunkt: 60-80 °C,
Ber.: (× l,5 $H_2O$) C 47,69 H 6,53 N 5,86 S 6,70 Cl l4,82
Gef.: 47,77 6,68 6,0l 7,07 l4,98

Beispiel l08

N-[2-(4-Carboxymethoxyphenyl)-l-methylethyl]-2-methoxy-2-(2-methyl-thiazol-4-yl)ethanamin × 0,5 $H_2O$

Hergestellt analog Beispiel l6 durch Umsetzung von N-[2-(4-Methoxycarbonylmethoxyphenyl)-l-methylethyl]-2-methoxy-2-(2-methyl-thiazol-4-yl)ethanamin in Methanol mit lN-Natronlauge. Nach Neutralisieren mit lN-Salzsäure wird im Vakuum eingedampft und dann zwischen Chloroform und Wasser verteilt. Der Chloroform-Extrakt wird im Vakuum eingedampft. Den schaumartigen Eindampfrückstand trocknet man bei 50 °C/0,0l Torr.
Ausbeute: 47 % der Theorie,
Schmelzpunkt: 80-90 °C
Ber.: (× 0,50 H20) C 57,88 H 6,73 N 7,50 S 8,59

Gef.: C 57,73 6,75 7,68 8,6l

$^1$H-NMR-Spektrum (400 MHz) ($CDCl_3/CD_3OD$):

δ = l,03 ppm (dd, =CH-Me)

δ = l,08 ppm (dd, =CH-Me)

Es liegt ein ca. 50:50-Gemisch der Diastereomeren vor.

Beispiel l09

N-[2-(4-Carbomethoxymethoxyphenyl)-l-methylethyl]-N-(2-hydroxyethyl)-2-hydroxy-2-(2-chlor-thiazol-4-yl)-ethanamin

Hergestellt analog Beispiel l06 durch Umsetzung von 2-Chlor-4-bromacetylthiazolmit N-(2-Hydroxyethyl)-2-(4-carbomethoxymethoxyphenyl)-l-methylethylamin und anschließender Reduktion bei 0° C. Das Rohprodukt wird über eine Kieselgelsäule mit Chloroform/Essigester = 3:l7 als Eluens gereinigt.

Ausbeute: l2 % der Theorie, Öl,

Ber.: C 53,20 H 5,87 N 6,53 S 7,48 Cl 8,27

Gef.: 52,96 5,83 6,26 7,65 8,37

$^1$H NMR-Spektrum (400 MHz) ($CDCl_3/CD_3OD$):

δ = 7,22 ppm (d, lH)

δ = 7,l3 ppm (d, lH)

Laut $^1$H-NMR-Spektrum liegt ein ca. 50:50-Gemisch der Diastereomeren vor.

Beispiel ll0

N-[2-(4-Carbomethoxyphenyl)-l-methylethyl]-2-hydroxy-2-(2-trifluormethyl-thiazol-4-yl)ethanamin

l g (0,0036 Mol) l-(2-Trifluormethyl-thiazol-4-yl)-l-hydroxy-2-bromethan werden in 20 ml Ethanol gelöst, mit l5 g 2-(4-Carbomethoxyphenyl)-l-methylethylamin versetzt und 2 Stunden zum Rückfluß erhitzt. Das Lösungsmittel wird abdestilliert, der verbleibende Rückstand in Methylenchlorid aufgenommen und mit 2n Natronlauge ausgeschüttelt. Die organische Phase wird getrocknet, eingeengt und das Rohprodukt über eine Kieselgelsäule mit Methylenchlorid/Methanol = 20:l als Eluens gereinigt.

Ausbeute: 0,9 g (65 % der Theorie),

Ber.: C 52,57 H 4,93 N 7,2l

Gef.: 52,27 5,04 7,l7

$^1$H-NMR-Spektrum (400 MHz) ($CDCl_3/CD_3OD$):

δ = 7,58 ppm (s, lH)

δ = 7,6l ppm (s, lH)

Laut $^1$H-NMR-Spektrum liegt ein ca. 50:50-Gemisch der Diastereomeren vor.

Beispiel lll

N-[2-(4-(2-Ethoxyethoxy)phenyl)-l-methylethyl]-2-hydroxy-2-(2-trifluormethyl-thiazol-4-yl)ethanamin

Hergestellt analog Beispiel l3 durch Umsetzung von 2-Hydroxy-2-(2-trifluormethyl-thiazol-4-yl)ethanamin mit l-(4-(2-Ethoxyethoxy)-phenyl)propan-2-on und anschließender Reinigung des Rohproduktes über eine Kieselgelsäule mit Methylenchlorid/Methanol = 95:5 als Eluens.

Ausbeute: 34 % der Theorie, Öl,

Ber.: C 54,53 H 6,02 N 6,69 S 7,66

Gef.: 54,45 6,l2 6,7l 7,63

$^1$H-NMR-Spektrum (400 MHz) ($CDCl_3/CD_3OD$)

δ = 7,60 ppm (s, lH)

δ = 7,57 ppm (s, lH)

Laut $^1$H-NMR-Spektrum liegt ein ca. 50:50-Gemisch der Diastereomeren vor.

Beispiel ll2

N-[2-(4-(2-Ethoxyethoxy)phenyl)-l-methylethyl]-2-(2-trifluormethyl-thiazol-4-yl)morpholin

Hergestellt analog Beispiel 87 durch Umsetzung von N-(2-Hydroxyethyl)-2-(4-(2-ethoxyethoxy)phenyl)-l-methylethylamin mit 2-Trifluormethyl-4-bromacetyl-thiazol in Gegenwart von Kaliumhydrogencarbonat, anschließender Reduktion und Reinigung des Rohproduktes über eine Kieselgelsäule mit Toluol/Essigester = 8:2 als Eluens.
Ausbeute: 35 % der Theorie, Öl,
Ber.: C 56,74 H 6,l2 N 6,30 S 7,2l
Gef.: 56,65 6,2l 6,l5 7,30
$^1$H-NMR-Spektrum (400 MHz) ($CDCl_3/CD_3OD$):
δ = 7,60 ppm (s, lH)
δ = 7,57 ppm (s, lH)
Laut $^1$H-NMR-Spektrum liegt ein ca. 50:50-Gemisch der Diastereomeren vor.

Beispiel ll3

N-[2-(4-(2-Ethoxyethoxy)phenyl)-l-methylethyl]-N-(2-hydroxyethyl)-2-hydroxy-2-(2-trifluormethyl-thiazol-4-yl)-ethanamin

Hergestellt analog Beispiel l06 durch Umsetzung von 2-Trifluormethyl-4-bromacetyl-thiazol mit N-(2-Hydroxyethyl)-2-(4-(2-ethoxyethoxy)phenyl)-l-methylethylamin in Gegenwart von Kaliumhydrogencarbonat, anschließender Reduktion mit Natriumborhydrid in Eisessig und Reinigung des Rohproduktes über eine Kieselgelsäule mit Toluol/Essigester = 6:4 als Eluens.
Ausbeute: 37 % der Theorie, Öl,
Ber.: C 54,53 H 6,32 N 6,06 S 6,93
Gef.: 54,4l 6,76 5,95 7,l5
$^1$H-NMR-Spektrum (400 MHz) ($CDCl_3/CD_3OD$):
δ = 7,60 ppm (s, lH)
δ = 7,49 ppm (s, lH)
Laut $^1$H-NMR-Spektrum liegt ein ca. 3:2-Gemisch der Diastereomeren vor.

Beispiel ll4

N-[2-(4-(2-Phenethoxyethoxy)phenyl)-l-methylethyl]-2-hydroxy-2-(2-trifluormethyl-thiazol-4-yl)ethanamin

Hergestellt analog Beispiel l3 durch Umsetzung von 2-Hydroxy-2-(2-trifluormethyl-thiazol-4-yl)ethanamin und l-(4-(2-Phenethoxy)phenyl)propan-2-on und anschließender Reinigung der Base über eine Kieselgelsäule mit Essigester/Methanol = 8:2 als Eluens.
Ausbeute: 43 % der Theorie, Öl,
Ber.: C 60,7l H 5,9l N 5,66 S 6,48
Gef.: 60,55 6,03 5,7l 6,80
$^1$H-NMR-Spektrum (400 MHz) ($CDCl_3/CD_3OD$):
δ = 7,62 ppm (s, lH)
δ = 7,55 ppm (s, lH)
Laut $^1$H-NMR-Spektrum liegt ein ca. 50:50-Gemisch der Diastereomeren vor.

Beispiel ll5

N-[2-(4-(2-Phenethoxyethoxy)phenyl)-l-methylethyl]-2-(2-trifluor-thiazol-4-yl)morpholin

Hergestellt analog Beispiel 87 durch Umsetzung von N-(2-Hydroxyethyl)-2-(4-(2-phenethoxyethoxy)-phenyl)-l-methylethylamin mit 2-Trifluormethyl-4-bromacetyl-thiazol in Gegenwart von Kaliumhydrogencarbonat, anschließender Reduktion und Reinigung des Rohproduktes über eine Kieselgelsäule mit Methylenchlorid/Methanol = als Eluens.
Ausbeute % der Theorie, Öl,
Ber.: C 62,29 H 6,00 N 5,38 S 6,l6
Gef.:
$^1$H-NMR-Spektrum (400 MHz) ($CDCl_3$/ ):
δ = ppm ( )
δ - ppm ( )

Laut $^1$H-NMR-Spektrum liegt ein ca. 50:50-Gemisch der Diastereomeren vor.

Beispiel II6

N-[2-(4-(2-Phenethoxyethoxy)phenyl)-l-methylethyl]-N-(2-hydroxyethyl)-2-hydroxy-2-(2-trifluormethyl-thiazol-4-yl)ethanamin

Hergestellt analog Beispiel I06 durch Umsetzung von 2-Trifluormethyl-4-bromacetyl-thiazol mit N-(2-Hydroxyethyl)-2-(4-(2-phenethoxyethoxy)phenyl)-l-methylethylamin in Gegenwart von Kaliumhydrogencarbonat, anschließender Reduktion und Reinigung des Rohproduktes über eine Kieselgelsäule mit Methylenchlorid/Methanol = : als Eluens.
Ausbeute: % der Theorie, Öl,
Ber.: C 60,2l H 6,l8 N 5,20 S 5,95
Gef.:
$^1$H-NMR-Spektrum (400 MHz) ($CDCl_3$/ ):
δ = ppm ( )
δ = ppm ( )
Laut $^1$H-NMR-Spektrum liegt ein ca. 50:50-Gemisch der Diastereomeren vor.

Beispiel II7

N-[2-(4-(3-Hydroxypropoxy)phenyl)-l-methylethyl]-2-hydroxy-2-(2-trifluormethyl-thiazol-4-yl)ethanamin

Hergestellt analog Beispiel I3 durch Umsetzung von 2-Hydroxy-2-(2-trifluormethyl-thiazol-4-yl)ethanamin und l-(4-(3-Hydroxypropoxy)phenyl)propan-2-on und anschließender Reinigung der Base über eine Kieselgelsäule mit Essigester/Methanol = 9:l als Eluens.
Ausbeute: 33 % der Theorie, Öl,
Ber.: C 53,46 H 5,73 N 6,93
Gef.: 53,34 5,88 6,78
$^1$H-NMR-Spektrum (400 MHz) ($CDCl_3CD_3OD$):
δ = 7,57 ppm (s, lH)
δ = 7,60 ppm (s, lH)
Laut $^1$H-NMR-Spektrum liegt ein ca. 50:50-Gemisch der Diastereomeren vor.

Beispiel I

Dragee mit 10 mg N-[2-(4-Carbomethoxymethoxyphenyl)-l-methylethyl]-2-hydroxy-2-(2-trifluormethyl-thiazol-4-yl)-ethanamin

Zusammensetzung:

1 Dragee enthält:

| | |
|---|---|
| (1) Wirksubstanz | 10,0 mg |
| (2) Milchzucker | 69,0 mg |
| (3) Maisstärke | 35,0 mg |
| (4) Polyvinylpyrrolidon | 5,0 mg |
| (5) Magnesiumstearat | 1,0 mg |
| | 120,0 mg |

Herstellung:

(1) + (2) + (3) werden gemischt und mit (4) in einer wäßrigen Lösung befeuchtet. Die feuchte Masse

wird durch ein Sieb mit 1,6 mm Maschenweite geschlagen und bei 45 °C im Umlufttrockenschrank getrocknet. Das trockene Granulat wird durch ein Sieb mit 1 mm Maschenweite gegeben und mit (5) gemischt. Die fertige Mischung wird zu Drageekernen verpreßt.

Kerngewicht: 120,0 mg
Durchmesser: 7,0 mm
Wölbungsradius: 6,0 mm

Die so hergestellten Drageekerne werden auf bekannte Weise mit einer Schicht überzogen, die im Wesentlichen aus Zucker und Talkum besteht. Diese Schicht kann auch Farbauszüge enthalten. Die fertigen Dragees werden mit Wachs poliert.

Drageegewicht: 180,0 mg

Beispiel II

Dragee mit 50 mg N-[2-(4-Carbomethoxymethoxyphenyl)-l-methylethyl]-2-hydroxy-2-(2-trifluormethyl-thiazol-4-yl)ethanamin

Zusammensetzung:

1 Dragee enthält:

| | | |
|---|---|---|
| (1) | Wirksubstanz | 50,0 mg |
| (2) | Milchzucker | 110,8 mg |
| (3) | Maisstärke | 50,0 mg |
| (4) | Polyvinylpyrrolidon | 8,0 mg |
| (5) | Magnesiumstearat | 1,2 mg |
| | | 220,0 mg |

Herstellung:

Die Herstellung erfolgt analog Beispiel I. Kerngewicht: 220,0 mg
Durchmesser: 9,0 mm
Wölbungsradius: 8,0 mm
Drageegewicht: 300,0 mg

Beispiel III

Tabletten mit l50 mg N-[2-(4-Carbomethoxymethoxyphenyl)-l-methylethyl]-2-hydroxy-2-(2-trifluormethyl-thiazol-4-yl)ethanamin

Zusammensetzung:

1 Tablette enthält:

| | | |
|---|---|---|
| (1) | Wirksubstanz | 150,0 mg |
| (2) | Milchzucker | 86,0 mg |
| (3) | Maisstärke | 50,8 mg |
| (4) | Mikrokristalline Zellulose | 25,0 mg |
| (5) | Polyvinylpyrrolidon | 7,0 mg |
| (6) | Magnesiumstearat | 1,2 mg |
| | | 320,0 mg |

Herstellung:

(I) + (2) + (3) + (4) + (5) werden gemischt und mit Wasser befeuchtet. Die feuchte Masse wird durch ein Sieb mit l,6 mm Maschenweite geschlagen und bei 45° C getrocknet. Das trockene Granulat wird nochmals durch dasselbe Sieb gegeben und mit (6) gemischt. Aus der fertigen Mischung werden Tabletten gepreßt.

Tablettengewicht: 320,0 mg

Durchmesser: l0,0 mm

Die Tabletten werden mit einer Teilkerbe versehen, um die Halbierung zu ermöglichen.

Beispiel IV

Hartgelatinekapseln zu l00 mg N-[2-(4-Carbomethoxymethoxyphenyl)-l-methylethyl]-2-hydroxy-2-(2-trifluormethyl-thiazol-4-yl)ethanamin

Zusammensetzung:

1 Kapsel enthält:

Kapselhülle: Hartgelatinekapseln Größe 3

Kapselinhaltsstoffe:

| | | |
|---|---|---|
| (1) | Wirksubstanz | 100,0 mg |
| (2) | Lactose x $1H_2O$ | 38,0 mg |
| (3) | Maisstärke getrocknet | 60,0 mg |
| (4) | Magnesiumstearat | 2,0 mg |
| | Kapselfüllgewicht: | 200,0 mg |
| (5) | Dest. Wasser | q.s. |

Herstellung:

Ein kleiner Teil Lactose wird ca. l0 %ig in dest. Wasser gelöst (Granulierflüssigkeit). Der Wirkstoff, die restliche Lactose sowie Maisstärke werden gemischt und mit der Granulierflüssigkeit durchfeuchtet. Die Masse wird gesiebt, getrocknet und nach nochmaligem Sieben mit Magnesiumstearat homogen gemischt. Das feinkörnige Granulat wird auf einer geeigneten Maschine in Kapseln abgefüllt.

Beispiel V

Hartgelatinekapseln zu 200 mg N-[2-(4-Carbomethoxymethoxyphenyl)-l-methylethyl]-2-hydroxy-2-(2-trifluormethyl-thiazol-4-yl)ethanamin

Zusammensetzung:

Kapselhülle: Hartgelatinekapseln Größe 1

Kapselinhaltsstoffe:

| | |
|---|---|
| (1) Wirksubstanz | 200,0 mg |
| (2) Lactose x $1H_2O$ | 47,0 mg |
| (3) Maisstärke getrocknet | 70,0 mg |
| (4) Magnesiumstearat | 3,0 mg |
| Kapselfüllgewicht: | 320,0 mg |
| (5) Dest. Wasser | q.s. |

Herstellung:

Ein kleiner Teil Lactose wird ca. l0 %ig in dest. Wasser gelöst (Granulierflüssigkeit). Der Wirkstoff, die restliche Lactose sowie Maisstärke werden gemischt und mit der Granulierflüssigkeit durchfeuchtet. Die Masse wird gesiebt, getrocknet und nach nochmaligem Sieben mit Magnesiumstearat homogen gemischt. Das feinkörnige Granulat wird auf einer geeigneten Maschine in Kapseln abgefüllt.

**Patentansprüche**

1. Neue substituierte Thiazole und Oxazole der allgemeinen Formel

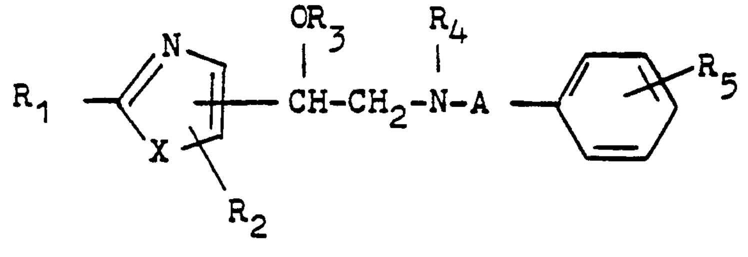

,(I)

in der

A eine gegebenenfalls durch Methyl- oder Ethylgruppen mono-oder disubstituierte n-Alkylengruppe mit 2 oder 3 Kohlenstoffatomen,

X ein Sauerstoff- oder Schwefelatom,

$R_1$ ein Wasserstoff- oder Halogenatom, eine Trifluormethyl-, Alkyl-, Phenyl- oder Piperidinogruppe oder eine gegebenenfalls durch ein oder zwei Alkylgruppen oder eine Alkanoyl- oder Benzoylgruppe substituierte Aminogruppe,

$R_2$ ein Wasserstoffatom oder eine Alkylgruppe,

$R_3$ ein Wasserstoffatom oder eine Alkylgruppe, die in 2-oder 3-Stellung durch eine Hydroxygruppe substituiert sein kann, oder $R_3$ zusammen mit $R_4$ eine Alkoxycarbonylmethylengruppe oder eine Ethylengruppe, wobei die zum N- oder O-Atom benachbarte Methylengruppe durch eine Carbonylgruppe ersetzt sein kann,

$R_4$ ein Wasserstoffatom, eine gegebenenfalls durch eine Phenyl-, Carboxy-, Alkoxycarbonyl- oder Cyanogruppe oder in 2- oder 3-Stellung durch eine Hydroxygruppe substituierte Alkylgruppe oder eine Alkenylgruppe und

$R_5$ eine Hydroxy-, Alkoxy-, Carboxy-, Alkoxycarbonyl-, Aminocarbonyl-, Alkylaminocarbonyl- oder Dialkylaminocarbonylgruppe, eine Alkoxygruppe mit I bis 6 Kohlenstoffatomen, die endständig durch eine Carboxy-, Alkoxycarbonyl-, Aminocarbonyl-, Alkylaminocarbonyl- oder Dialkylaminocarbonylgruppe substituiert ist, eine Alkoxygruppe mit 2 bis 7 Kohlenstoffatomen, die endständig durch eine Hydroxy-, Alkoxy-, Phenylalkoxy-, Amino-, Alkylamino-, Dialkylamino-, Pyrrolidino-, Piperidino- oder Hexamethyleniminogruppe substituiert ist, oder eine gegebenenfalls durch eine Alkylgruppe substituierte Ethenylengruppe, die endständig durch eine Carboxy-, Alkoxycarbonyl-, Aminocarbonyl-, Alkylaminocarbonyl- oder Dialkylaminocarbonylgruppe substituiert ist,

wobei alle vorstehend erwähnten Alkyl-, Alkoxy- oder Alkanoylgruppen, sofern nichts anderes erwähnt wurde, jeweils I bis 3 Kohlenstoffatome und die vorstehend erwähnten Alkenylgruppen 3 bis 5 Kohlenstoffatomen enthalten können, bedeuten, deren optische Isomere und deren Diastereomere sowie deren Säureadditionssalze.

**2.** Neue substituierte Thiazole und Oxazole der allgemeinen Formel I gemäß Anspruch I, in der

A eine gegebenenfalls durch eine Methylgruppe substituierte Ethylen- oder n-Propylengruppe,

X ein Sauerstoff- oder Schwefelatom,

$R_1$ ein Wasserstoff- oder Chloratom, eine Alkylgruppe mit I bis 3 Kohlenstoffatomen, eine Trifluormethyl-, Phenyl-, Amino-, Methylamino-, Dimethylamino-, Piperidino-, Acetylamino- oder Benzoylaminogruppe,

$R_2$ ein Wasserstoffatom oder eine Methylgrupppe,

$R_3$ ein Wasserstoffatom, eine Methylgruppe oder $R_3$ und $R_4$ zusammen eine Methoxycarbonylmethylengruppe oder eine Ethylengruppe, wobei die zum O-Atom benachbarte Methylengruppe durch eine Carbonylgruppe ersetzt sein kann,

$R_4$ ein Wasserstoffatom, eine Methyl-, 2-Hydroxy-ethyl-, Carboxymethyl-, Carbethoxymethyl- oder Benzylgruppe und

$R_5$ eine Hydroxy-, Methoxy-, Carboxy-, Methoxycarbonyl-, Ethoxycarbonyl-, Aminocarbonyl-, Carboxymethoxy-, Methoxycarbonylmethoxy-, Ethoxycarbonylmethoxy-, Aminocarbonylmethoxy-, Methylaminocarbonylmethoxy-, 2-Hydroxy-ethoxy-, 2-Ethoxy-ethoxy-, 2-Phenethoxy-ethoxy-, 2-Amino-ethoxy-, 2-Methylamino-ethoxy-, 2-(l-Piperidino)-ethoxy-, 6-Hydroxy-n-hexoxy- oder 2-Carbomethoxy-l-methyl-ethenylgruppe darstellen, bedeuten.

deren optische Isomere und deren Diastereomere sowie deren Säureadditionssalze.

3. Neue substituierte Thiazole der allgemeinen Formel

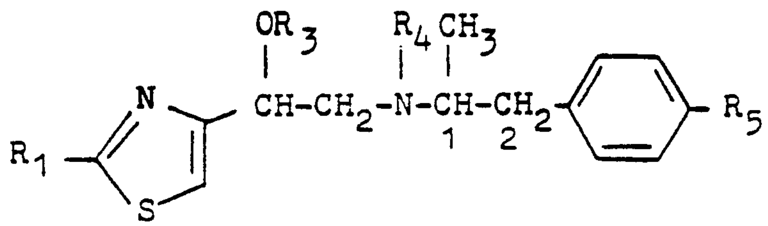

, (Ia)

in der

$R_1$ ein Chloratom, eine Methyl- oder Trifluormethylgruppe,

$R_3$ ein Wasserstoffatom oder $R_3$ und $R_4$ zusammen die Ethylen- oder Methoxycarbonylmethylengruppe,

$R_4$ ein Wasserstoffatom, eine Methyl-, 2-Hydroxy-ethyl-oder Carbethoxymethylgruppe und

$R_5$ eine Carboxymethoxy-, Carbomethoxymethoxy, Ethoxycarbonylmethoxy-, Aminocarbonylmethoxy, Methylaminocarbonylmethoxy-, 2-Methylaminoethoxy, 2-Hydroxy-ethoxy- oder 2-Carbomethoxy-1-methyl-ethenylgruppe bedeuten, deren optische Isomere und deren Diastereomere sowie deren Säureadditionssalze.

4. N-[2(4-Carbomethoxymethoxyphenyl)-l-methylethyl]-2-hydroxy-2-(2-trifluormethyl-thiazol-4-yl)ethanamin, dessen optische Isomere und dessen Diastereomere sowie dessen Säureadditionssalze.

5. N-[2-(4-Carboxymethoxyphenyl)-1-methylethyl]-2-(2-trifluormethyl-thiazol-4-yl)morpholin, dessen optische Isomere und dessen Diastereomere sowie dessen Säureadditionssalze.

6. N[2-(4-Carbomethoxymethoxyphenyl)-1-methylethyl]-N-(2-hydroxy-ethyl)-2-hydroxy-2-(2-trifluormethyl-thiazol-4-yl)-ethanamin, dessen optische Isomere und dessen Diastereomere sowie dessen Säureadditionssalze.

7. N-[2-(4-(2-Hydroxyethoxy)phenyl)-1-methylethyl]-2-hydroxy-2-(2-trifluormethyl-thiazol-4-yl)ethanamin, dessen optische Isomere und dessen Diastereomere sowie dessen Säureadditionssalze.

8. N-[2-(4-Carboxymethoxyphenyl)-1-methylethyl]-2-hydroxy-2-(2-trifluormethyl-thiazol-4-yl)-ethanamin, dessen optische Isomere und dessen Diastereomere sowie dessen Säureadditionssalze.

9. Physiologisch verträgliche Salze der Verbindungen gemäß den Ansprüchen 1 bis 8 mit anorganischen oder organischen Säuren.

10. Arzneimittel, enthaltend eine Verbindung gemäß den Ansprüchen 1 bis 8 oder ein physiologisch verträgliches Säureadditionssalz gemäß Anspruch 9 neben gegebenenfalls einem oder mehreren inerten Trägerstoffen und/oder Verdünnungsmitteln.

11. Arzneimittel gemäß Anspruch 10, welches zur Behandlung des Diabetes mellitus, der Adipositas und zur Behandlung und Prophylaxe atherosklerotischer Veränderungen geeignet ist.

12. Verwendung der Verbindungen gemäß den Ansprüchen 1 bis 9 zur Herstellung eines Arzneimittels, das für die Behandlung des Diabetes mellitus, der Adipositas und zur Behandlung und Prophylaxe

atherosklerotischer Veränderungen.

**13.** Verfahren zur Herstellung eines Arzneimittels gemäß Anspruch 10 oder 11, dadurch gekennzeichnet, daß auf nichtchemischem Wege eine Verbindung gemäß den Ansprüchen 1 bis 9 in einen oder mehrere inerte Trägerstoffe und/oder Verdünnungsmittel eingearbeitet wird.

**14.** Verfahren zur Herstellung der neuen Thiazole und Oxazole gemäß den Ansprüchen 1 bis 9, dadurch gekennzeichnet, daß

a) eine Verbindung der allgemeinen Formel

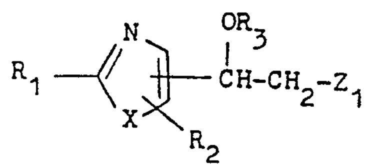

,(II)

mit einer Verbindung der allgemeinen Formel

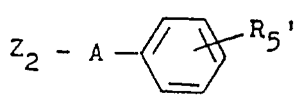

,(III)

in denen

$R_1$ bis $R_3$, A und X wie in den Ansprüchen 1 bis 8 definiert sind,

$Z_1$ eine nukleophile Austrittsgruppe und $Z_2$ eine $R_4$-NH-Gruppe oder

$Z_2$ eine nukleophile Austrittsgruppe und $Z_1$ eine $R_4$-NH-Gruppe darstellen, wobei $R_4$ wie in den Ansprüchen 1 bis 8 definiert ist, und

$R_5'$ die für $R_5$ in den Ansprüchen 1 bis 8 erwähnten Bedeutungen besitzt, wobei jedoch $R_5'$ keine der in den Ansprüchen 1 bis 8 erwähnten Alkoxycarbonylmethoxygruppen darstellen kann und eine im Rest $R_5$ enthaltene Carboxy-, Amino- oder Alkylaminogruppe durch einen Schutzrest geschützt sein kann, umgesetzt und gegebenenfalls anschließend ein verwendeter Schutzrest abgespalten wird oder

b) eine gegebenenfalls im Reaktionsgemisch gebildete Schiff'schen Base der allgemeinen Formel

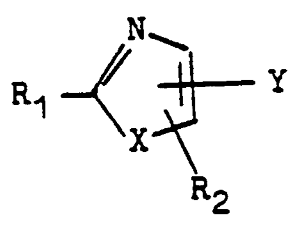

,(IV)

in der

$R_1$, $R_2$ und X wie in den Ansprüchen 1 bis 8 definiert sind und

Y eine Gruppe der Formel

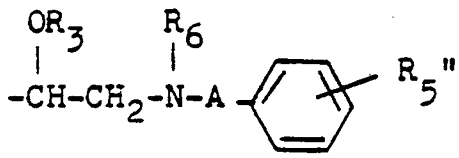

oder

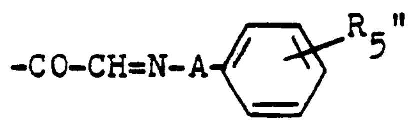

darstellen, wobei

$R_3$ und A wie in den Ansprüchen 1 bis 8 definiert sind,
$R_5$" die für $R_5$ in den Ansprüchen 1 bis 8 erwähnten Bedeutungen besitzt, wobei jedoch eine im Rest $R_5$ enthaltene Amino- oder Alkylaminogruppe durch einen Schutzrest geschützt sein kann, und
$R_6$ zusammen mit einem Wasserstoffatom des benachbarten Kohlenstoffatomes des Restes A eine weitere Bindung darstellt, reduziert und gegebenenfalls anschließend ein verwendeter Schutzrest abgespalten wird oder
c) zur Herstellung von Verbindungen der allgemeinen Formel 1, in der $R_3$ und $R_4$ wie in den Ansprüchen 1 bis 8 definiert sind, wobei jedoch $R_3$ und $R_4$ keine Ethylengruppe, in der die zum N-Atom benachbarte Methylengruppe durch eine Carbonylgruppe ersetzt ist, darstellen, eine Garbonylverbindung der allgemeinen Formel

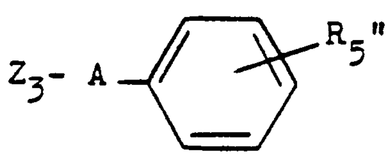

,(V)

in der
A wie in den Ansprüchen 1 bis 8 definiert ist,
$R_5$" die für $R_5$ in den Ansprüchen 1 bis 8 erwähnten Bedeutungen besitzt, wobei jedoch eine im Rest $R_5$ enthaltene Amino- oder Alkylaminogruppe durch einen Schutzrest geschützt sein kann, und
$Z_3$ zusammen mit einem Wasserstoffatom des benachbarten Kohlenstoffatomes des Restes A ein Sauerstoffatom darstellt, mit einem Amin der allgemeinen Formel

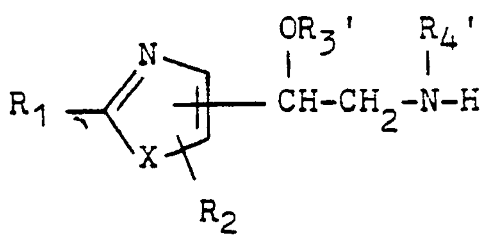

,(VI)

in der
$R_1$, $R_2$ und X wie in den Ansprüchen I bis 8 definiert sind,

$R_3'$ und $R_4'$ die für $R_3$ und $R_4$ in den Ansprüchen I
bis 8 erwähnten Bedeutungen besitzen, wobei jedoch $R_3$ und $R_4$ zusammen keine Ethylengruppe, in der die zum N-Atom benachbarte Methylengruppe durch eine Carbonylgruppe ersetzt ist, bedeuten,

in Gegenwart eines geeigneten Reduktionsmittels reduktiv aminiert und gegebenenfalls anschließend ein verwendeter Schutzrest abgespalten wird oder
d) zur Herstellung von Verbindungen der allgemeinen Formel I, in der $R_3$ ein Wasserstoffatom darstellt, eine gegebenenfalls im Reaktionsgemisch gebildete Verbindung der allgemeinen Formel

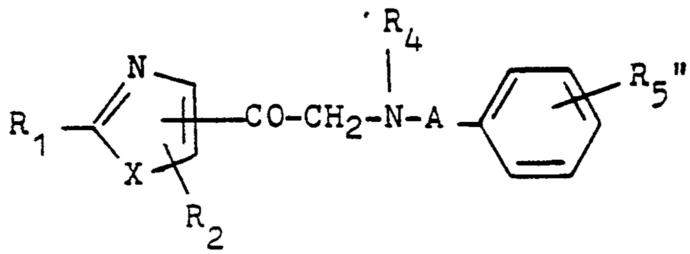

,(VII)

in der
A, X, $R_1$, $R_2$ und $R_4$ wie in den Ansprüchen I bis 8 definiert sind und

$R_5''$ die für $R_5$ in den Ansprüchen I bis 8 erwähnten Bedeutungen besitzt, wobei jedoch eine im Rest $R_5$ enthaltene Amino- oder Alkylaminogruppe durch einen Schutzrest geschützt sein kann, reduziert und gegebenenfalls anschließend ein verwendeter Schutzrest abgespalten wird oder
e) zur Herstellung von Verbindungen der allgemeinen Formel I, in der $R_3$ und $R_4$ zusammen eine Alkoxycarbonylmethylengruppe darstellen, eine Verbindung der allgemeinen Formel

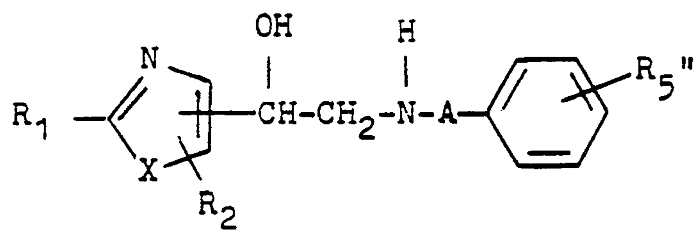

,(VIII)

in der
A, X, $R_1$ und $R_2$ wie in den Ansprüchen I bis 8 definiert sind und

$R_5''$ die für $R_5$ in den Ansprüchen I bis 8 erwähnten Bedeutungen besitzt, wobei jedoch eine im Rest $R_5$ enthaltene Amino- oder Alkylaminogruppe durch einen Schutzrest geschützt sein kann, mit einer Verbindung der allgemeinen Formel

$O = CH - COOR_7$ ,(IX)

in der
$R_7$ eine Alkylgruppe mit I bis 3 Kohlenstoffatomen darstellt, umgesetzt und gegebenenfalls anschließend ein verwendeter Schutzrest abgespalten wird oder
f) zur Herstellung von Verbindungen der allgemeinen Formel I, in der $R_5$ eine Alkoxygruppe mit I bis 3 Kohlenstoffatomen, eine Alkoxygruppe mit I bis 6 Kohlenstoffatomen, die endständig durch eine Carboxy-, Alkoxycarbonyl-, Aminocarbonyl-, Alkylaminocarbonyl- oder Dialkylaminocarbonylgruppe substituiert ist, oder eine Alkoxygruppe mit 2 bis 7 Kohlenstoffatomen, die endständig durch eine Hydroxy-, Alkoxy-, Amino-, Alkylamino-, Dialkylamino-, Pyrrolidino-, Piperidino- oder Hexamethyleniminogruppe substituiert ist, darstellt, eine Verbindung der allgemeinen Formel

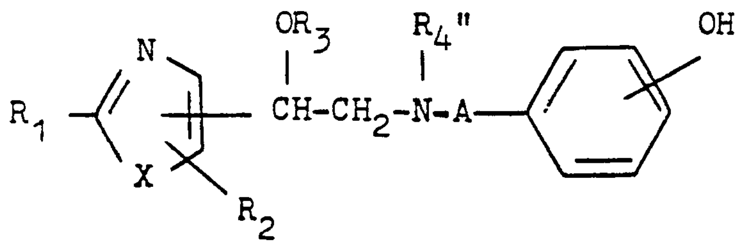

,(X)

in der
A, X und $R_1$ bis $R_3$ wie in den Ansprüchen I bis B definiert sind und
$R_4''$ die für $R_4$ in den Ansprüchen I bis 8 erwähnten Bedeutungen besitzt oder eine leicht abspaltbare Schutzgruppe für eine Aminogruppe darstellt, mit einer Verbindung der allgemeinen Formel

$Z_4$ - $R_8$ ,(XI)

in der
$R_8$ eine Alkylgruppe mit I bis 3 Kohlenstoffatomen, eine Alkylgruppe mit I bis 6 Kohlenstoffatomen, die endständig durch eine Carboxy-, Alkoxycarbonyl-, Aminocarbonyl-, Alkylaminocarbonyl- oder Dialkylaminocarbonylgruppe substituiert ist, oder eine Alkylgruppe mit 2 bis 7 Kohlenstoffatomen, die endständig durch eine Hydroxy-, Alkoxy-, Amino-, Alkylamino-, Dialkylamino-, Pyrrolidino-, Piperidino- oder Hexamethyleniminogruppe substituiert ist, und

$Z_4$ eine nukleophile Austrittsgruppe wie ein Halogenatom oder eine Sulfonyloxygruppe oder
$Z_4$ zusammen mit einem $\beta$-Wasserstoffatom des Restes $R_8$ ein Sauerstoffatom darstellen, umgesetzt und gegebenenfalls anschließend ein verwendeter Schutzrest abgespalten wird oder
g) zur Herstellung von Verbindungen der allgemeinen Formel I, in der $R_3$ ein Wasserstoffatom darstellt, eine Verbindung der allgemeinen Formel

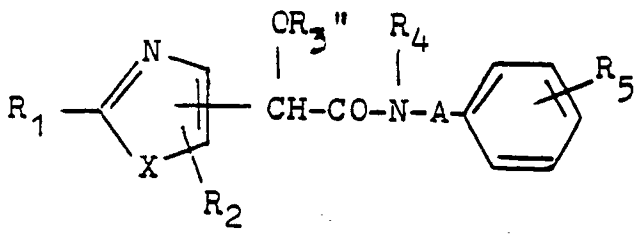

,(XII)

in der
A, X, $R_1$, $R_2$ und $R_4$ wie in den Ansprüchen I bis 8 definiert sind und
$R_3''$ ein Wasserstoffatom oder eine Alkylgruppe bedeutet, reduziert wird oder
h) zur Herstellung von Verbindungen der allgemeinen Formel I, in der $R_5$ eine Alkoxycarbonyl-, Aminocarbonyl-, Alkylaminocarbonyl- oder Dialkylaminocarbonylgruppe darstellt oder enthält, eine Verbindung der allgemeinen Formel

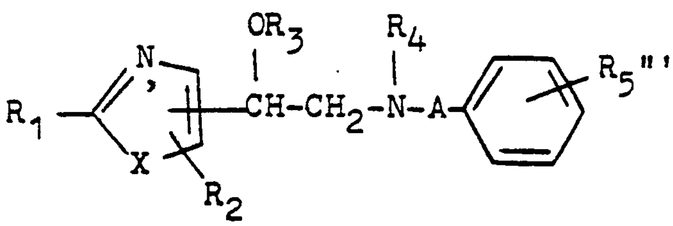

,(XIII)

in der
$R_1$ bis $R_4$, A und X wie in den Ansprüchen I bis S definiert sind und
$R_5'''$ eine Carboxygruppe oder eine Alkoxygruppe mit I bis 6 Kohlenstoffatomen, welche endständig durch eine Carboxygruppe substituiert ist, darstellt, oder deren reaktionsfähige Derivate, mit einer Verbindung der allgemeinen Formel

$H\text{-}R_9$ ,(XIV)

in der
$R_9$ eine Alkoxy-, Amino-, Alkylamino- oder Dialkylaminogruppe darstellt, wobei jeweils der Alkyl- oder Alkoxyteil I bis 3 Kohlenstoffatome enthalten kann, umgesetzt wird oder
i) zur Herstellung von Verbindungen der allgemeinen Formel I, in der $R_4$ eine gegebenenfalls durch eine Phenyl-, Carboxy-, Alkoxycarbonyl- oder Cyanogruppe oder in 2- oder 3-Stellung durch eine Hydroxygruppe substituierte Alkylgruppe oder eine Alkenylgruppe darstellt, eine Verbindung der allgemeinen Formel

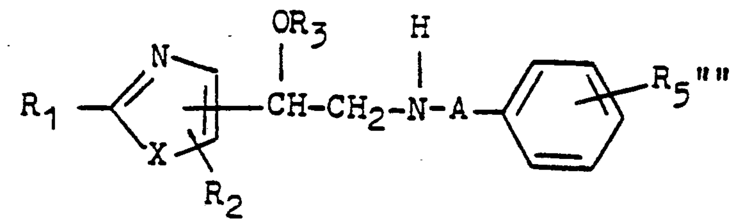
,(XV)

in der
A, X und $R_1$ bis $R_3$ wie in den Ansprüchen I bis 8 definiert sind und

$R_5''''$ die für $R_5$ in den Ansprüchen I bis 8 erwähnten Bedeutungen besitzt, wobei jedoch eine im Rest $R_5$ enthaltene Amino- oder Alkylaminogruppe erforderlichenfalls durch einen Schutzrest geschützt sein kann, mit einer Verbindung der allgemeinen Formel

$Z_5 - R_4''$ ,(XVI)

in der
$R_4'''$ mit Ausnahme des Wasserstoffatomes die für $R_4$ eingangs erwähnten Bedeutungen besitzt und $Z_5$ eine nukleophile Austrittsgruppe oder $Z_5$ zusammen mit einem $\alpha$- oder $\beta$-Wasserstoffatom des Alkylrestes $R_4'''$ ein Sauerstoffatom bedeuten, umgesetzt und gegebenenfalls anschließend ein verwendeter Schutzrest abgespalten wird oder
k) zur Herstellung von Verbindungen der allgemeinen Formel I, in der $R_3$ und $R_4$ zusammen eine Ethylengruppe darstellen, eine gegebenenfalls im Reaktionsgemisch gebildete Verbindung der allgemeinen Formel

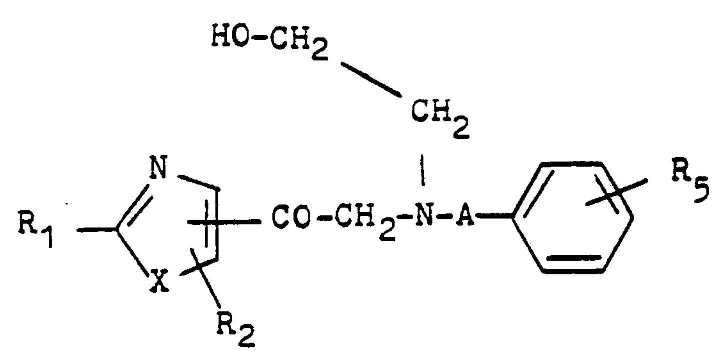
,(XXII)

in der
A, X, $R_1$, $R_2$ und $R_5$ wie in den Ansprüchen I bis 8 definiert sind, oder dessen cyclischen Halbacetals reduziert wird oder
l) zur Herstellung von Verbindungen der allgemeinen Formel I in der $R_3$ und $R_4$ zusammen eine Ethylengruppe, in der die zum N- oder O-Atom benachbarte Methylengruppe durch eine Carbonylgruppe ersetzt ist, bedeuten, eine Verbindung der allgemeinen Formel

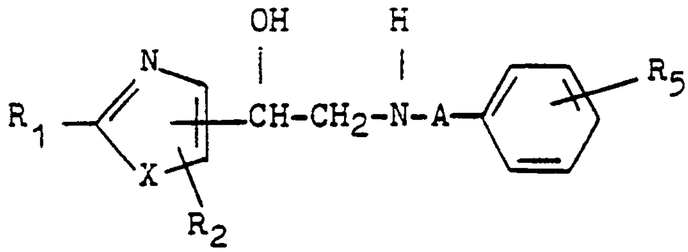
,(XVIII)

in der
A, X, $R_1$, $R_2$ und $R_5$ wie in den Ansprüchen I bis 8 definiert sind, mit einem Halogenacetylhalogenid oder Halogen essigester umgesetzt wird oder
m) zur Herstellung von Verbindungen der allgemeinen Formel I, in der $R_3$ und $R_4$ zusammen eine Ethylengruppe bedeuten, eine Verbindung der allgemeinen Formel

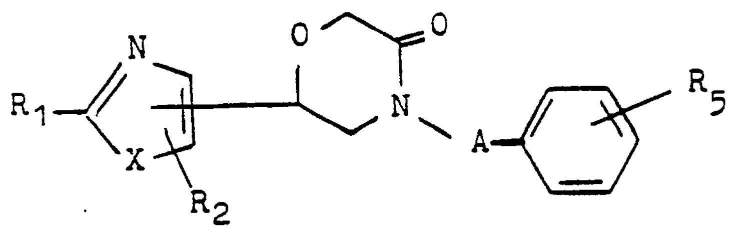
, (XIX)

in der
A, X, $R_1$, $R_2$ und $R_5$ wie in den Ansprüchen I bis 8 definiert sind, reduziert wird oder
n) zur Herstellung von Verbindungen der allgemeinen Formel I, in der $R_3$ und $R_4$ zusammen eine Ethylengruppe, in welcher die zum O-Atom benachbarte Methylengruppe durch eine Carbonylgruppe ersetzt ist, darstellen, eine gegebenenfalls im Reaktionsgemisch gebildete Verbindung der allgemeinen Formel

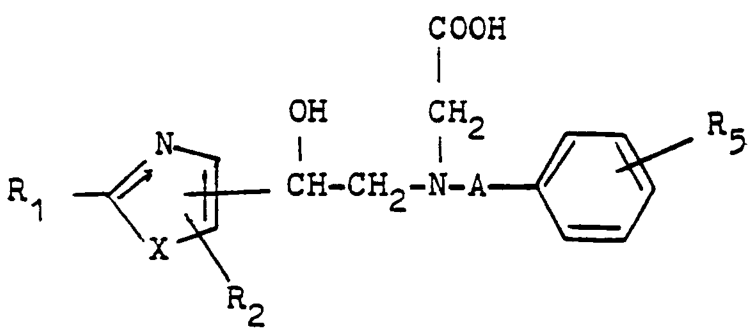
,(XX)

in der
A, X, $R_1$, $R_2$ und $R_5$ wie in den Ansprüchen I bis 8 definiert sind, oder deren reaktionsfähige Derivate wie deren Ester oder Halogenide cyclisiert wird oder
o) zur Herstellung von Verbindungen der allgemeinen Formel I, in der $R_1$ ein Wasserstoff- oder Halogenatom, eine Trifluormethyl- oder Alkylgruppe und $R_3$ ein Wasserstoffatom darstellen, eine

Verbindung der allgemeinen Formel

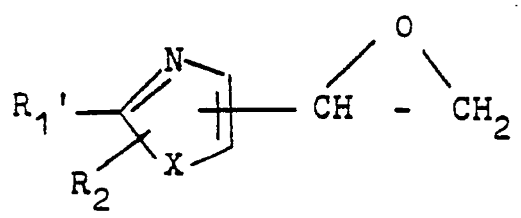

, (XXI)

in der
X und $R_2$ wie in den Ansprüchen I bis 8 definiert sind und
$R_1'$ ein Wasserstoff- oder Halogenatom, eine Trifluormethyl- oder Alkylgruppe darstellt, mit einem Amin der allgemeinen Formel

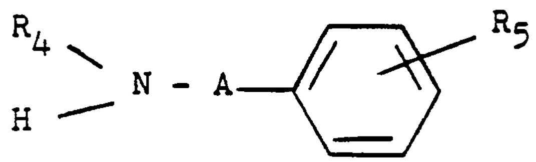

, (XXII)

in der
A, $R_4$ und $R_5$ wie in den Ansprüchen I bis B definiert sind, umgesetzt wird und

gewünschtenfalls eine so erhaltene Verbindung der allgemeinen Formel I, in der $R_5$ eine Alkoxycarbonyl-, Aminocarbonyl-, Alkylaminocarbonyl- oder Dialkylaminocarbonylgruppe darstellt oder enthält und/oder $R_1$ eine durch eine Alkanoyl- oder Benzoylgruppe substituierte Aminogruppe darstellt, mittels Hydrolyse in eine entsprechende Verbindung der allgemeinen Formel I, in der $R_5$ eine Carboxygruppe darstellt oder enthält und/oder $R_1$ eine Aminogruppe darstellt, übergeführt wird und/oder

eine so erhaltene Verbindung der allgemeinen Formel I, in der $R_5$ eine durch eine Carboxy-, Alkoxycarbonyl-, Aminocarbonyl-, Alkylaminocarbonyl- oder Dialkylaminocarbonylgruppe substituierte Alkoxygruppe darstellt, mittels Reduktion durch ein geeignetes Metallhydrid in eine Verbindung der allgemeinen Formel I, in der der vorstehend erwähnte substituierte Alkoxyrest anstelle der Carbonylgruppe eine Methylengruppe enthält, übergeführt wird und/oder

eine so erhaltene Verbindung der allgemeinen Formel I, in der $R_3$ eine Alkylgruppe und/oder $R_5$ eine der in den Ansprüchen I bis 7 erwähnten Alkoxygruppen darstellt, mittels Etherspaltung in eine entsprechende Verbindung der allgemeinen Formel I, in der $R_3$ ein Wasserstoffatom und/oder $R_5$ eine Hydroxygruppe oder eine durch eine Hydroxygruppe substituierte Alkoxygruppe darstellt, übergeführt wird und/oder

eine so erhaltene Verbindung der allgemeinen Formel I in ihre optischen Isomere und Diastereomere aufgetrennt wird und/oder

eine so erhaltene Verbindung der allgemeinen Formel I in ihre Säureadditionssalze, insbesondere in ihre physiologisch verträglichen Säureadditionssalze übergeführt werden.

## Claims

1. New substituted thiazoles and oxazoles of the general formula

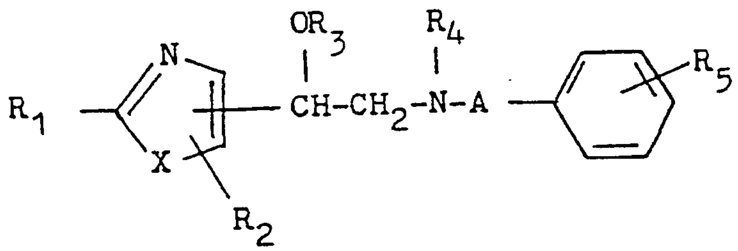

(I)

in which

A denotes an n-alkylene group which is optionally monosubstituted or disubstituted by methyl or ethyl groups and has 2 or 3 carbon atoms,

X denotes an oxygen or sulphur atom,

$R_1$ denotes a hydrogen or halogen atom, a trifluoromethyl, alkyl, phenyl or piperidino group or an amino group which is optionally substituted by one or two alkyl groups or one alkanoyl or benzoyl group,

$R_2$ denotes a hydrogen atom or an alkyl group,

$R_3$ denotes a hydrogen atom or an alkyl group which can be substituted in the 2- or 3-position by a hydroxyl group, or $R_3$ together with $R_4$ denotes an alkoxycarbonylmethylene group or an ethylene group, it being possible for the methylene group adjacent to the N or O atom to be replaced by a carbonyl group,

$R_4$ denotes a hydrogen atom, an alkyl group which is optionally substituted by a phenyl, carboxyl, alkoxycarbonyl or cyano group or, in the 2- or 3-position, by a hydroxyl group, or denotes an alkenyl group, and

$R_5$ denotes a hydroxyl, alkoxy, carboxyl, alkoxycarbonyl, aminocarbonyl, alkylaminocarbonyl or dialkylaminocarbonyl group, an alkoxy group which has 1 to 6 carbon atoms and is substituted by a terminal carboxyl, alkoxycarbonyl, aminocarbonyl, alkylaminocarbonyl or dialkylaminocarbonyl group, an alkoxy group which has to 2 to 7 carbon atoms and is substituted by a terminal hydroxyl, alkoxy, phenylalkoxy, amino, alkylamino, dialkylamino, pyrrolidino, piperidino or hexamethyleneimino group, or an ethylene group which is optionally substituted by an alkyl group and is substituted by a terminal carboxyl, alkoxycarbonyl, aminocarbonyl, alkylaminocarbonyl or dialkylaminocarbonyl group,

it being possible for all the above-mentioned alkyl, alkoxy or alkanoyl groups each to contain, unless mentioned otherwise, 1 to 3 carbon atoms, and for the above-mentioned alkenyl groups to contain 3 to 5 carbon atoms

their optical isomers and their diastereomers as well as their acid addition salts.

2. New substituted thiazoles and oxazoles of the general formula I according to claim 1, in which

A denotes an ethylene or n-propylene group which is optionally substituted by a methyl group,

X denotes an oxygen or sulphur atom,

$R_1$ denotes a hydrogen or chlorine atom, an alkyl group with 1 to 3 carbon atoms, a trifluoromethyl, phenyl, amino, methylamino, dimethylamino, piperidino, acetylamino or benzoylamino group,

$R_2$ denotes a hydrogen atom or a methyl group,

$R_3$ denotes a hydrogen atom or a methyl group, or $R_3$ and $R_4$ together denote a methoxycarbonylmethylene group or an ethylene group, it being possible for the methylene group adjacent to the O atom to be replaced by a carbonyl group,

$R_4$ denotes a hydrogen atom, a methyl, 2-hydroxyethyl, carboxymethyl, carbethoxymethyl or benzyl group and

$R_5$ denotes a hydroxy, methoxy, carboxy, methoxycarbonyl, ethoxycarbonyl, aminocarbonyl, carboxymethoxy, methoxycarbonylmethoxy, ethoxycarbonylmethoxy, aminocarbonylmethoxy, methylaminocarbonylmethoxy, 2-hydroxy-ethoxy, 2-ethoxyethoxy, 2-phenethoxyethoxy, 2-aminoethoxy, 2-methylaminoethoxy, 2-(1-piperidino)ethoxy, 6-hydroxy-n-hexoxy or 2-carbomethoxy-1-methylethenyl group,

their optical isomers and their diastereomers as well as their acid addition salts.

3. New substituted thiazoles of the general formula

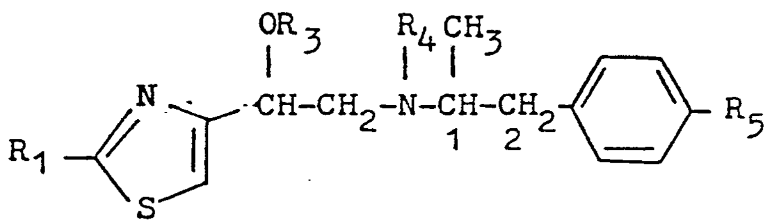

(Ia)

in which
$R_1$ denotes a chlorine atom, a methyl or trifluoromethyl group,

$R_3$ denotes a hydrogen atom, or $R_3$ and $R_4$ together denote an ethylene or methoxycarbonylmethylene group,

$R_4$ denotes a hydrogen atom, a methyl, 2-hydroxyethyl or carbethoxymethyl group, and

$R_5$ denotes a carboxymethoxy, carbomethoxymethoxy, ethoxycarbonylmethoxy, aminocarbonylmethoxy, methylaminocarbonylmethoxy, 2-methylaminoethoxy, 2-hydroxyethoxy or 2-carbomethoxy-1-methylethenyl group, their optical isomers and their diastereomers as well as their acid addition salts.

4. N-[2(4-Carbomethoxymethoxyphenyl)-1-methylethyl]-2-hydroxy-2- (2-trifluoromethyl-thiazol-4-yl) ethanamine, its optical isomers and its diastereomers as well as its acid addition salts.

5. N-[2-(4-Carboxymethoxyphenyl)-1-methylethyl]-2- (2-trifluoromethyl-thiazol-4-yl)morpholine, its optical isomers and its diastereomers as well as its acid addition salts.

6. N-[2-(4-Carbomethoxymethoxyphenyl)-1-methylethyl]-N- (2-hydroxy-ethyl) -2-hydroxy-2- (2-trifluoromethyl-thiazol-4-yl)ethanamine, its optical isomers and its diastereomers as well as its acid addition salts.

7. N-[2-(4-(2-Hydroxyethoxy)phenyl)-1-methylethyl]-2-hydroxy-2- ( 2-trifluoromethyl-thiazol-4-yl) ethanamine, its optical isomers and its diastereomers as well as its acid addition salts.

8. N[2-(4-Carboxymethoxyphenyl)-1-methylethyl] -2-hydroxy-2- (2-trifluoromethyl-thiazol-4-yl) -ethanamine, its optical isomers and its diastereomers as well as its acid addition salts.

9. Physiologically acceptable salts of the compounds according to claims 1 to 8 with inorganic or organic

acids.

**10.** Medicaments containing a compound according to claims 1 to 8 or a physiologically acceptable acid addition salt according to claim 9, in addition to, where appropriate, one or more inert carriers and/or diluents.

**11.** Medicament according to claim 10 which is suitable for the treatment of diabetes mellitus and obesity and for the treatment and prophylaxis of atherosclerotic changes.

**12.** Use of the compounds according to claims 1 to 9 for the preparation of a medicament which is suitable for the treatment of diabetes mellitus and of obesity and for the treatment and prophylaxis of atherosclerotic changes.

**13.** Process for the preparation of a medicament according to claim 10 or 11, characterised in that, by non-chemical means, a compound according to claims 1 to 9 is incorporated into one or more inert carriers and/or diluents.

**14.** Process for the preparation of the new thiazoles and oxazoles according to claims 1 to 9, characterised in that

a) a compound of the general formula

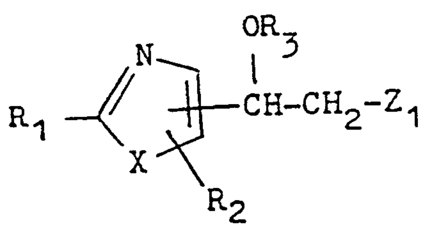

(II)

is reacted with a compound of the general formula

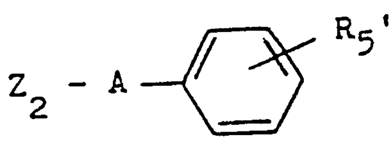

(III)

(in which

$R_1$ to $R_3$, A and X are as defined in claims 1 to 8,

$Z_1$ represents a nucleophilic leaving group, and
$Z_2$ represents a $R_4$-NH group, or

$Z_2$ represents a nucleophilic leaving group, and
$Z_1$ represents a $R_4$-NH group, $R_4$ being defined as in claims 1 to 8, and

$R_5$' has the meanings mentioned for $R_5$ in claims 1 to 8, but it not being possible for $R_5$' to represent any of the alkoxycarbonylmethoxy groups mentioned in claims 1 to 8, and it being possible for a carboxyl, amino or alkylamino group contained in the radical $R_5$ to be protected by a protecting group), and then, where appropriate, any protecting group which has been used is eliminated, or

b) a Schiff's base, which is optionally formed in the reaction mixture, of the general formula

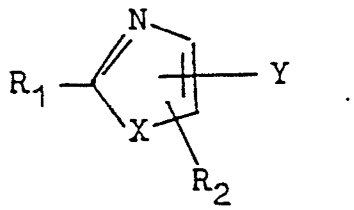

(IV)

(in which
$R_1$, $R_2$ and X are as defined in claims 1 to 8, and Y represents a group of the formula

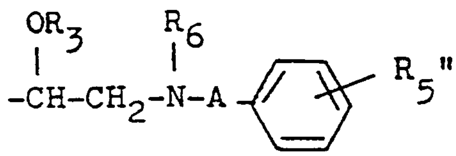

or

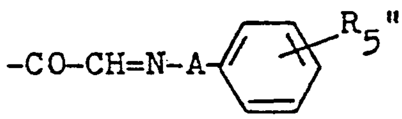

$R_3$ and A being defined as in claims 1 to 8,
$R_5$" having the meanings mentioned for $R_5$ in claims 1 to 8, but it being possible for an amino or alkylamino group contained in the radical $R_5$ to be protected by a protecting group, and
$R_6$ together with a hydrogen atom on the adjacent carbon atom of the radical A, represents another bond), is reduced and then, where appropriate, any protecting group which has been used is eliminated, or
c) for the preparation of compounds of the general formula I in which $R_3$ and $R_4$ are as defined in claims 1 to 8, (but $R_3$ and $R_4$ do not represent an ethylene group in which the methylene group adjacent to the N atom has been replaced by a carbonyl group), a carbonyl compound of the general formula

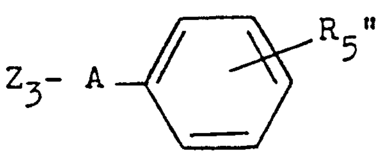

(V)

(in which
A is as defined in claims 1 to 8,
$R_5$" has the meanings mentioned for $R_5$ in claims 1 to 8, but it being possible for an amino or alkylamino group contained in the radical $R_5$ to be protected by a protecting group, and
$Z_3$, together with a hydrogen atom of the adjacent carbon atom of the radical A, represents an oxygen atom), is reductively aminated with an amine of the general formula

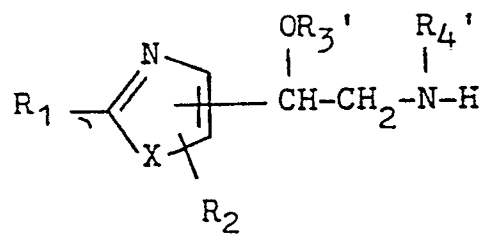

(VI)

(in which
$R_1$, $R_2$ and X are as defined in claims 1 to 8,

$R_3$' and $R_4$' have the meanings mentioned for $R_3$ and $R_4$ in claims 1 to 8, but $R_3$ and $R_4$ do not together denote an ethylene group in which the methylene group adjacent to the N atom has been replaced by a carbonyl group), in the presence of a suitable reducing agent and then, where appropriate, any protecting group which has been used is eliminated, or
d) for the preparation of compounds of the general formula I in which $R_3$ represents a hydrogen atom, a compound, which is optionally formed in the reaction mixture, of the general formula

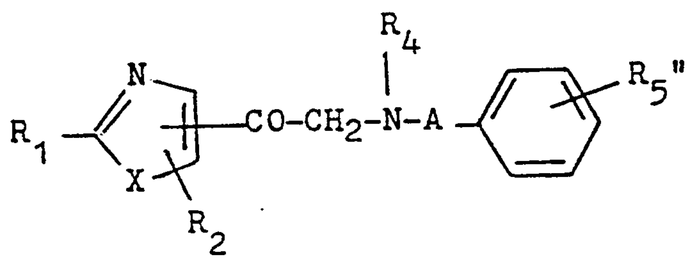

(VII)

(in which
A, X, $R_1$, $R_2$ and $R_4$ are as defined in claims 1 to 8, and $R_5$" has the meanings mentioned for $R_5$ in claims 1 to 8, but it being possible for an amino or alkylamino group contained in the radical $R_5$ to be protected by a protecting group), is reduced, and then, where appropriate, any protecting group which has been used is eliminated, or
e) for the preparation of compounds of the general formula I in which $R_3$ and $R_4$ together represent an alkoxycarbonylmethylene group, a compound of the general formula

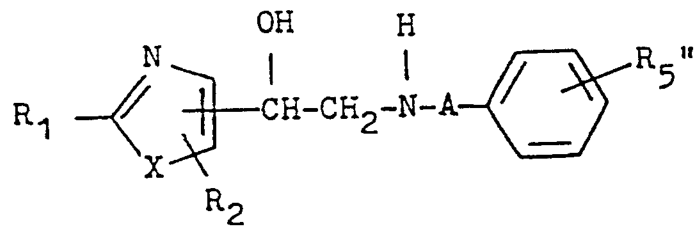

(VIII)

(in which
A, X, $R_1$ and $R_2$ are as defined in claims 1 to 8, and

$R_5$" has the meanings mentioned for $R_5$ in claims 1 to 8, but it being possible for an amino or alkylamino group contained in the radical $R_5$ to be protected by a protecting group), is reacted with a compound of the general formula

$O = CH - COOR_7$ (IX)

(in which
$R_7$ represents an alkyl group with 1 to 3 carbon atoms), and then, where appropriate, any protecting group which has been used is eliminated, or
f) for the preparation of compounds of the general formula I in which $R_5$ represents an alkoxy group with 1 to 3 carbon atoms, an alkoxy group which has 1 to 6 carbon atoms and is substituted by a terminal carboxyl, alkoxycarbonyl, aminocarbonyl, alkylaminocarbonyl or dialkylaminocarbonyl group, or an alkoxy group which has 2 to 7 carbon atoms and is substituted by a terminal hydroxy, alkoxy, amino, alkylamino, dialkylamino, pyrrolidino, piperidino or hexamethyleneimino group, a compound of the general formula

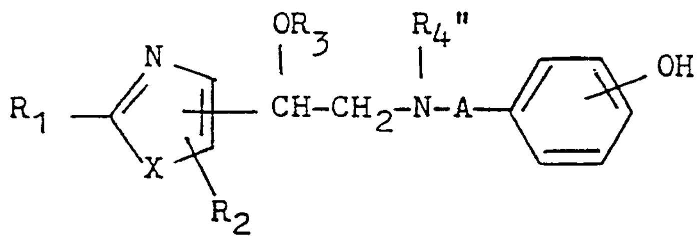

(X)

(in which
A, X and $R_1$ to $R_3$ are as defined in claims 1 to 8, and
$R_4$" has the meanings mentioned for $R_4$ in claims 1 to 8, or represents a protecting group for an amino group which can be readily eliminated) is reacted with a compound of the general formula

$Z_4 - R_8$ (XI)

(in which
$R_8$ represents an alkyl group with 1 to 3 carbon atoms, an alkyl group which has 1 to 6 carbon atoms and is substituted by a terminal carboxyl, alkoxycarbonyl, aminocarbonyl, alkylaminocarbonyl or dialkylaminocarbonyl group, or an alkoxy group which has 2 to 7 carbon atoms and is substituted by a terminal hydroxyl, alkoxy, amino, alkylamino, dialkylamino, pyrrolidino, piperidino or hexamethyleneimino group, and

$Z_4$ represents a nucleophilic leaving group such as a halogen atom or a sulphonyloxy group, or $Z_4$ together with a $\beta$-hydrogen atom of the radical $R_8$ represents an oxygen atom) and then, where appropriate, any protecting group which has been used is eliminated, or
g) for the preparation of compounds of the general formula I in which $R_3$ represents a hydrogen atom, a compound of the general formula

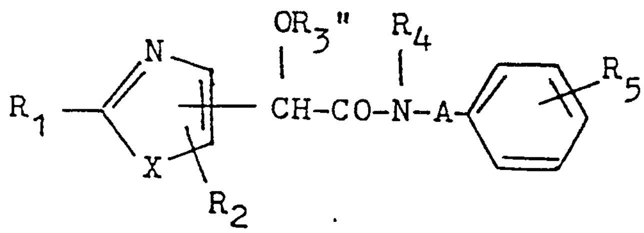

(XII)

(in which
A, X, $R_1$, $R_2$ and $R_4$ are as defined in claims 1 to 8, and

$R_3$" denotes a hydrogen atom or an alkyl group), is reduced, or

h) for the preparation of compounds of the general formula I in which $R_5$ represents or contains an alkoxycarbonyl, aminocarbonyl, alkylaminocarbonyl, or dialkylaminocarbonyl group, a compound of the general formula

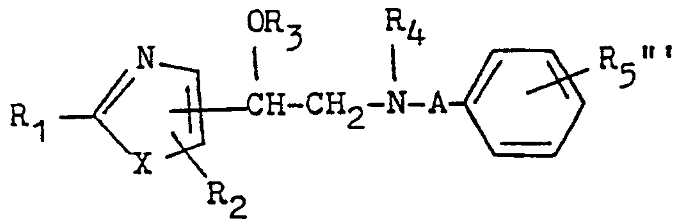

(XIII)

(in which

$R_1$ to $R_4$, A and X are as defined in claims 1 to 8, and

$R_5$"' represents a carboxyl group or an alkoxy group which has 1 to 6 carbon atoms and is substituted by a terminal carboxyl group), or a reactive derivative thereof, is reacted with a compound of the general formula

$H - R_9$ (XIV)

in which

$R_9$ represents an alkoxy, amino, alkylamino or dialkylamino group, it being possible for each alkyl or alkoxy moiety to contain 1 to 3 carbon atoms, or

i) for the preparation of compounds of the general formula I in which $R_4$ represents an alkyl group which is optionally substituted by a phenyl, carboxyl, alkoxycarbonyl or cyano group or, in the 2- or 3-position, by a hydroxyl group, or represents an alkenyl group, a compound of the general formula

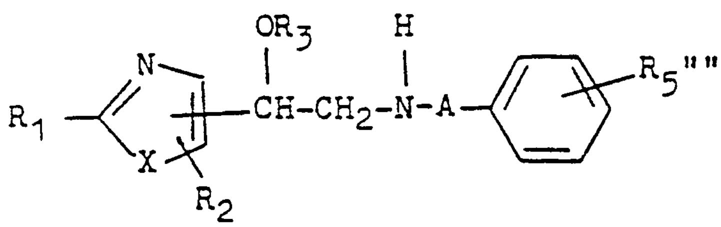

(XV)

(in which

A, X and $R_1$ to $R_3$ are as defined in claims 1 to 8, and

$R_5$"" has the meanings mentioned for $R_5$ in claims 1 to 8, but it being possible for an amino or alkylamino group contained in the radical $R_5$ to be, if necessary, protected by a protecting group), is reacted with a compound of the general formula

$Z_5 - R_4$"' (XVI)

(in which

$R_4$"' has the meanings mentioned above for $R^4$ with the exception of the hydrogen atom, and

$Z_5$ denotes a nucleophilic leaving group, or $Z_5$ together with an $\alpha$ or $\beta$ hydrogen atom of the alkyl radical $R_4$"' denotes an oxygen atom) and, where appropriate, any protecting group which has been used is eliminated, or

k) for the preparation of compounds of the general formula I in which $R_3$ and $R_4$ together represent an ethylene group, a compound, which is optionally formed in the reaction mixture, of the general

formula

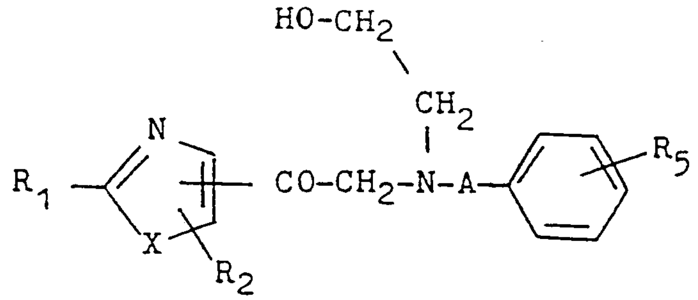

(XVII)

(in which

A, X, $R_1$, $R_2$ and $R_5$ are as defined in claims 1 to 8), or its cyclic hemiacetal, is reduced, or

1) for the preparation of compounds of the general formula I in which $R_3$ and $R_4$ together denote an ethylene group in which the methylene group adjacent to the N or O atom has been replaced by a carbonyl group, a compound of the general formula

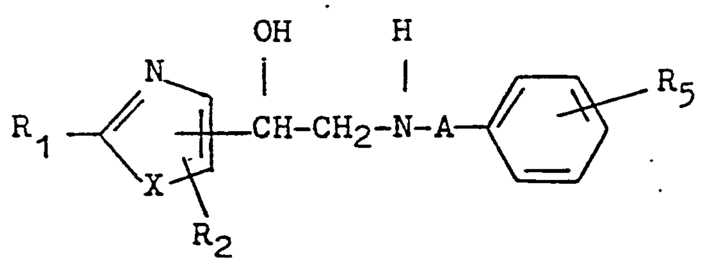

(XVIII)

(in which

A, X, $R_1$, $R_2$ and $R_5$ are as defined in claims 1 to 8), is reacted with a haloacetyl halide or haloacetic acid ester, or

m) for the preparation of compounds of the general formula I in which $R_3$ and $R_4$ together denote an ethylene group, a compound of the general formula

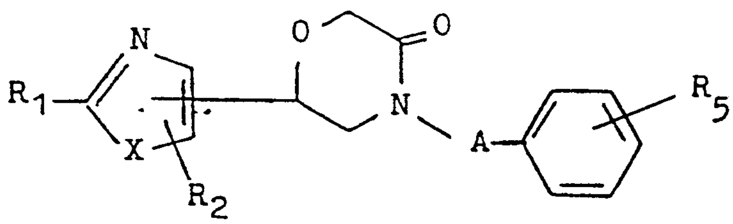

(XIX)

(in which

A, X, $R_1$, $R_2$ and $R_5$ are as defined in claims 1 to 8), is reduced, or

n) for the preparation of compounds of the general formula I in which $R_3$ and $R_4$ together represent an ethylene group in which the methylene group adjacent to the O atom has been replaced by a carbonyl group, a compound, which is optionally formed in the reaction mixture, of the general formula

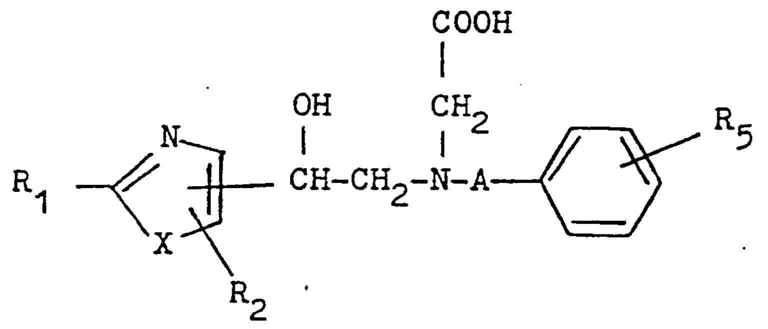

(XX)

(in which
A, X, $R_1$, $R_2$ and $R_5$ are as defined in claims 1 to 8), or a reactive derivative thereof such as an ester or halide, is cyclized, or
o) for the preparation of compounds of the general formula I in which $R_1$ represents a hydrogen or halogen atom, a trifluoromethyl or alkyl group, and $R_3$ represents a hydrogen atom, a compound of the general formula

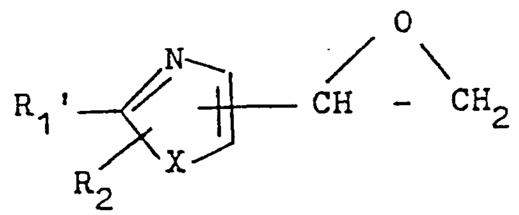

(XXI)

(in which
X and $R_2$ are as defined in claims 1 to 8, and $R_1$' represents a hydrogen or halogen atom, a trifluoromethyl or alkyl group), is reacted with an amine of the general formula

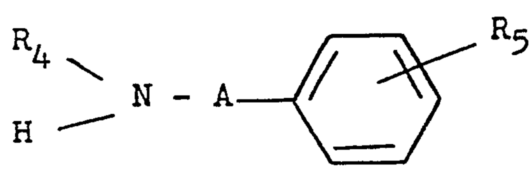

(XXII)

(in which
A, $R_4$ and $R_5$ are as defined in claims 1 to 8), and

if desired, a compound, which has thus been obtained, of the general formula I in which $R_5$ represents or contains an alkoxycarbonyl, aminocarbonyl, alkylaminocarbonyl or dialkylaminocarbonyl group and/or $R_1$ represents an amino group which is substituted by an alkanoyl or benzoyl group, is converted by hydrolysis into a corresponding compound of the general formula I, in which $R_5$ represents or contains a carboxyl group and/or $R_1$ represents an amino group, and/or

a compound, which has thus been obtained, of the general formula I in which $R_5$ represents an alkoxy group, which is substituted by a carboxyl, alkoxycarbonyl, aminocarbonyl, alkylaminocarbonyl or dialkylaminocarbonyl group is converted, by reduction with a suitable metal hydride, into a compound of general formula I in which the above-mentioned substituted alkoxy radical contains a methylene group in place of the carbonyl group, and/or

a compound, which has thus been obtained, of the general formula I in which $R_3$ represents an alkyl group, and/or $R_5$ represents one of the alkoxy groups mentioned in claims 1 to 7, is converted, by ether cleavage, into a corresponding compound of the general formula I in which $R_3$ represents a hydrogen atom and/or $R_5$ represents a hydroxy group or an alkoxy group which is substituted by a hydroxyl group and/or

a compound, which has thus been obtained, of the general formula I is separated into its optical isomers and diastereomers, and/or

a compound, which has thus been obtained, of the general formula I is converted into its acid addition salts, in particular into its physiologically acceptable acid addition salts.

**Revendications**

1. Nouveaux thiazoles et oxazoles substitués de formule générale

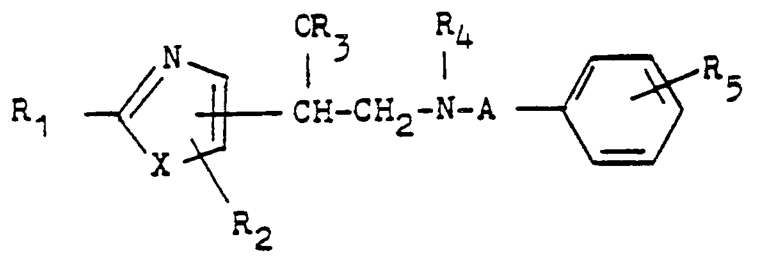

, (I)

dans laquelle

A représente un groupe n-alcoylène avec 2 ou 3 atomes de carbone, éventuellement mono- ou disubstitué par des groupes méthyle ou éthyle,

X représente un atome d'oxygène ou de soufre,

$R_1$ représente un atome d'hydrogène ou d'halogéne, un groupe trifluorométhyle, alcoyle, phényle ou pipéridino ou un groupe amino éventuellement substitué par un ou deux groupes alcoyle ou par un groupe alcanoyle ou benzoyle,

$R_2$ représente un atome d'hydrogène ou un groupe alcoyle,

$R_3$ représente un atome d'hydrogène ou un groupe alcoyle qui peut être substitué en position 2 ou 3 par un groupe hydroxy, ou $R_3$ ensemble avec $R_4$ représente un groupe alcoxycarbonylméthylène ou un groupe éthylène, le groupe méthylène voisin de l'atome de N ou de O pouvant être remplacé par un groupe carbonyle,

$R_4$ représente un atome d'hydrogène, un groupe alcoyle éventuellement substitué par un groupe phényle, carboxy, alcoxycarbonyle ou cyano ou en position 2 ou 3 par un groupe hydroxy ou représente un groupe alcényle et

$R_5$ représente un groupe hydroxy, alcoxy, carboxy, alcoxycarbonyle, aminocarbonyle, alcoylaminocarbonyle ou dialcoylaminocarbonyle, un groupe alcoxy avec 1 à 6 atomes de carbone qui est substitué en position terminale par un groupe carboxy, alcoxycarbonyle, aminocarbonyle, alcoylaminocarbonyle ou dialcoylaminocarbonyle, ou représente un groupe alcoxy avec 2 à 7 atomes de carbone qui est substitué en position terminale par un groupe hydroxy, alcoxy, phénylalcoxy, amino, alcoylamino, dialcoylamino, pyrrolidino, pipéridino ou hexaméthylènimino, ou représente un groupe éthénylène éventuellement substitué par un groupe alcoyle et qui est substitué en position terminale par un groupe carboxy, alcoxycarbonyle, aminocarbonyle, alcoylaminocarbonyle ou dialcoylaminocarbonyle,

tous les groupes alcoyle, alcoxy ou alcanoyle mentionnés plus haut, sauf indication contraire, pouvant contenir chacun 1 à 3 atomes de carbone et les groupes alcényle mentionnés plus haut pouvant contenir 3 à 5 atomes de carbone, leurs isomères optiques et leurs diastéréo-isomères ainsi que leurs sels d'addition d'acides.

2. Nouveaux thiazoles et oxazoles substitués de formule générale I selon la revendication 1, dans laquelle

A représente un groupe éthylène ou n-propylène éventuellement substitué par un groupe méthyle,

X représente un atome d'oxygène ou de soufre,

$R_1$ représente un atome d'hydrogène ou de chlore, un groupe alcoyle avec 1 à 3 atomes de carbone, un groupe trifluorométhyle, phényle, amino, méthylamino, diméthylamino, pipéridino, acétylamino ou benzoylamino,

$R_2$ représente un atome d'hydrogène ou un groupe méthyle,

$R_3$ représente un atome d'hydrogène, un groupe méthyle ou $R_3$ et $R_4$ représentent ensemble un groupe méthoxycarbonylméthylène ou un groupe éthylène, le groupe méthylène voisin de l'atome de O pouvant être remplacé par un groupe carbonyle,

$R_4$ représente un atome d'hydrogène, un groupe méthyle, 2-hydoxy-éthyle, carboxyméthyle, carbéthoxyméthyle ou benzyle et

$R_5$ représente un groupe hydroxy, méthoxy, carboxy, méthoxycarbonyle, éthoxycarbonyle, aminocarbonyle, carboxyméthoxy, méthoxycarbonylméthoxy, éthoxycarbonylméthoxy, aminocarbonylméthoxy, méthylaminocarbonylméthoxy, 2-hydroxy-éthoxy, 2-éthoxy-éthoxy, 2-phénéthoxy-éthoxy, 2-amino-éthoxy, 2-méthylamino-éthoxy, 2-(1-pipéridino)-éthoxy, 6-hydroxy-n-hexoxy ou 2-carbométhoxy-1-méthyl-éthényle,

leurs isomères optiques et leurs diastéréo-isomères ainsi que leurs sels d'addition d'acides.

**3.** Nouveaux thiazoles substitués de formule générale

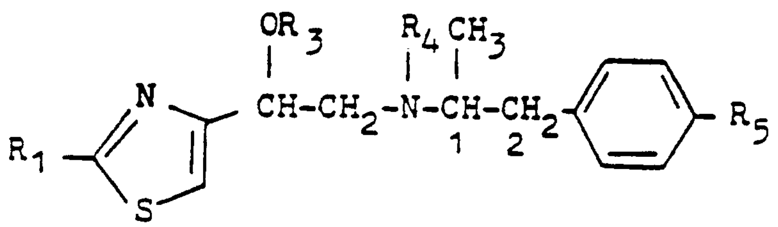

, (Ia)

dans laquelle

$R_1$ représente un atome de chlore, un groupe méthyle ou trifluorométhyle,

$R_3$ représente un atome d'hydrogène ou $R_3$ et $R_4$ représentent ensemble le groupe éthylène ou méthoxycarbonylméthylène,

$R_4$ représente un atome d'hydrogène, un groupe méthyle, 2-hydroxy-éthyle ou carbéthoxyméthyle et

$R_5$ représente un groupe carboxyméthoxy, carbométhoxyméthoxy, éthoxycarbonylméthoxy, aminocarbonylméthoxy, méthylaminocarbonylméthoxy, 2-méthylamino-éthoxy, 2-hydroxy-éthoxy ou 2-carbométhoxy-1-méthyl-éthènyle, leurs isomères optiques et leurs diastéréo-isomères ainsi que leurs sels d'addition d'acides.

**4.** La N-[2-(4-carbométhoxyméthoxyphényl)-1-méthyléthyl]-2-hydroxy-2-(2-trifluorométhyl-thiazol-4-yl)-éthanamine, ses isomères optiques et ses diastéréo-isomères ainsi que ses sels d'addition d'acides.

**5.** La N-[2-(4-carboxyméthoxyphényl)-1-méthyléthyl]-2-(2-trifluorométhyl-thiazol-4-yl) morpholine, ses isomères optiques et ses diastéréo-isomères ainsi que ses sels d'addition d'acides.

**6.** La N-[2-(4-carbométhoxyméthoxyphényl)-1-méthyléthyl]-N-(2-hydroxy-éthyl)-2-hydroxy-2-(2-trifluorométhyl-thiazol-4-yl)-éthanamine, ses isomères optiques et ses diastéréo-isomères ainsi que ses sels d'addition d'acides.

**7.** La N-[2-(4-(2-hydroxyéthoxy)phényl)-1-méthyléthyl]-2-hydroxy-2(2-trifluorométhyl-thiazol-4-yl)éthan amine, ses isomères optiques et ses diastéréo-isomères ainsi que ses sels d'addition d'acides.

**8.** La N-[2-(4-carboxyméthoxyphényl)-1-méthyléthyl]-2-hydroxy-2-(2-trifluorométhyl-thiazol-4-yl)-éthanamine, ses isomères optiques et ses diastéréo-isomères ainsi que ses sels d'addition d'acides.

**9.** Sels physiologiquement supportables des composés selon les revendications 1 à 8 avec des acides non organiques ou organiques.

**10.** Médicament contenant un composé selon les revendications 1 à 8 ou un sel d'addition d'acide physiologiquement supportable selon la revendication 9, éventuellement conjointement à un ou plusieurs excipients et/ou diluants inertes.

**11.** Médicament selon la revendication 10 qui convient au traitement du diabète mellitus, de l'adiposité et au traitement et à la prophylaxie des modifications athérosclérotiques.

**12.** Utilisation des composés selon les revendications 1 à 9 pour la fabrication d'un médicament qui convient au traitement du biabète mellitus, de l'adiposité et au traitement et à la prophylaxie des modifications athérosclérotiques.

**13.** Procédé pour la fabrication d'un médicament selon la revendication 10 ou 11, caractérisé en ce que, par voie non chimique, on introduit un composé selon les revendications 1 à 9 dans un ou plusieurs excipients et/ou diluants inertes.

**14.** Procédé pour la préparation des nouveaux thiazoles et oxazoles selon les revendications 1 à 9, caractérisé en ce que

a) on fait réagir un composé de formule générale

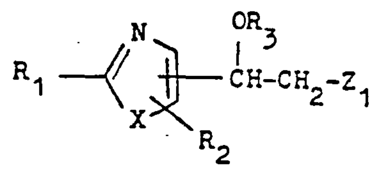

,(II)

avec un composé de formule générale

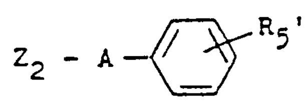

,(III)

dans lesquelles

$R_1$ à $R_3$, A et X sont définis comme dans les revendications 1 à 8,

$Z_1$ représente un groupe partant nucléophile et $Z_2$ un groupe $R_4$-NH- ou

$Z_2$ représente un groupe partant nucléophile et $Z_1$ représente un groupe $R_4$-NH-, $R_4$ étant défini comme dans les revendications 1 à 8, et

$R_5'$ possède les significations mentionnées dans les revendications 1 à 8 pour $R_5$, $R_5'$ ne pouvant toutefois représenter aucun des groupes alcoxycarbonylméthoxy mentionnés dans les revendications 1 à 8 et un groupe carboxy, amino ou alcoylamino contenu dans le radical $R_5$ pouvant être protégé par un radical protecteur et en ce qu'éventuellement ensuite on clive un radical protecteur utilisé ou

b) on réduit une base de Schiff éventuellement formée dans le mélange réactionnel, de formule générale

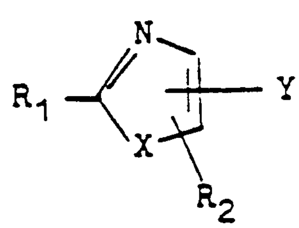

,(IV)

dans laquelle

$R_1$, $R_2$ et X sont définis comme dans les revendications 1 à 8 et

Y représente un groupe de formule

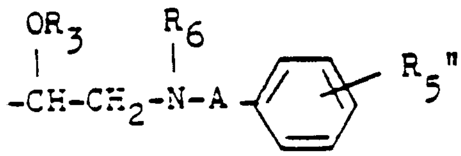

ou

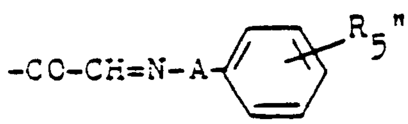

où

$R_3$ et A sont définis comme dans les revendications 1 à 8,

$R_5''$ possède les significations mentionnées pour $R_5$ dans les revendications 1 à 8, toutefois un groupe amino ou alcoylamino contenu dans le radical $R_5$ pouvant être protégé par un radical protecteur, et

$R_6$, ensemble avec un atome d'hydrogène de l'atome de carbone voisin du radical A, représente une liaison supplémentaire et en ce qu'éventuellement ensuite on clive un radical protecteur utilisé ou

c) pour la préparation des composés de formule générale I, dans laquelle $R_3$ et $R_4$ sont définis comme dans les revendications 1 à 8, $R_3$ et $R_4$ ne représentant toutefois pas un groupe éthylène dans lequel le groupe méthylène voisin de l'atome de N est remplacé par un groupe carbonyle, on amine de façon réductrice, en présence d'un agent réducteur approprié, un composé carbonylé de formule générale

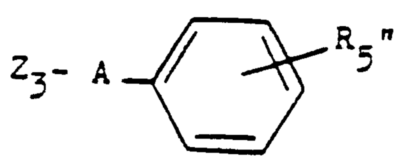

, (V)

dans laquelle

A est défini comme dans les revendications 1 à 8,

$R_5''$ possède les significations mentionnées pour $R_5$ dans les revendications 1 à 8, un groupe amino ou alcoylamino contenu dans le radical $R_5$ pouvant toutefois être protégé par un radical protecteur, et $Z_3$, ensemble avec un atome d'hydrogène de l'atome de carbone voisin du radical A représentant un atome d'oxygène, au moyen d' une amine de formule générale

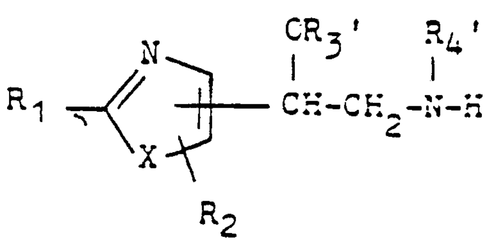

, (VI)

dans laquelle

$R_1$, $R_2$ et X sont définis comme dans les revendications 1 à 8,

$R_3$' et $R_4$' possèdent les significations mentionnées dans les revendications 1 à 8 pour $R_3$ et $R_4$, toutefois $R_3$ et $R_4$ ne représentant pas un groupe éthylène dans lequel le groupe méthylène voisin de l'atome de N est remplacé par un groupe carbonyle, et éventuellement ensuite on clive un radical protecteur utilisé ou

d) pour la préparation des composés de formule générale I dans laquelle $R_3$ représente un atome d'hydrogène, on réduit un composé éventuellement formé dans le mélange réactionnel, de formule générale

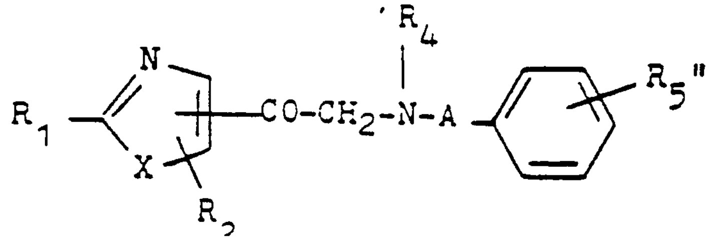

,(VII)

dans laquelle

A, X, $R_1$, $R_2$ et $R_4$ sont définis comme dans les revendications 1 à 8 et

$R_5$" possède les significations mentionnées pour $R_5$ dans les revendications 1 à 8, toutefois un groupe amino ou alcoylamino contenu dans le radical $R_5$ pouvant être protégé par un radical protecteur et éventuellement ensuite on clive un radical protecteur utilisé ou

e) pour la préparation des composés de formule générale I dans laquelle $R_3$ et $R_4$ représentent ensemble un groupe alcoxycarbonylméthylène, on fait réagir un composé de formule générale

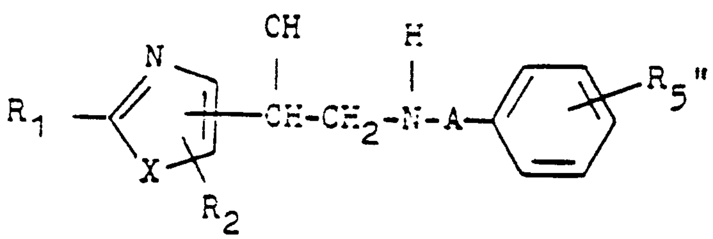

,(VIII)

dans laquelle

A, X, $R_1$ et $R_2$ sont définis comme dans les revendications 1 à 8 et

$R_5$" possède les significations mentionnées pour $R_5$ dans les revendications 1 à 8, toutefois un groupe amino ou alcoylamino contenu dans le radical $R_5$ pouvant être protégé par un radical protecteur, avec un composé de formule générale

$O = CH - COOR_7$ ,(IX)

dans laquelle

$R_7$ représente un groupe alcoyle avec 1 à 3 atomes de carbone et éventuellement ensuite on clive un radical protecteur utilisé ou

f) pour la préparation des composés de formule générale I, dans laquelle $R_5$ représente un groupe alcoxy avec 1 à 3 atomes de carbone, un groupe alcoxy avec 1 à 6 atomes de carbone qui est substitué en position terminale par un groupe carboxy, alcoxycarbonyle, aminocarbonyle, alcoylaminocarbonyle ou dialcoylaminocarbonyle, ou représente un groupe alcoxy avec 2 à 7 atomes de carbone qui est substitué en position terminale par un groupe hydroxy, alcoxy, amino, alcoylamino, dialcoylamino, pyrrolidino, pipéridino ou hexaméthylènimino, on fait réagir un composé de formule générale

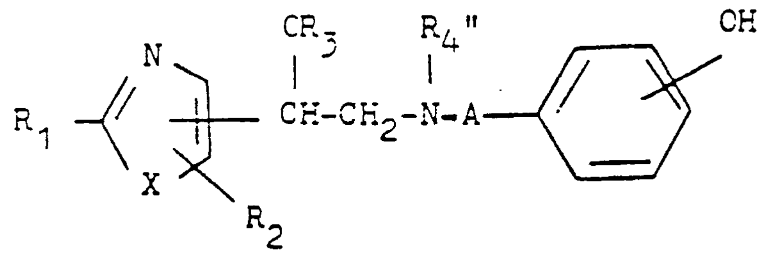

,(X)

dans laquelle

A, X et $R_1$ à $R_3$ sont définis comme dans les revendications 1 à 8 et

$R_4''$ possède les significations mentionnées pour $R_4$ dans les revendications 1 à 8 ou représente un groupe protecteur facilement clivable pour un groupe amino, avec un composé de formule générale

$Z_4$ - $R_8$ ,(XI)

dans laquelle

$R_8$ représente un groupe alcoyle avec 1 à 3 atomes de carbone, un groupe alcoyle avec 1 à 6 atomes de carbone qui est substitué en position terminale par un groupe carboxy, alcoxycarbonyle, aminocarbonyle, alcoylaminocarbonyle, ou dialcoylaminocarbonyle, ou représente un groupe alcoyle avec 2 à 7 atomes de carbone qui est substitué en position terminale par un groupe hydroxy, alcoxy, amino, alcoylamino, dialcoylamino, pyrrolidino, pipéridino ou hexaméthylène-imino, et

$Z_4$ représente un groupe partant nucléophile tel qu'un atome d'halogène ou un groupe sulfonyloxy ou $Z_4$, ensemble avec un atome d'hydrogène en position $\beta$ du radical $R_8$, représente un atome d'oxygène et éventuellement ensuite on clive un radical protecteur utilisé ou

g) pour la préparation des composés de formule générale I dans laquelle $R_3$ représente un atome d'hydrogène, on réduit un composé de formule générale

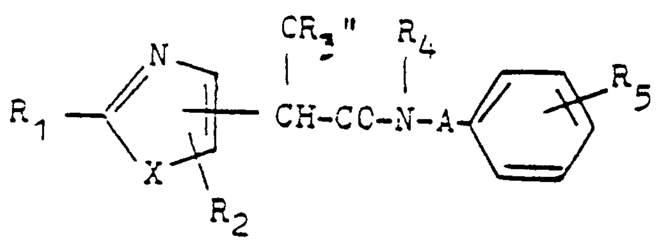

,(XII)

dans laquelle

A, X, $R_1$, $R_2$ et $R_4$ sont définis comme dans les revendications 1 à 8 et

$R_3''$ représente un atome d'hydrogène ou un groupe alcoyle ou

h) pour la préparation des composés de formule générale I dans laquelle $R_5$ représente ou contient un groupe alcoxycarbonyle, aminocarbonyle, alcoylaminocarbonyle ou dialcoylaminocarbonyle, on fait réagir un composé de formule générale

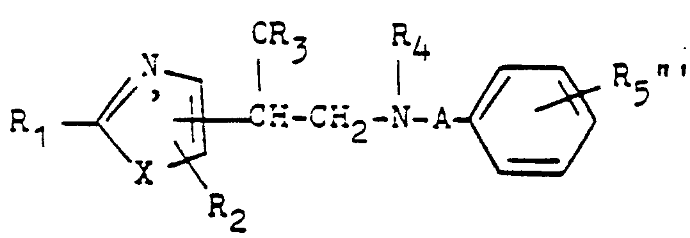

,(XIII)

dans laquelle

$R_1$ à $R_4$, A et X sont définis comme dans les revendications 1 à 8 et

$R_5'''$ représente un groupe carboxy ou un groupe alcoxy avec 1 à 6 atomes de carbone, lequel est substitué en position terminale par un groupe carboxy, ou leurs dérivés réactifs, avec un composé de formule générale

H - $R_9$ ,(XIV)

dans laquelle

$R_9$ représente un groupe alcoxy, amino, alcoylamino ou dialcoylamino, où chaque partie alcoyle ou alcoxy peut contenir 1 à 3 atomes de carbone ou

i) pour la préparation des composés de formule générale I dans laquelle $R_4$ représente un groupe alcoyle éventuellement substitué par un groupe phényle, carboxy, alcoxycarbonyle ou cyano ou en position 2 ou 3 par un groupe hydroxy, ou représente un groupe alcényle, on fait réagir un composé de formule générale

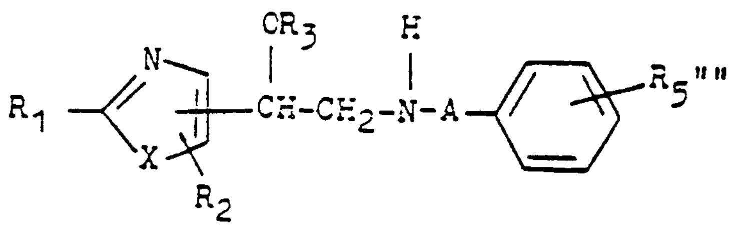

,(XV)

dans laquelle

A, X et $R_1$ à $R_3$ sont définis comme dans les revendications 1 à 8 et

$R_5''''$ possède les significations mentionnées pour $R_5$ dans les revendications 1 à 8, toutefois un groupe amino ou alcoylamino contenu dans le radical $R_5$ pouvant si nécessaire être protégé par un radical protecteur, avec un composé de formule générale

$Z_5$ - $R_4'''$ ,(XVI)

dans laquelle

$R_4'''$ possède les significations mentionnées au début pour $R_4$, à l'exception de l'atome d'hydrogène et $Z_5$ représente un groupe partant nucléophile ou $Z_5$ ensemble avec un atome d'hydrogène $\alpha$ ou $\beta$ du radical alcoyle $R_4'''$ représente un atome d'oxygène et éventuellement ensuite on clive un radical protecteur utilisé ou

k) pour la préparation des composés de formule générale I dans laquelle $R_3$ et $R_4$ représentent ensemble un groupe éthylène, on réduit un composé, éventuellement formé dans le mélange réactionnel, de formule générale

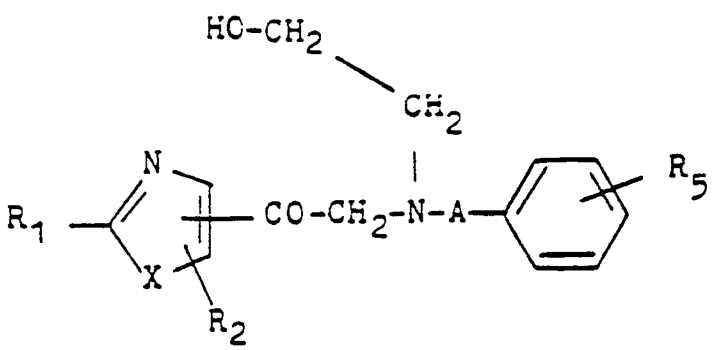

,(XXII)

dans laquelle

A, X, $R_1$, $R_2$ et $R_5$ sont définis comme dans les revendications 1 à 8, ou son semi-acétal

cyclique ou

l) pour la préparation des composés de formule générale I dans laquelle $R_3$ et $R_4$ représentent ensemble un groupe éthylène dans lequel le groupe méthylène voisin de l'atome de N ou de O est remplacé par un groupe carbonyle, on fait réagir un composé de formule générale

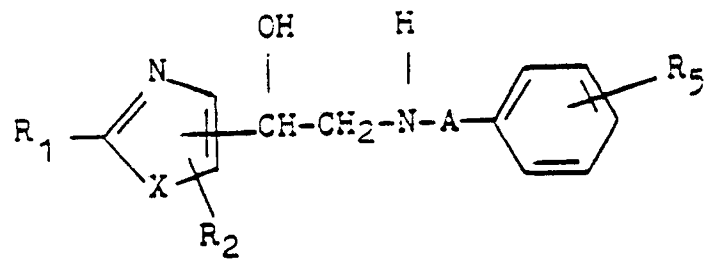

, (XVIII)

dans laquelle

A, X, $R_1$, $R_2$ et $R_5$ sont définis comme dans les revendications 1 à 8, avec un halogénure d'halogénoacétyle ou un ester d'acide halogénoacétique ou

m) pour la préparation des composés de formule générale I dans laquelle $R_3$ et $R_4$ représentent ensemble un groupe éthylène, on réduit un composé de formule générale

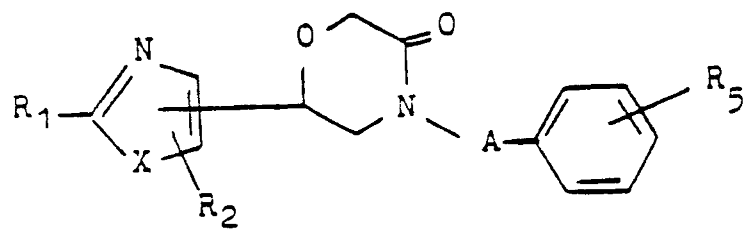

, (XIX)

dans laquelle

A, X, $R_1$, $R_2$ et $R_5$ sont définis comme dans les revendications 1 à 8 ou

n) pour la préparation des composés de formule générale I dans laquelle $R_3$ et $R_4$ représentent ensemble un groupe éthylène dans lequel le groupe méthylène voisin de l'atome de O est remplacé par un groupe carbonyle, on cyclise un composé, éventuellement formé dans le mélange réactionnel, de formule générale

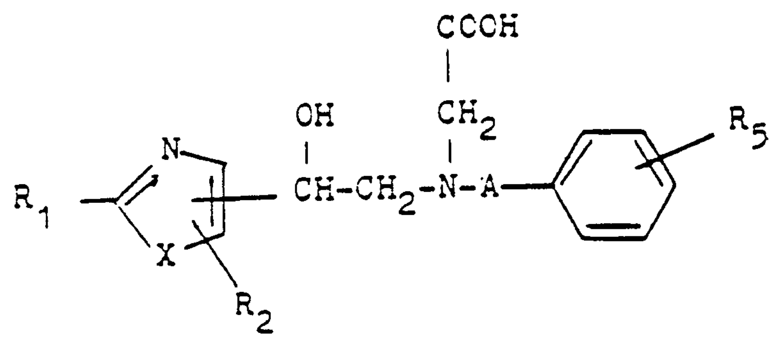

, (XX)

dans laquelle

A, X, $R_1$, $R_2$ et $R_5$ sont définis comme dans les revendications 1 à 8, ou leurs dérivés réactifs tels que leurs esters ou leurs halogénures ou

o) pour la préparation des composés de formule générale I dans laquelle $R_1$ représente un atome d'hydrogène ou d'halogène, un groupe trifluorométhyle ou alcoyle et $R_3$ représente un atome d'hydrogène, on fait réagir un composé de formule générale

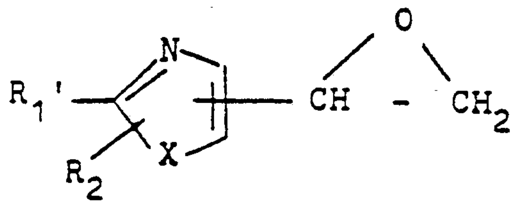

,(XXI)

dans laquelle

X et $R_2$ sont définis comme dans les revendications 1 à 8 et

$R_1'$ représente un atome d'hydrogène ou d'halogène, un groupe trifluorométhyle ou alcoyle, avec une amine de formule générale

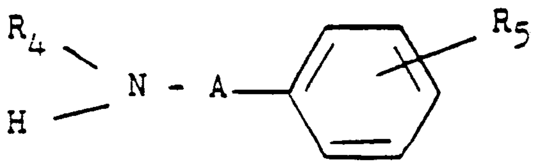

,(XXII)

dans laquelle

A, $R_4$ et $R_5$ sont définis comme dans les revendications 1 à 8 et

si on le souhaite, on transforme un composé ainsi obtenu de formule générale I dans laquelle $R_5$ représente ou contient un groupe alcoxycarbonyle, aminocarbonyle, alcoylaminocarbonyle ou dialcoylaminocarbonyle et/ou $R_1$ représente un groupe amino substitué par un groupe alcanoyle ou benzoyle, au moyen d'une hydrolyse en un composé correspondant de formule générale I dans laquelle $R_5$ représente ou contient un groupe carboxy et/ou $R_1$ représente un groupe amino et/ou

on transforme un composé ainsi obtenu de formule générale I dans laquelle $R_5$ représente un groupe alcoxy substitué par un groupe carboxy, alcoxycarbonyle, aminocarbonyle, alcoylaminocarbonyle ou dialcoylaminocarbonyle, au moyen d'une réduction par un hydrure métallique approprié, en un composé de formule générale I dans laquelle le radical alcoxy substitué mentionné plus haut contient, au lieu du groupe carbonyle, un groupe méthylène et/ou

on transforme un composé ainsi obtenu de formule générale I dans laquelle $R_3$ représente un groupe alcoyle et/ou $R_5$ représente l'un des groupes alcoxy mentionnés dans les revendications 1 à 7, au moyen d'un clivage d'éther en un composé correspondant de formule générale I dans laquelle $R_3$ représente un atome d'hydrogène et/ou $R_5$ représente un groupe hydroxy ou un groupe alcoxy substitué par un groupe hydroxy et/ou

on sépare un composé ainsi obtenu de formule générale I en ses isomères optiques et diastéréoisomères et/ou

on transforme un composé ainsi obtenu de formule générale I en ses sels d'addition d'acides, en particulier en ses sels d'addition d'acides physiologiquement supportables.